# EUROPEAN PATENT APPLICATION

(11) **EP 2 805 703 A1**
(43) Date of publication of application: **26.11.2014**
(21) Application number: 12866207.9
(22) Date of filing: 06.12.2012
(51) Int. Cl.: A61G 7/05, G01G 19/52, G08B 25/04

(54) **BED PROVIDED WITH LOAD DETECTION FUNCTION AND LOAD DETECTION FUNCTION FOR BED**

(30) Priority: 20.01.2012 JP 2012010400; 07.05.2012 JP 2012106299; 14.06.2012 JP 2012135199
(71) Applicant: Showa Denko K.K., Tokyo 105-8518 (JP)
(72) Inventor: HIROSE Kazuo, Oyama-shi Tochigi 323-8678 (JP); TOCHIGI Masaharu, Oyama-shi Tochigi 323-8678 (JP); MOTOMURA Junichi, Oyama-shi Tochigi 323-8678 (JP); ISHIKAWA Motoki, Oyama-shi Tochigi 323-8678 (JP); NOGUCHI Shingo, Oyama-shi Tochigi 323-8678 (JP)
(74) Representative: Tack, Jens
(86) International application number: PCT/JP2012/081666
(87) International publication number: WO 2013/108503

(57) **Abstract**

The object of the invention is to provide a bed with a load detection function. The invention provides a bed with a load detection function that detects a load applied to a bed body, using a load detector attached to the bed body, and detects the state of a user on a bed surface of the bed body. Additionally, a load detector suitable for the bed is provided.

## Description

### BACKGROUND OF THE INVENTION

### Field of the Invention

The present invention relates to a bed with a load detection function that detects a change in the load applied to a bed body and detects the state of a user on a bed surface of a bed body, using a load detector attached to the bed body, and a load detector for adding such a load detection function to a bed.

Priority is claimed on Japanese Patent Application No. 2012-10400, filed January 20, 2012, Japanese Patent Application No. 2012-106299, filed May 7, 2012, and Japanese Patent Application No. 2012-135199, filed June 14,2012, the contents of which are incorporated herein by reference.

### Description of Related Art

For example, in beds to be used in medical institutions, nursing facilities, child care institutions, lodging facilities, ordinary homes, or the like, a method of detecting a change in the load applied to a bed body, and detecting the conditions (getting into bed, getting out of bed, positions while staying in bed, body motions, or the like) of a user (a sick person, a person to be cared for, an infant, a healthy person, or the like) on a bed surface of the bed body is suggested (refer to Patent Documents 1 to 3).

Specifically, Patent Document 1 discloses a method of arranging a load sensor arranged between a leg section provided on a bed body, and an installation surface (floor surface or the like) on which the bed body is installed, and detecting the situation-while-staying-in-bed of a person on the basis of an electrical signal from the load sensor. Additionally, this load sensor is formed with a slope portion for guiding a caster provided on the leg section of the bed body from the installation surface of the bed body onto a load-receiving portion of the load sensor.

Meanwhile, Patent Document 2 discloses a method of detecting the load applied to the bed body by providing a load detector in an empty space between a bed body and an installation surface on which the bed body is installed. Additionally, this load detector is provided with means for lifting a bed.

### PRIOR ART DOCUMENTS

### Patent Documents

[Patent Document 1] Japanese Unexamined Patent Application, First Publication No. 2000-105884
[Patent Document 2] Japanese Unexamined Patent Application, First Publication No. 2008-304397
[Patent Document 3] Japanese Unexamined Patent Application, First Publication No. 2007-256074

### SUMMARY OF THE INVENTION

However, in the invention described in PTL 1, when the load of the bed body is detected using the load sensor, the caster provided on the leg section of the bed should be placed on the load-receiving portion of the load sensor after being moved to the vicinity of a front side of the slope portion of the load sensor and being passed above this slope portion. This is extremely troublesome.

Meanwhile, in the invention described in PTL 2, for example, when a bed body is installed along a wall, an installation person cannot enter a space between the bed body and the wall. Therefore, it is extremely difficult to arrange the load detector in the empty space between the bed body and the installation surface.

Meanwhile, in the invention described in PTL 3, the load detector is incorporated into the bed body in advance, but the bed body should be designed for the load detector, and new parts therefor are required. For this reason, a bed with a load detection function will become extremely expensive. Moreover, it is difficult to achieve weight reduction due to the increase in the number of parts.

The invention has been made in view of such related-art circumstances, and an object of the invention is to provide a bed with a load detection function enabled to add a load detection function with a simple structure while suppressing an increase in the number of parts, and a load detector for a bed that is made to be simply and easily incorporated into a bed body in order to add such a load detection function to the existing bed.

In order to achieve the above object, the invention provides respective aspects described in the following (1) to (24).
(1) A bed provided with a load detection function that detects a change in a load applied to a bed body, using a load detector attached to the bed body, and detects the state of a user on a bed surface of the bed body. The bed body is configured to have a bed surface-forming section that forms the bed surface, a leg section that touches an installation surface on which the bed body is to be installed, and a connecting and supporting section that connects the bed surface-forming section and the leg section together and transmits a load from the bed surface-forming section toward the leg section so that the bed surface-forming section is located above the installation surface. The load detector has a load cell that measures a strain generated by the load being applied to the bed body. The load cell is provided in a portion that receives the load from the bed surface-forming section side and transmits the load to the installation surface side, in any place in a load transmission path that leads from the bed surface-forming section via the connecting and supporting section to the installation surface.
   In the aspect stated in the above (1), the "load transmission path" is equivalent to a structural member that supports the load applied to the bed surface-forming section between the bed surface-forming section and the installation surface, and can be said to be a structural member that transmits the load applied to the bed surface-forming section to the leg section that touches the installation surface.
   The "load transmission path" is equivalent to, for example, one including the connecting and supporting section and the leg section, or one including the connecting and supporting section, the leg section, the bed surface-forming section, and a caster.
   Additionally, in the aspect stated in the above (1), as a specific aspect of the above "the load cell is provided in a portion that receives the load from the bed surface-forming section side and transmits the load to the installation surface side, in any place in a load transmission path", it is desirable to adopt an aspect in which an arbitrary surface (split surface) that vertically splits the load transmission path of the bed body into the installation surface side and the bed surface-forming section side is supposed, and the load cell is provided in at least one place in a structural member that vertically passes through the split surface. For example, the surface of the load transmission path of the bed body where a spindle and a bearing portion receiving the spindle touch each other can be supposed to be the split surface. In this case, the spindle and the bearing portion are equivalent to the structural member that vertically passes through the split surface.
(2) In the aspect of the bed with a load detection function stated in the above (1), the load cell has a substrate that generates a strain according to the load from the bed surface-forming section side, and a strain sensor that is attached to the substrate in order to detect the strain of the substrate, the substrate has a load-receiving portion receiving the load from the bed surface-forming section formed on one end side and has a load-transmitting portion transmitting the load to a structural member on the installation surface side in the bed body formed on the other end side, the actuating portion deflected by the load is formed between the load-receiving portion and the load-transmitting portion, and the strain sensor is attached to the actuating portion.
(3) In the aspect of the bed with a load detection function stated in the above (2), the actuating portion in the substrate constituting the load cell has one end that is constituted by a cantilever portion that is continuously connected to the load-receiving portion and has the other end continuously connected to the load-transmitting portion.
(4) In the aspect of the bed with a load detection function stated in the above (2), a spindle having a substantially horizontal axis is interposed in the load transmission path of the bed body, and any one of the load-receiving portion and the load-transmitting portion of the substrate is formed with a bearing portion equipped with a supporting surface that touches a portion on a lower surface side of an outer peripheral surface of the spindle.
   In the aspect stated in the above (4), the spindle that is provided in the load transmission path of the bed body and has a substantially horizontal axis includes a case where the spindle is slightly inclined from a horizontal direction. Specifically, strictly, when the spindle is slightly inclined from the horizontal direction due to, for example, manufacturing errors of the bed body, the inclination or irregularities of the installation surface, changes over time caused by the prolonged use of the bed, and the movement of a user on the bed surface, or the like, a case where the spindle is inclined, for example, at about less than 5° is also included.
(5) In the aspect of the bed with a load detection function stated in the above aspect (4), the bearing portion has a recessed portion opening to the spindle side so as to accommodate at least a portion of the outer peripheral surface of the spindle, and at least a portion of an inner surface of the recessed portion forms the supporting surface.
(6) In the aspect of the bed with a load detection function stated in the above aspect (4), the supporting surface of the bearing portion is formed by an upward, substantially horizontal plane.
   In the aspect stated in the above (6), the substantially horizontal plane, similar to that described regarding the aspect stated in the above (4), includes a case where the supporting surface is slightly inclined from the horizontal direction. Specifically, strictly, when the supporting surface is slightly inclined from the horizontal direction due to, for example, manufacturing errors of the bed body, the inclination or irregularities of the installation surface, changes over time caused by the prolonged use of the bed, and the movement of a user on the bed surface, or the like, a case where the supporting surface is inclined, for example, at about less than 5° is also included.
(7) In the aspect of the bed with a load detection function stated in the above (4), a stopper member, which covers the spindle with a gap from the spindle and is not mechanically coupled with at least the load-receiving portion and the actuating portion of the substrate, is provided on the side of the spindle that faces the bearing portion.
   In the aspect stated in the above (7), as a configuration in which the stopper member is not mechanically coupled with at least the load-receiving portion and the actuating portion of the substrate, for example, it is desirable to adopt a configuration in which the stopper member is spatially separated from at least the load-receiving portion and the actuating portion of the substrate (a configuration in which a gap is spatially present between the stopper member and at least the load-receiving portion and the actuating portion of the substrate). Additionally, functionally speaking, a state where a force applied to the stopper member is not transmitted to the bearing portion and the actuating portion of the substrate is preferable so that a strain does not occur in the actuating portion due to the force applied to the stopper member (accordingly, the strain sensor does not detect a strain caused by the force).
(8) In the aspect of the bed with a load detection function stated in the above (4), the substrate is configured so that at least the load-transmitting portion of the substrate is inserted into a tubular member fixed to the structural member on the installation surface side of the bed body, and the stopper member is configured so as to be supported by the tubular member.
(9) In the aspect of the bed with a load detection function stated in the above any one of (1) to (3), the substrate constituting the load cell is interposed in the middle of the connecting and supporting section.
(10) In the aspect of the bed with a load detection function stated in the above (9), the connecting and supporting section includes a lifting link mechanism that lifts and lowers the bed surface-forming section, and the substrate constituting the load cell is incorporated into the lifting link mechanism.
(11) In the aspect of the bed with a load detection function stated in the above (10), the connecting and supporting section includes a bottom frame that is supported via the leg section above the installation surface in addition to the lifting link mechanism, and the lifting link mechanism has at least a first arm and a second arm as arms that connect the bed surface-forming section and the bottom arm together, in which the first arm is connected to the bed surface-forming section side, the second arm is connected to the bottom frame side, and the substrate constituting the load cell is interposed between the bed surface-forming section and the bottom frame.
(12) In the aspect of the bed with a load detection function stated in the above (11), the bed surface-forming section has a bed plate, and a top frame that supports the bed plate, and the lifting link mechanism has at least a first arm and a second arm as arms that connect the top frame and the bottom arm together, in which the first arm is connected to the top frame side, the second arm is connected to the bottom frame side, and the substrate constituting the load cell is interposed between the top frame and the bottom arm.
(13) In the aspect of the bed with a load detection function stated in the above (12), the spindle is provided at the end of any one arm of the top arm and the bottom arm where the substrate constituting the load cell is located, and the bearing portion that receives the spindle is formed in any one of the load-receiving portion and load-transmitting portion in the substrate.
(14) In the aspect of the bed with a load detection function stated in the above (13), the load-transmitting portion in the substrate constituting the load cell is an attachment portion that is attached to the other arm of the top arm and the bottom arm.
(15) In the aspect of the bed with a load detection function stated in the above aspect (14), a hollow tube portion is formed at the end of the other arm on the substrate side, the attachment portion of the substrate is inserted into the hollow tube portion, and the attachment portion is configured so as to be supported by the hollow tube portion.
(16) In the aspect of the bed with a load detection function stated in the above any one of (1) to (3), the substrate constituting the load cell is interposed between the bed surface-forming section and the connecting and supporting section.
(17) In the aspect of the bed with a load detection function stated in the above any one of (1) to (3), the substrate constituting the load cell is interposed between the connecting and supporting section and the leg section.
(18) In the aspect of the bed with a load detection function stated in the above any one of (1) to (3), the substrate constituting the load cell is incorporated into the leg section.
(19) In the aspect of the bed with a load detection function stated in the above any one of (1) to (3), the leg section includes a caster mechanism, and the substrate constituting the load cell is incorporated into the caster mechanism.
(20) A load detector is provided for a bed that is attached to a bed body, including a bed surface-forming section that forms a bed surface; a leg section that touches an installation surface on which the bed body is to be installed; and a connecting and supporting section that connects the bed surface-forming section and the leg section together and transmits a load from the bed surface-forming section toward the leg section so that the bed surface-forming section is located above the installation surface, in any place in a load transmission path that leads from the bed surface-forming section via the connecting and supporting section to the installation surface, and that thereby measures a change in a load applied to the bed body, and detects the state of a user on a bed surface of the bed body. The load detector includes a load cell having a substrate that generates a strain according to the load from the bed surface-forming section side; and a strain sensor that is attached to the substrate in order to detect the strain of the substrate. The substrate is configured so as to be attached to a portion that receives the load from the bed surface-forming section side and transmits the load to the installation surface side, in any place in a load transmission path that leads from the bed surface-forming section of the bed body via the connecting and supporting section to the leg section. The substrate has a load-receiving portion that receives the load from the bed surface-forming section; an actuating portion that has the strain sensor attached thereto and is deflected by the load; and a load-transmitting portion that transmits the load to the structural member of the bed body on the installation surface side. A bearing portion, that is equipped with a supporting surface that touches a portion of an outer peripheral surface of a spindle that is provided in the load transmission path of the bed body and has a substantially horizontal axis, is formed in any one of the load-receiving portion and the load-transmitting portion of the substrate.
   In the aspect stated in the above (20), the "load transmission path", similar to that described regarding the aspect stated in the above (1), is equivalent to a structural member that supports the load applied to the bed surface-forming section between the bed surface-forming section and the installation surface, and is equivalent to, for example, a structural member that transmits the load applied to the bed surface-forming section to the leg section that touches the installation surface, the leg section itself, the caster attached to the leg section, or the like.
   Additionally, in the aspect stated in the above (20), as a specific aspect, "the substrate is configured so as to be attached to a portion that receives the load from the bed surface-forming section side and transmits the load to the installation surface side, in any place in a load transmission path that leads from the bed surface-forming section of the bed body via the connecting and supporting section to the leg section" is the same as that already described regarding the aspect stated in the above (1).
   Moreover, in the aspect stated in the above (20), "the spindle having a substantially horizontal axis" is the same as that already described regarding the aspect stated in the above (4).
(21) In the aspect of the load detector for a bed stated in the above (20), the bearing portion has a recessed portion opening to the spindle side so as to accommodate at least a portion of the outer peripheral surface of the spindle, and a portion of an inner surface of the recessed portion forms the supporting surface.
(22) In the aspect of the load detector for a bed stated in the above (20), the supporting surface is a substantially horizontal plane.
   Moreover, in the aspect stated in the above (22), "the supporting surface is a substantially horizontal plane" is the same as that already described regarding the aspect stated in the above (6).
(23) In the aspect of the load detector for a bed stated in the above (20), a stopper member, which covers the spindle with a gap from the spindle and is not mechanically coupled with the substrate is provided on the side of the spindle that faces the bearing portion.
   Moreover, in the aspect stated in the above (23), "is not mechanically coupled with" is the same as that already described regarding the aspect stated in the above (7).
(24) In the aspect of the load detector for a bed stated in the above (23), the stopper member has wall surfaces that face the outer peripheral surface of the spindle at positions at both ends of the supporting surface.
(25) In the aspect of the load detector for a bed stated in the above (20), the substrate is configured so that at least the load-transmitting portion of the substrate is inserted into a tubular member fixed to the structural member on the installation surface side of the bed body, and the stopper member is configured so as to be supported by the tubular member.

According to the invention, it is possible to provide a bed with a load detection function enabled to add a load detection function with a simple structure while suppressing an increase in the number of parts, and a load detector that is made easily to be separately incorporated into a bed body in order to add such a load detection function to an existing bed.

### BRIEF DESCRIPTION OF THE DRAWINGS

FIG. 1 is a side view showing an example of a bed with a load detection function to which the invention is applied.
FIG. 2A is a side view of main portions of a bed body showing a state where a bed plate is lowered by lifting link mechanisms, in the bed shown in FIG. 1.
FIG. 2B is a side view of the main portions of the bed body showing a state where the bed plate is lifted by the lifting link mechanisms, in the bed shown in FIG. 1.
FIG. 3A is an enlarged side view of main portions of a lifting link mechanism into which a load cell is incorporated, in the bed shown in FIG. 1.
FIG. 3B is an enlarged front view of the main portions of the lifting link mechanism into which the load cell is incorporated, in the bed shown in FIG. 1.
FIG. 4A is a side view of a substrate (elastic body) in the load cell.
FIG. 4B is an enlarged side view of a cantilever portion (actuating portion) of the substrate (elastic body) in the load cell.
FIG. 4C is an enlarged top view of the cantilever portion (actuating portion) of the substrate (elastic body) in the load cell.
FIG. 5 is a circuit diagram showing a Wheatstone bridge circuit.
FIG. 6A is a side view showing the state of the substrate (elastic body) before a load is applied to the bed body.
FIG. 6B is a side view showing the state of the substrate (elastic body) after the load is applied to the bed body.
FIG. 7A is a side view showing another example of the substrate (elastic body).
FIG. 7B is a side view showing still another example of the substrate (elastic body).
FIG. 8 is an enlarged side view showing, in the track of FIG. 3A, main portions of another example of a lifting link mechanism into which the load cell is incorporated.
FIG. 9 is an enlarged side view showing main portions of still another example of the lifting link mechanism into which the load cell is incorporated.
FIG. 10 is a partial cutaway side view showing an example of a load cell of a load detector to be used for the bed with a load detection function of the invention.
FIG. 11 is a partial cutaway side view showing another example of the load cell of the load detector to be used for the bed with a load detection function of the invention.
FIG. 12 is a partial cutaway side view of an example in which a substrate constituting the load cell is configured so as to be split into three pieces, as the load cell of the load detector to be used for the bed with a load detection function of the invention.
FIG. 13 is a partial cutaway side view of an example in which the substrate constituting the load cell is configured so as to be split into two pieces, as the load cell of the load detector to be used for the bed with a load detection function of the invention.
FIG. 14 is a partial cutaway side view of another example in which the substrate constituting the load cell is configured so as to be split into two pieces, as the load cell of the load detector to be used for the bed with a load detection function of the invention.
FIG. 15 is a side view showing another example of the bed with a load detection function to which the load detector for a bed of the invention is applied.
FIG. 16A is an enlarged side view of main portions of lifting link mechanisms into which a load cell is incorporated, in the bed shown in FIG. 15.
FIG. 16B is an enlarged front view of the main portions of the lifting link mechanisms into which the load cell is incorporated, in the bed shown in FIG. 15.
FIG. 17 is a side view of a substrate in the load cell of the bed shown in FIG. 15.
FIG. 18 is a conceptual diagram illustrating the relationship between the shape and dimension of a bearing portion of the substrate shown in FIG. 17 and a spindle in more detail.
FIG. 19A is a conceptual diagram for conceptually illustrating the influence exerted by the relationship, regarding a first example of the relationship between the shape and dimension of the bearing portion of the substrate, and the spindle.
FIG. 19B is a conceptual diagram for conceptually illustrating the influence exerted by the relationship, regarding a second example of the relationship between the shape and dimension of the bearing portion of the substrate, and the spindle.
FIG. 19C is a conceptual diagram for conceptually illustrating the influence exerted by the relationship, regarding a third example of the relationship between the shape and dimension of the bearing portion of the substrate, and the spindle.
FIG. 20A is a rough explanatory view showing main portions of the bearing portion illustrating a first example regarding the shape of the bearing portion of the substrate shown in FIG. 17.
FIG. 20B is a rough explanatory view showing the main portions of the bearing portion illustrating a second example regarding the shape of the bearing portion of the substrate shown in FIG. 17.
FIG. 20C is a rough explanatory view showing the main portions of the bearing portion illustrating a third example regarding the shape of the bearing portion of the substrate shown in FIG. 17.
FIG. 20D is a rough explanatory view showing the main portions of the bearing portion illustrating a fourth example regarding the shape of the bearing portion of the substrate shown in FIG. 17.
FIG. 21A is an enlarged front view showing main portions of still another example of the load cell of the load detector of the invention.
FIG. 21B is a right side view of the load cell shown in FIG. 21A.
FIG. 21C is a vertical cross-sectional view in line XXI-XXI of FIG. 21B.
FIG. 22 is a perspective view from a bottom side of the load cell shown in FIGS. 21A to 21C.
FIG. 23 is a perspective view of the substrate in the load cell shown in FIGS. 21A to 21C and FIG. 22.
FIG. 24A is a vertical cross-sectional view showing main portions of the load cell illustrating the function of a stopper member of the load cell shown in FIGS. 21A to 21C and FIG. 22.
FIG. 24B is a vertical cross-sectional view showing the main portions of the load cell illustrating the function of a stopper member having a configuration different from that of FIG. 24A.
FIG. 25 is a partial cutaway side view of an example in which a substrate constituting the load cell is configured so as to be split into three pieces, as the load cell of the load detector for a bed of the invention.
FIG. 26 is a partial cutaway side view of an example in which the substrate constituting the load cell is configured so as to be split into two pieces, as the load cell of the load detector for a bed of the invention.
FIG. 27 is a partial cutaway side view of another example in which the substrate constituting the load cell is configured so as to be split into two pieces, as the load cell of the load detector for a bed of the invention.
FIG. 28A is a front view showing another example of the load cell of the load detector for a bed of the invention.
FIG. 28B is a right side view of the load cell shown in FIG. 28A.
FIG. 28C is a vertical cross-sectional view in line XXVIII-XXVIII in FIG. 28B.
FIG. 29 is a perspective view of the load cell shown in FIGS. 28A to 28C.
FIG. 30 is a perspective view shown the load cell shown in FIGS. 28A to 28C in a state where a casing is detached.
FIG. 31 is a front view of a substrate to be used for the load cell shown in FIGS. 28A to 28C.
FIG. 32 is a perspective view of the substrate to be used for the load cell shown in FIGS. 28A to 28C.
FIG. 33 is an enlarged vertical cross-sectional view showing main portions of the substrate and a stopper member to be used for the load cell shown in FIGS. 28A to 28C.
FIG. 34 is a cross-sectional plan view in line XXXIV-XXXIV of FIG. 33.
FIG. 35 is a front view showing still another example of the load cell of the load detector for a bed of the invention.
FIG. 36 is a left side view of the load cell shown in FIG. 35.
FIG. 37 is a right side view of the load cell shown in FIG. 35.
FIG. 38 is a vertical cross-sectional side view in line XXXVIII-XXXVIII in FIG. 35.
FIG. 39 is a perspective view from the bottom side of the load cell shown in FIG. 35.
FIG. 40 is a front view corresponding to FIG. 35, showing a situation during the load application of the load cell shown in FIG. 35.
FIG. 41 is a front view of the load cell, showing an example of the attachment situation of the load cell shown in FIG. 35.
FIG. 42 is a front view of the load cell, showing another example of the attachment situation of the load cell shown in FIG. 35.
FIG. 43 is a side view showing another example of the bed with a load detection function to which the load detector of the invention is applied.
FIG. 44 is a side view showing still another example of the bed with a load detection function to which the load detector of the invention is applied.
FIG. 45 is a side view showing a still further example of the bed with a load detection function to which the load detector of the invention is applied.

### DETAILED DESCRIPTION OF THE INVENTION

Hereinafter, the configuration of a bed with a load detection function and a load detector to which the invention is applied will be described with reference to the drawings. In addition, in the drawings to be used with the following description, characteristic portions may be shown in an enlarged manner for convenience in order to make characteristics easily understood, and the dimension scales or the like of respective structural elements are not necessarily the same as actual dimension scales. Additionally, materials, dimensions, and the like that are exemplified in the following description are examples, and the invention is not limited to these and can be appropriately changed and carried out without departing from the scope of the invention.

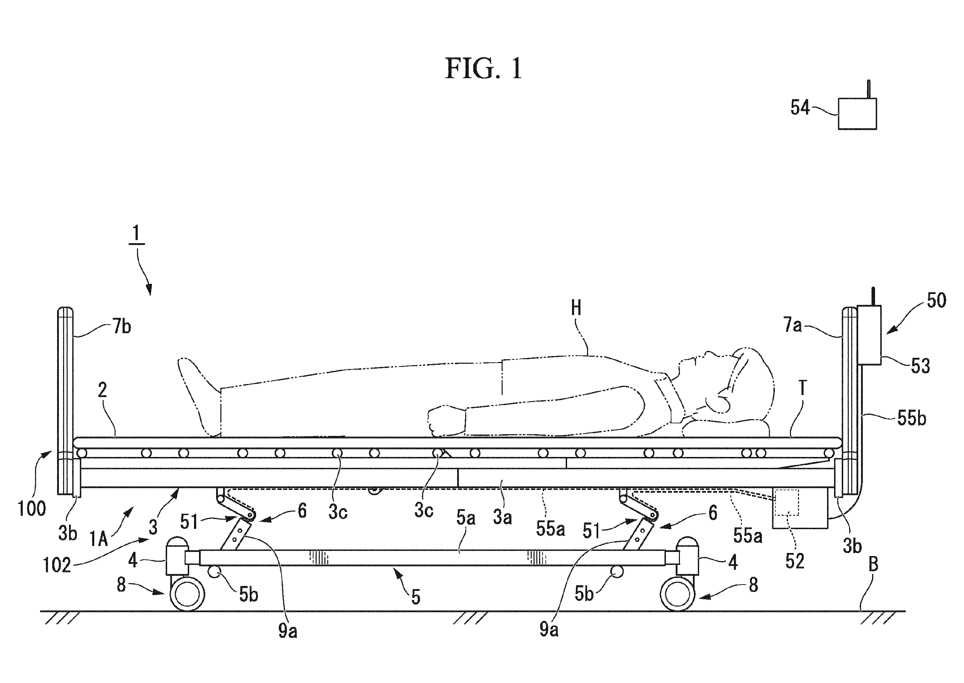

FIG. 1 is a side view showing an example of a bed 1 with a load detection function to which the invention is applied, that is, a side view of the bed 1 incorporating a load detector 50 for a bed according to a first example.

The bed 1 with a load detection function includes a bed body 1A installed, for example, on an installation surface B, such as a floor surface, and has the function of detecting a change in the load applied to the bed body 1A and detecting the state of the user H on a bed surface T of the bed body 1A, using a load detector 50 attached to the bed body 1A.

In addition, in the following description, the installation surface B and the bed surface T of the bed body 1A shown in FIG. 1 are referred to as horizontal surfaces (surfaces orthogonal to the gravitational direction). Also, in a state where the user H goes to bed in a supine posture on the bed surface T of the bed body 1A, a head side of the user H is referred to as "a front side of the bed body 1A", a leg side of the user H is referred to as "a rear side of the bed body 1A", a right side of the user H is referred to as "a right side of the bed body 1A", and a left side of the user H is referred to as "a left side of the bed body 1A".

Specifically, the bed body 1A is configured to generally include a bed surface-forming section 100 that forms the bed surface T, a leg section 4 that touches the installation surface B on which the bed body 1A is to be installed, and a connecting and supporting section 102 that connects the bed surface-forming section 100 and the leg section 4 and transmits the load from the bed surface-forming section 100 toward the leg section 4 so that the bed surface-forming section 100 is located above the installation surface B.

Here, in the example shown in FIG. 1, the bed surface-forming section 100 is constituted by a bed plate 2, and a top frame 3 that supports the bed plate 2. Additionally, the connecting and supporting section 102 includes a bottom frame 5, and a lifting link mechanism 6 that lifts and lowers the bed plate 2 together with the top frame 3 while coupling the top frame 3 and the bottom frame 5.

The bed plate 2 is made of a rectangular plate having length and width that are sufficient for the user H to go to bed on.

In the bed body 1A, the user H is enabled to stay on the bed plate 2 in a state where, for example, a mattress, a sleeping mat, or the like is laid on the bed plate. (In addition, in FIG. 1, a state where the user H directly lies on one's side on an upper surface (bed surface T) of the bed plate 2 is shown).

The top frame 3 has the structure (frame structure) in which a pair of left and right pipe frames 3 a extending in a length direction (longitudinal direction of the bed body 1A) of the bed plate 2 and a pair of front and rear pipe frames 3b extending in a width direction (lateral direction of the bed body 1 A) of the bed plate 2 are connected together in the shape of a frame as a whole, and a plurality of pipe frames 3c extending in the width direction (lateral direction of the bed body 1 A) of the bed plate 2 are connected with the pair of left and right pipe frames 3a in a state where the pipe frames 3c are lined up in the length direction (longitudinal direction of the bed body 1A) of the bed plate 2.

Also, the bed plate 2 is attached in a state where the bed plate is fixed on the plurality of pipe frames 3c. Additionally, a head plate 7a and a foot plate 7b are respectively attached to the pair of front and rear pipe frames 3b constituting the top frame 3, in a state where these plates are erected vertically upward.

Four leg sections 4 are arranged at four corners (the front left side, the front right side, the rear left side, and the rear right side) of the bed body 1 A that is in a mutually symmetrical positional relationship. Additionally, the four leg sections 4 are respectively provided with caster mechanisms 8 for facilitating the movement of the bed body 1A, which is a heavy load. In addition, the configuration of the caster mechanisms 8 is not particularly limited, and it is possible to use caster mechanisms that are well-known in the related art. Additionally, the leg sections 4 that do not have the caster mechanism are also allowed depending on the case.

A bottom frame 5 has the structure (frame structure) in which a pair of left and right pipe frames 5a extending in the longitudinal direction of the bed body 1A and a pair of front and rear pipe frames 5b extending in the lateral direction of the bed body 1 A are coupled together in the shape of a frame as a whole. Also, the leg sections 4 (caster mechanisms 8) are respectively provided at both ends of the pair of left and right pipe frames 5a that constitute the bottom frame 5.

A pair of the lifting link mechanisms 6 in the aforementioned connecting and supporting section 102 are arranged side by side on the front side and the rear side of the bed body 1A. Additionally, the front and rear lifting link mechanisms 6 basically have the same structure except that the attachment positions thereof are different from each other. Moreover, the front and rear lifting link mechanisms 6 have a bilaterally symmetrical structure between the right side and the left side of the bed body 1A, respectively.

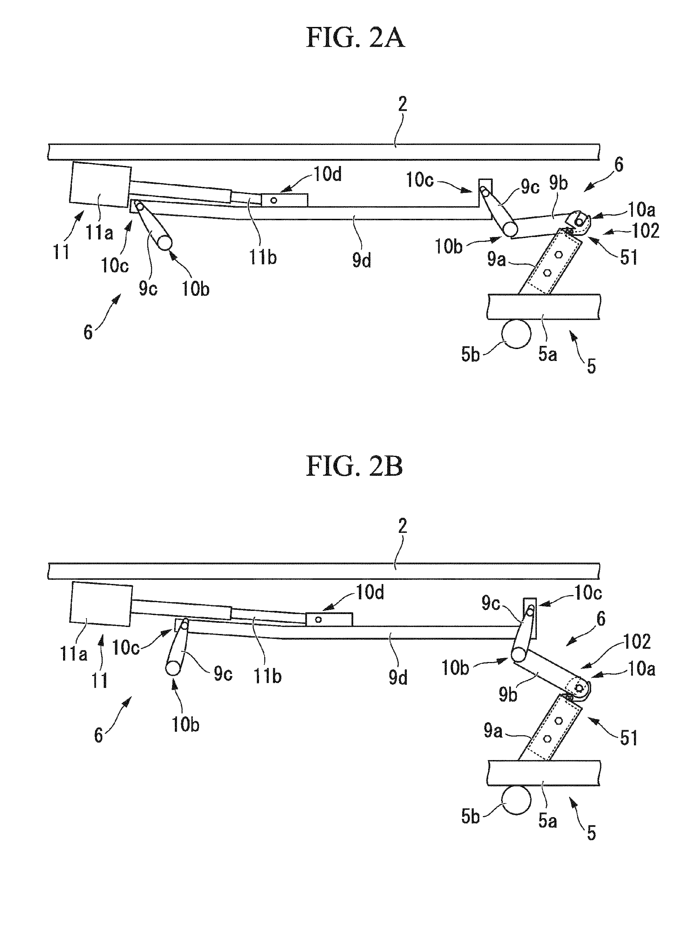

Accordingly, the front and rear lifting link mechanisms 6 will be collectively described if necessary, for example, as shown in FIGS. 2A and 2B.

In addition, here, the swing lifting type lifting link mechanisms 6 are shown as an example of the lifting mechanisms for lifting and lowering the bed plate 2. However, other link mechanisms, pantagraph type or vertical hoisting type lifting mechanisms, or the like can be applied as the lifting mechanisms. Even in these cases, if a spindle (pin) 13 having a substantially horizontal axis as will be described below is provided in the middle of or an end of a lifting mechanism as a member to which the load from the bed plate 2 is applied, the invention can be applied similar to a case where the lifting mechanism is constituted by the swing lifting type lifting link mechanism 6 as will be described later.

FIG. 2A is a side view of main portions of the bed body 1A showing a state where the bed plate 2 is lowered together with the top frame (not shown) by the lifting link mechanisms 6. Meanwhile, FIG. 2B is a side view of the main portions of the bed body 1A showing a state where the bed plate 2 is lifted together with the top frame (not shown) by the lifting link mechanisms 6.

Specifically, the lifting link mechanism 6, as shown in FIGS. 2A and 2B, has first to third coupling arms 9a, 9b, and 9c that are coupled together between the top frame 3 and the bottom frame 5 and are provided in pairs on left and right, respectively.

Among these, the first coupling arms 9a are attached in a state where lower ends thereof are fixed to the pair of front and rear pipe frames 5b constituting the bottom frame 5. In addition, a hollow tube portion 103 is formed at least on an upper end side (a side far from the bottom frame 5) of the first coupling arm 9a. Meanwhile, the second coupling arm 9b has a lower end rotatably attached to an upper end of the first coupling arm 9a via a first hinge portion 10a. Meanwhile, the third coupling arm 9c has a lower end rotatably attached to an upper end of the second coupling arm 9b via a second hinge portion 10b.

Additionally, the lifting link mechanism 6 has a pair of fourth left and right coupling arms 9d that connects the third front and rear coupling arms 9c. Also, upper ends of the third front and rear coupling arms 9c are rotatably attached to the fourth coupling arms 9d via third hinge portions 10c, respectively.

Additionally, the lifting link mechanism 6 has an actuator (drive mechanism) 11 for driving the bed plate 2 for lifting and lowering together with the top frame (not shown). The actuator 11 electrically moves (extends and retracts) a piston 11b in a front-and-rear direction from a cylinder 11 a. Among these, the cylinder 11a is attached in a state where the cylinder is fixed to the top frame 5 (not shown FIGS. 2A and FIG. 2B). Meanwhile, the piston 11b has a tip portion rotatably attached to the fourth coupling arm 9d via a fourth hinge portion 10d. In addition, the actuator 11 is provided only on one side of the right and left sides of the bed body 1A.

Also, in the lifting link mechanism 6, as shown in FIG. 2A, as the piston 11b is moved (extended) forward by the driving of the actuator 11 from a state where the bed plate 2 is lowered together with the top frame (not shown), the first to fourth coupling arms 9a to 9d, as shown in Fig. 2B, are brought into a state where the bed plate 2 is lifted together with the top frame (not shown) while cooperating with each other. On the contrary, as shown in FIG. 2B, as the piston 11b is moved (retracted) rearward by the driving of the actuator 11 from a state where the bed plate 2 is lifted together with the bottom frame (not shown), the first to fourth coupling arms 9a to 9d, as shown in Fig. 2B, are brought into a state where the bed plate 2 is lowered together with the top frame (not shown) while cooperating with each other. Accordingly, it is possible to adjust the height of the bed plate 2 while carrying out the lifting and lowering operation of the bed plate 2 together with the top frame (not shown). Also, the load from the bed plate 2 is applied to the pin (spindle) 13, having a substantially horizontal axis, of the first hinge portion 10a in the lifting link mechanism 6.

The load detector 50, as shown in FIG. 1, has a load cell 51 that measures the strain generated by a load being applied to the bed body 1A. Moreover, in the present example, as shown in FIG. 1, the load detector includes, in addition to the load cell 51, a computing unit 52 that computes the state of the user H on the bed surface T of the bed body 1A on the basis of a load signal output from the load cell 51, a transmitting unit 53 that remotely transmits a result computed by the computing unit 52, and a receiving unit 54 that receives a signal transmitted from the transmitting unit 53.

In addition, the load cell 51 and the computing unit 52 are electrically connected to each other by wiring line 55a, and the computing unit 52 and the transmitting unit 53 are electrically connected to each other by a wiring line 55b. Meanwhile, transmission and reception are enabled between the transmitting unit 53 and the receiving unit 54 by radio (electric wave).

Incidentally, the bed 1 with a load detection function to which the load detector for a bed of the invention is applied is a bed in which the load cell 51 is incorporated into a portion that receives the load from the bed surface-forming section 100 side and transmits the load to the installation surface B side in any place in a load transmission path that leads from the bed surface-forming section 100 via the connecting and supporting section 102 to the leg section 4.

Also, particularly in the case of the example of FIG. 1, the load cell 51 is incorporated into the lifting link mechanism 6 of the connecting and supporting section 102 in the above load transmission path. Thus, a case where the load cell 51 is incorporated into the lifting link mechanism 6 as described above will first be described herein.

Specifically, load cells 51 (four in total), as shown in FIGS. 2A and 2B, are respectively attached to first hinge portions 10a arranged at four corners (the front left side, the front right side, the rear left side, and the rear right side), which are in a mutually symmetrical positional relationship, among the first to fourth hinge portions 10a to 10d that constitute the lifting link mechanisms 6.

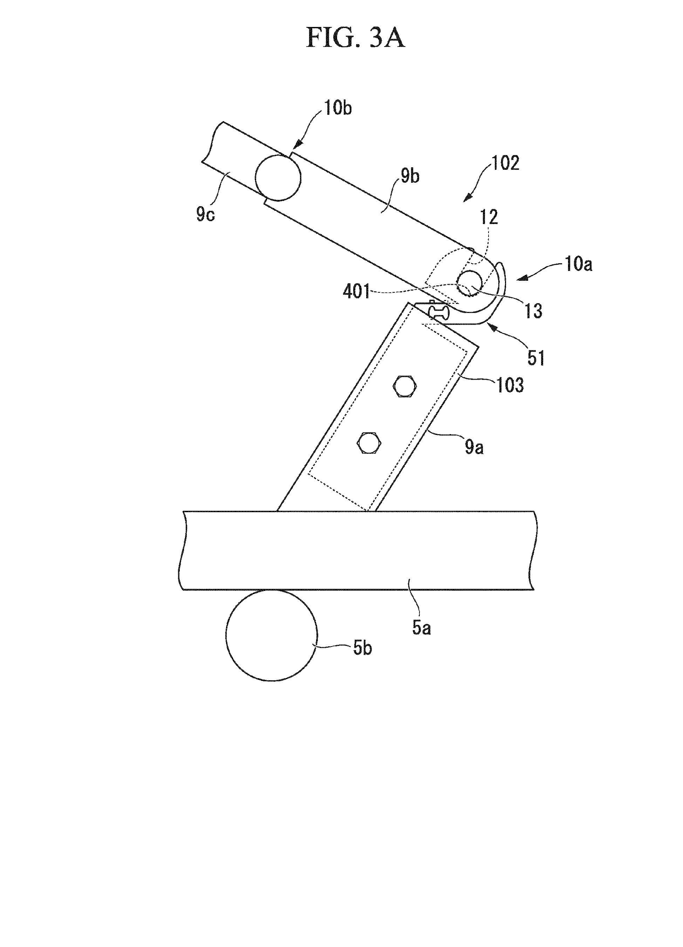

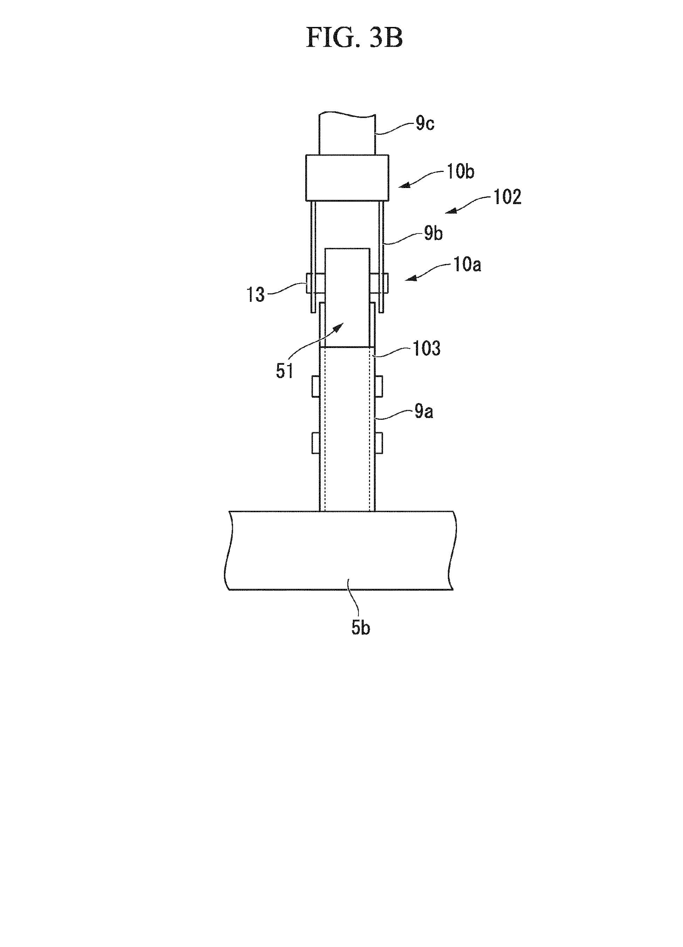

Additionally, the four load cells 51 basically have the same structure except that the attachment positions thereof are different from each other. Accordingly, the four load cells 51 will be collectively described, for example, as shown in FIGS. 3A and 3B.

In addition, FIG. 3A is an enlarged side view of main portions of a lifting link mechanism 6 into which a load cell 51 is incorporated. Meanwhile, FIG. 3B is an enlarged front view of the main portions of the lifting link mechanism 6 into which the load cell 51 is incorporated.

Specifically, the first hinge portion 10a has the structure in which the second coupling arm 9b is rotatably supported with respect to the first coupling arm 9a by the pin (spindle) 13 provided in the second coupling arm (the other coupling arm) 9b being journalled to the bearing provided in the first coupling arm (one coupling arm) 9a in an engaged state.

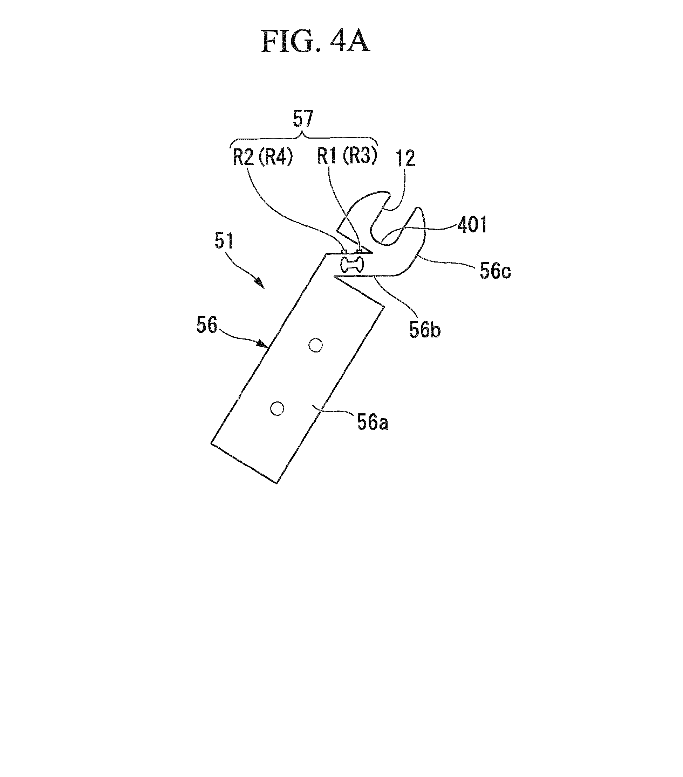

The load cell 51 basically has a substrate 56 that generates strain according to the load from the bed surface-forming section 100 side, and a strain sensor 57 that is attached to the substrate in order to detect the strain of the substrate (refer to FIG. 4A).

Here, the substrate 56 is equivalent to a so-called elastic body. Also, the substrate 56 has on one end side a bearing portion 56c to be described below formed as a load-receiving portion that receives the load from the bed surface-forming section 100 and has on the other end side an attaching portion 56a to be described below formed as a load-transmitting portion that transmits the load to a structural member on the installation surface side in the bed body. A cantilever portion 56b to be described below is formed between the load-receiving portion (bearing portion 56c) and the load-transmitting portion (attaching portion 56a) as an actuating portion that is deflected by the load, and the strain sensor 57 is attached to the actuating portion (cantilever portion 56b). Also, the bearing portion 56c as the load-receiving portion is formed with the aforementioned guide slit (bearing) 12. In the substrate 56, the attaching portion 56a as the load-transmitting portion is inserted into a hollow tube portion 103 of the first coupling arm 9a, and is attached to the first coupling arm 9a by screw-stopping.

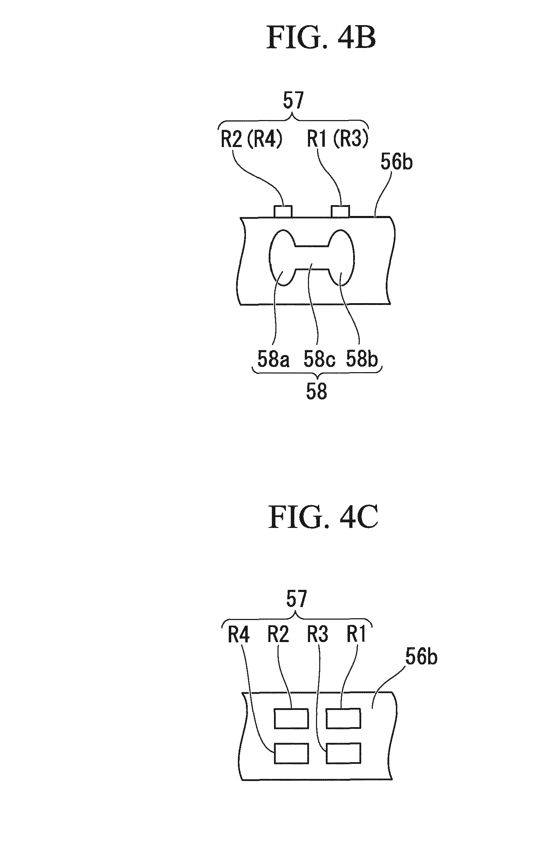

As shown in FIGS. 4A, 4B, and 4C, the substrate 56 as the elastic body specifically has the attaching portion (load-transmitting portion) 56a that is attached in a state where the attaching portion is inserted inward from a tip side of the first coupling arm 9a, the cantilever portion (actuating portion) 56b that extends in a horizontal direction from the attaching portion 56a, and the bearing portion (load-receiving portion) 56c that has the guide slit 12 formed on a tip side of the cantilever portion 56b. In addition, although the materials of the substrate 56 as the elastic body are not limited, for example, metals such as an aluminum alloy, iron, steel, and stainless steel, and other resins such as engineering plastic can be used.

Additionally, the cantilever portion 56b is provided with a hole portion 58 for constituting a Roberval mechanism. The hole portion 58 is configured to include a pair of circular holes 58a and 58b that are lined up horizontally in a length direction of the cantilever portion 56b in a state where these holes pass through the cantilever portion 56b in a thickness direction, and a communicating hole 58c that connects the centers of the pair of circular holes 58a and 58b together.

The strain sensor 57 is adhered to the cantilever portion 56b, and detects changes in resistance according to the magnitude of the strain caused in the cantilever portion 56b. The strain sensor 57 includes four strain gauges (strain-sensitive resistors) R1, R2, R3, and R4 in the illustrated example, and the strain gauges R1, R2, R3, and R4 are arranged side by side in pairs in a width direction of the cantilever portion 56b directly above positions where the pair of circular holes 58a and 58b of the cantilever portion 56b are formed.

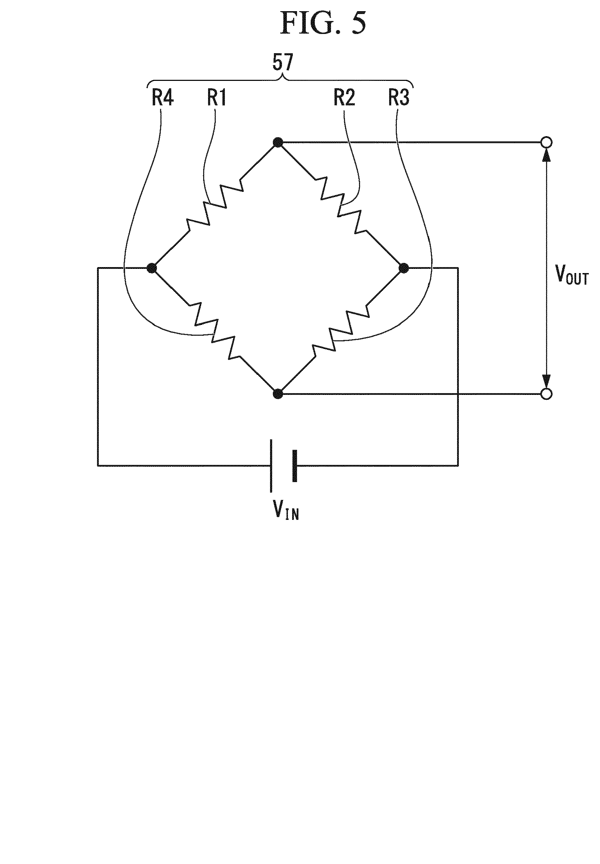

Additionally, the four strain gauges R1, R2, R3, and R4 constitute the Wheatstone bridge circuit as shown in FIG. 5. Among these strain gauges, R1 and R3 are strain gauges on a compression side, and R2 and R4 are strain gauges on a tension side. Also, this Wheatstone bridge circuit is enabled to output an output voltage V_{OUT} (load signal) according to the magnitude of the strain caused in the cantilever portion 56b with respect to an input voltage V_{IN} (constant).

In addition, the load cell 51 may have a configuration in which at least two or three strain gauges (strain-sensitive resistor) are arranged. In this case, one or two strain gauges among the strain gauges R1, R2, R3, and R4 constituting the Wheatstone bridge circuit shown in FIG. 5 may be substituted with a dummy resistor as a resistor that has no strain sensitivity.

Here, the bearing portion 56c of the substrate 56 has a recessed portion 56d that opens to a spindle 13 side so as to accommodate at least a portion of an outer peripheral surface of the spindle 13. An inner surface, particularly, a bottom surface of the recessed portion 56d receives the spindle 13 as a supporting surface 401, and is made so that the load from the bed surface-forming section 100 side, such as the bed plate 2, is applied thereto.

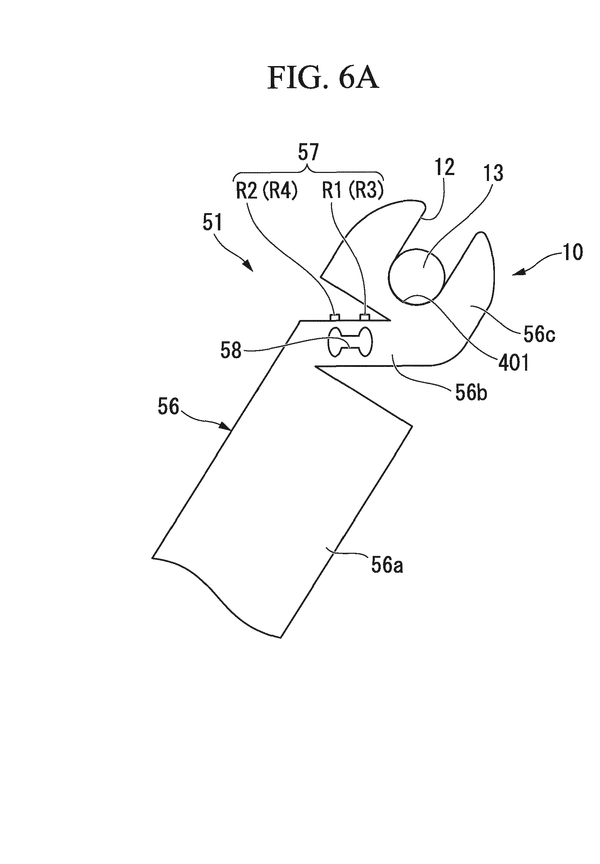

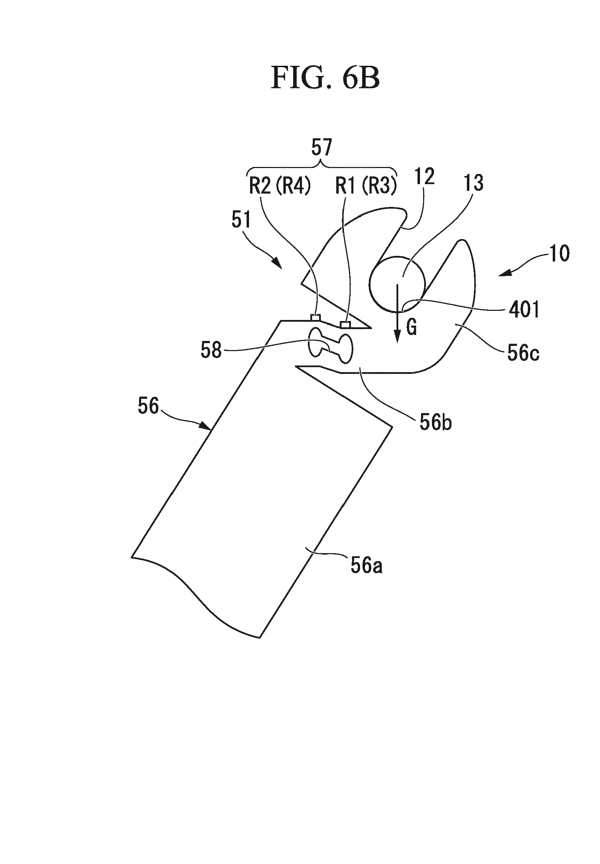

In such a load cell 51, as shown in FIGS. 6A and 6B, when a load is applied to the bed surface-forming section 100, such as the bed plate 2, a vertical downward load G is applied to the supporting surface 401 of the bearing portion 56c via the spindle (pin) 13 of the link mechanism 6. Moreover, as the load is applied to a tip portion of the cantilever portion (actuating portion) 56b that is continuously connected to the bearing portion 56c, strain is caused in the cantilever portion 56b. At this time, the strain sensor 57 detects changes in resistance according to the magnitude of the strain caused in the cantilever portion 56b, and outputs strain signals according to the magnitude of the strain caused in the cantilever portion 56b, that is, signals corresponding to changes in load. Also, a change in the load applied to the bed surface-forming section 100, such as the bed plate 2, can be detected by the load detector 50 including the load cell 51.

Additionally, in the load detector 50, changes in the loads applied to the four corners of the bed body 1A are detected by the four load cells 51 arranged at the four corners (the front left side, the front right side, the rear left side, and the rear right side) of the bed body 1A, respectively. Also, load signals detected by the four load cells 51 detected are output to the computing unit 52.

The computing unit 52 includes a computer that has a ROM, a RAM, other memories, a CPU, or the like, and has programs, numerical values, or the like required to calculate the state of the user H on the bed surface T of the bed body 1A stored in advance.

Also, in the computing unit 52, the state of the user H on the bed surface T of the bed body 1A is computed on the basis of the load signals output from the four load cells 51, and computation results are output to the transmitting unit 53.

For example, the computing unit 52 determines, from the load signals output from the four load cells 51, that the user H stays on the bed surface T of the bed body 1A when a total value of the loads applied to the four load cells 51 is greater than a threshold value stored in advance, and outputs the computation results to the transmitting unit 53.

In addition, the computing unit 52 is also enabled to perform the computation of predicting the getting-out-of-bed of the user H, for example, from the movement distance and/or movement speed of a center-of-gravity position of the user H on the bed surface T of the bed body 1A, in addition to the getting-into-bed (going-to-bed) and getting-out-of-bed (rising) of the user H. Moreover, it is possible to detect the body motions (examples: tossing about in bed or the like), postures (examples: lying on one's back, lying on one's stomach, lying on one's side, or the like), or the like of the user H through the computation, and it is also possible to predict occurrence of a bedsore as will be further described.

The transmitting unit 53, which is a transmitter attached to the bed body 1A, transmits the result computed by the computing unit 52 to the remote receiving unit 54. Meanwhile, the receiving unit 54, which is a receiver that receives a signal transmitted from the transmitting unit 53, is enabled to receive the signal from the transmitting unit 53 to thereby monitor the state of (situation while staying in bed) of the user H condition from a remote place.

Additionally, on the receiving unit 54 side, it is also possible to display the detection results detected by the load cells 51 and the computation results obtained by the computing unit 52, for example, on a monitor (not shown) or to output the results to a printer.

Additionally, for example, an observer may be notified of the state of the user H if necessary from the computation results obtained by the computing unit 52. Methods for the notification are not particularly limited. For example, it is possible to issue an alarm from a loudspeaker (not shown) or to display the alarm on a monitor.

The bed 1 with a load detection function having the structure as described above is preferably used in, for example, medical facilities (examples: hospitals, clinics, or the like), nursing facilities, child care institutions, or the like.

In the invention, it is possible to use such a bed 1 with a load detection function, thereby monitoring, for example, the state (situation while staying in bed) of the user H, such as getting into bed (going to bed), getting out of bed (rising), positions while staying in bed, body motions (examples: tossing about in bed or the like), postures (examples: lying on one's back, lying on one's stomach, lying on one's side, or the like) from a remote place. Additionally, it is possible to use such a bed 1 with a load detection function, thereby reducing the mental burden of the user H of being monitored by someone and the physical or mental burden of an observer that should monitor the user H constantly without being limited to not only midnight but also early morning.

In addition, such a bed 1 with a load detection function is not limited to being used in the above-described facilities (institutions). For example, such a bed is also available in lodging facilities (examples: hotels, inns, or the like), ordinary homes (examples: home care or the like), or the like. That is, the utility of the bed 1 with a load detection function is not particularly limited.

An application example using the load detection function of the bed 1 with a load detection function to which the invention is applied may include, for example, a "bedsore-preventing function". Specifically, when the user H does not move out of a constant circle with a center-of-gravity position beyond a certain period of time (for example, 2 hours) or when a load change in each load cell 51 does not occur beyond a certain value (for example, 1 kg), it is possible to add the function of determining that a bedsore may occur in the user H and notifying the observer of this possibility.

Additionally, another application example may include an "illumination control function". Specifically, by measuring the presence/absence of weight, a center-of-gravity position, the movement distance of a center of gravity, the movement speed of the center of gravity, or the like regarding the user H on the bed surface T of the bed body 1A, it is possible to add the function of turning on or turning off illumination when the user gets into bed or gets out of bed.

Additionally, still another application example may include a "weight control function". Specifically, when periodically measuring the weight of the user H on the bed surface T of the bed body 1A (for example, at a fixed time every day), it is possible to add the function to perform the weight control of the user H.

Additionally, a still further application example may include an "air-conditioning control function". Specifically, when detecting the body motions (tossing about in bed or the like) of the user H on the bed surface T of the bed body 1A, it is possible to add the function of measuring the sleep depth of the user H and managing air-conditioning according to the state of the user.

Additionally, a still further application example may include a "weight-monitoring function during dialysis". Specifically, when measuring the weight of the user H on the bed surface T of the bed body 1A, it is possible to add the function of detecting the start and end of dialysis.

In this way, the invention is not limited to the above-described functions, and it is also possible to use the load detection function of the bed 1 with a load detection function to add various functions.

Additionally, a bed with a load detection function of the load detector 50 to which the invention is applied may be incorporated into the bed body 1 A in advance or be obtained by separately incorporating the load detector 50 to which the invention is applied into the bed body 1A, thereby adding the load detection function to an existing bed.

That is, a bed with a load detection function to which the invention is applied is enabled to measure a change in the load applied to the bed body 1A, using the load detector 50 attached in advance or separately attached to the bed body 1A, thereby detecting the state of the user H on the bed surface T of the bed body 1 A.

Additionally, in the invention, by attaching to the bed body 1A of the load detector 50 to which the invention is applied, it is possible to add the load detection function to the bed through a simple structure while suppressing an increase in the number of parts.

Specifically, in the load detector 50 to which the invention is applied, the load cell 51 constitutes a load-detecting part that can be replaced with a part (bearing member formed with the guide slit (bearing) 12) that constitutes the first hinge portion 10 of the first coupling arm 9a that the existing bed has.

For this reason, it is preferable that the substrate 56 as the elastic body constituting the load cell 51 have an attachment structure in which the attaching portion 56a and the bearing portion 56c are the same as those of a bearing member of the existing bed.

In that case, simply by replacing four bearing members arranged at four corners (the front left side, the front right side, the rear left side, and the rear right side) of the existing bed body 1A with the load cells 51, it is possible to easily incorporate the load detector 50 into the existing bed body 1A.

Accordingly, it is possible to add the load detection function to the existing bed cheaply. Additionally, even when a failure or the like occurs in the load cell 51, replacement is easily possible. Moreover, since there is little difference from the existing bed, it is possible for the user H to use the bed without feeling uncomfortable.

In addition, the load cell 51 is not necessarily limited to the above example, and it is possible to add various changes without departing from the scope of the invention.

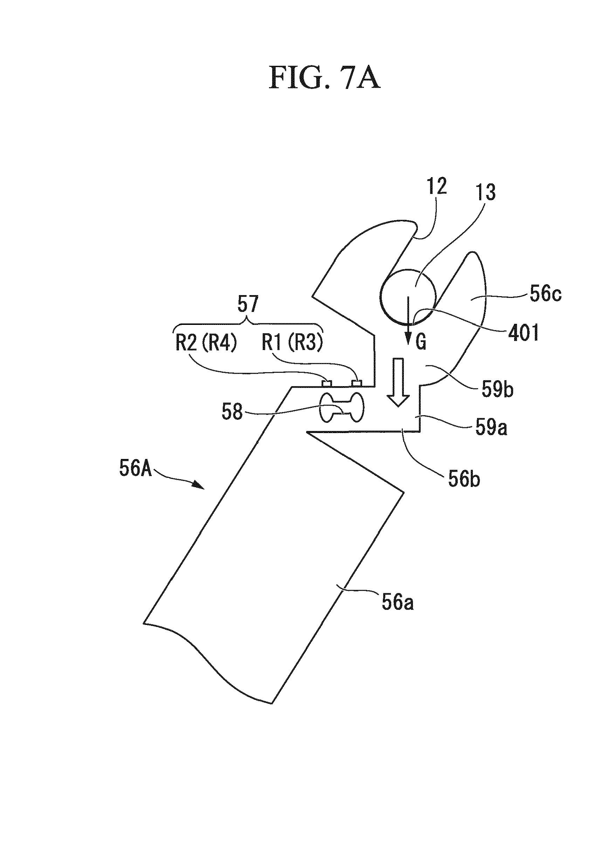

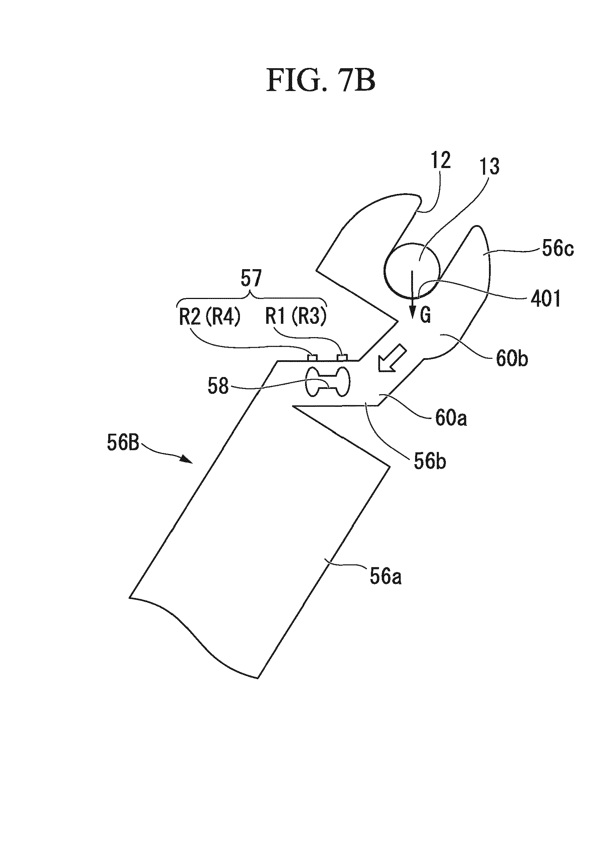

For example, the load cell 51 is able to have a configuration including a substrate (elastic body) 56B, for example, as shown in FIG. 7A, or is able to have a configuration including a substrate (elastic body) 56A as shown in FIG. 7B. Specifically, the strain bodies 56A and 56B have the same configuration as the elastic body 56 shown in FIG. 4A, except that the shape of a cantilever portion (actuating portion) 56b is different.

Out of these substrates, the substrate (elastic body) 56A shown in FIG. 7A has a first extension portion 59a that extends in a horizontal direction from the attaching portion (load-transmitting portion) 56a, and a second extension portion 59b that extends vertically upward from a tip side of the first extension portion 59a, the bearing portion (load-receiving portion) 56c is provided on a tip side of the second extension portion 59b, and the first extension portion 59a has the same configuration as the aforementioned cantilever portion (actuating portion) 56b.

Meanwhile, the substrate (elastic body) 56B shown in FIG. 7B has a first extension portion 60a that extends in a horizontal direction from the attaching portion (load-transmitting portion) 56a, and a second extension portion 60b that extends obliquely upward from a tip side of the first extension portion 60a, the bearing portion (load-receiving portion) 56c is provided on a tip side of this second extension portion 60b, and the first extension portion 60a has the same configuration as the aforementioned cantilever portion (actuating portion) 56b.

Additionally, the bed 1 with a load detection function has the configuration in which the load cell 51 is incorporated into the first hinge portion 10a that constitutes the lifting link mechanism 6. However, it is also possible to adopt not only such a configuration but also, for example, adopt a configuration in which the load cell 51 is incorporated into the second hinge portion 10b and the third hinge portion 10c constituting the lifting link mechanism 6.

Although the load cell 51 has a configuration in which the strain gauge (strain-sensitive resistor) 57 is used as the strain sensor that detects the magnitude of strain, the load cell is not limited to such a strain-sensitive resistor. For example, a conductive elastomer sensor, an optical strain sensor, an electrostrictive device sensor, a piezoelectric device sensor, a magnetostrictive device sensor, or the like can be used as the strain sensor.

Additionally, the bed body 1A may be obtained by laying a mattress or the like in advance on the bed plate 2. Additionally, the bed plate 2 may have the structure in which the bed plate is split in a length direction (longitudinal direction of the bed body 1 A) thereof, and may have a reclining function in which a portion of the user H on an upper body side or a leg side rises. Moreover, as for the top frame 3 and the bottom frame 5, it is possible to adopt not only the above-described frame structure but also various frame structures.

Additionally, the load detector 50 may adopt the configuration in which electrical connection is made between the above-described load cell 51 and the computing unit 52 and between the above-described computing unit 52 and transmitting unit 53 by the wiring lines 55a and 55b but also may have a configuration in which electrical connection is made by radio. Meanwhile, as methods for communication between the transmitting unit 53 and the receiving unit 54, not only the method using the above-described radio communication network, but also a method using a wire communication network may be used. Moreover, in the load detector 50, it is also possible to integrally form the computing unit 52 and the transmitting unit 53.

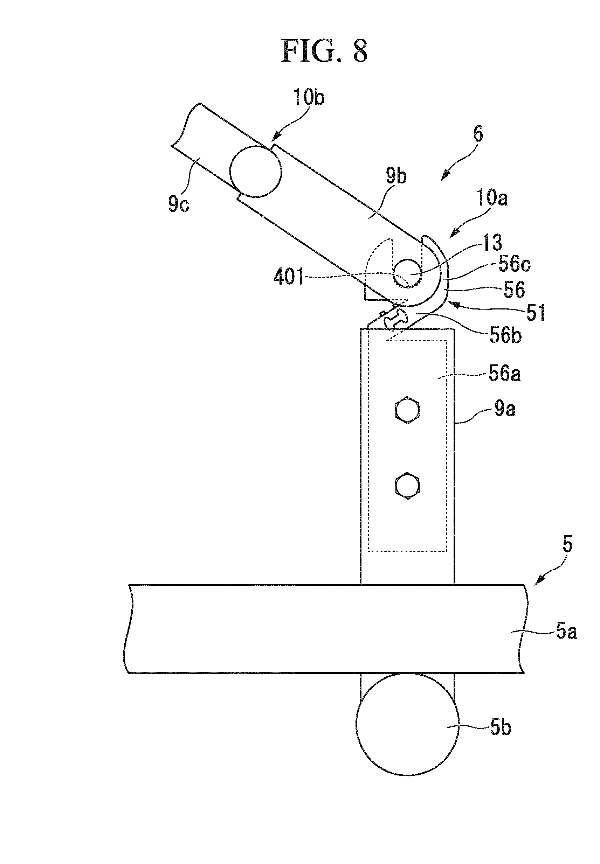

Moreover, in the aforementioned embodiment, the first coupling arm 9a in the lifting link mechanism 6 is fixed to the pair of front and rear pipe frames 5b of the bottom frame 5 so as to incline with respect to the bottom frame 5 (refer to FIG. 3A). However, the first coupling arm 9a may be fixed so as to become vertical with respect to the bottom frame 5. The situation in that case is shown in FIG. 8 pursuant to FIG. 3A.

As shown in FIG. 8, even when the first coupling arm 9a of the lifting link mechanism 6 is vertical, a load can be detected similar to the above by fixing the load cell 51 to the coupling arm 9a so that the whole load cell runs along a vertical direction, and by receiving the pin (spindle) 13 provided in the second coupling arm (the other coupling arm) 9b using the supporting surface 401 of the bearing portion 56c.

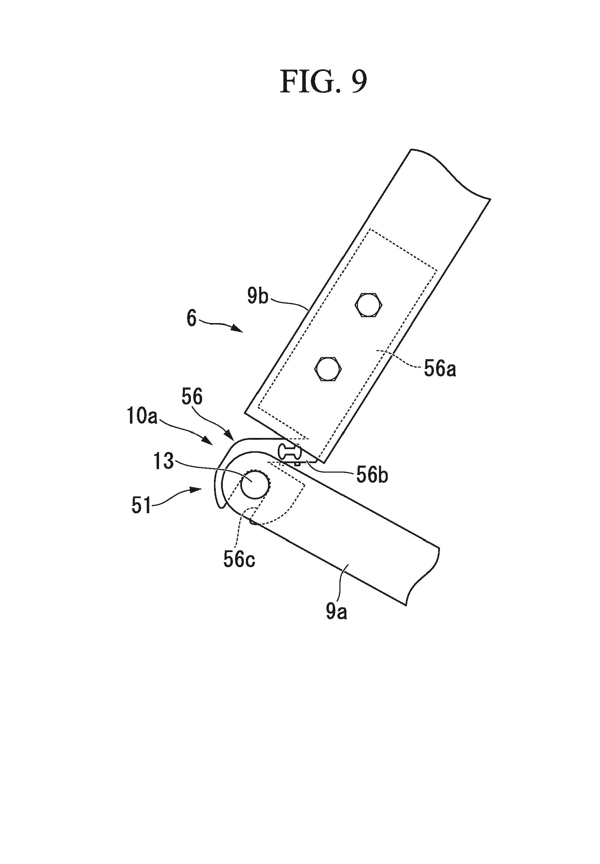

Additionally, in the aforementioned respective examples, the load cell 51 is incorporated into the bed body so that the bearing portion (load-receiving portion) 56c thereof is located on the upper side and the attaching portion (load-transmitting portion) 56a is located on the lower side. However, it goes without saying that the relationship between the load-receiving portion and the load-transmitting portion is relative, and these portions may be reversed upside down and incorporated into the bed body. That is, the attaching portion 56a may be located on the upper side as the load-receiving portion, and the bearing portion 56c may be located on the lower side as the load-transmitting portion. An example in that case is shown in FIG. 9.

In FIG. 9, an upper end of the first coupling arm 9a of the lifting link mechanism 6 and a lower end of the second coupling arm 9b are rotatably attached to each other via the first hinge portion 10a, and the pin (spindle) 13 of the first hinge portion 10a is provided to the lower first coupling arm 9a side. Also, the bearing portion 56c of the substrate (elastic body) 56 in the load cell 51 is engaged with the pin 13, and the attaching portion 56a of the substrate (elastic body) 56 in the load cell 51 is fixed to the upper second coupling arm 9b. Even in a case such as this, although the load of the bed body is applied to the attaching portion 56a of the substrate (elastic body) 56, a load can be detected depending on the strain of the cantilever portion 56b.

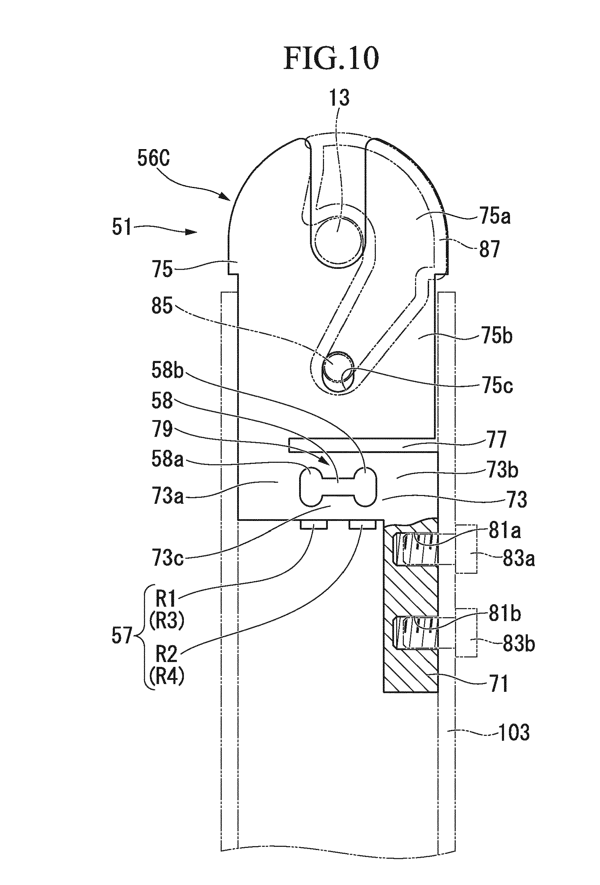

Another example of the load cell 51 incorporated into the bed body in the invention is shown in FIG. 10.

In FIG. 10, a substrate 56C as the elastic body in the load cell 51 is constituted by a load-transmitting portion 71, an actuating portion 73, and a load-receiving portion 75. The load-transmitting portion 71 corresponds to the attaching portion 56a in shown in the substrate 56 shown in FIG. 4A, or the substrate 56A or the substrate 56B (hereinafter, the substrate in FIGS. 7A or 7B is referred to as a substrate of each example for convenience) shown in FIG. 7A or FIG 7B, the actuating portion 73 is equivalent to the cantilever portion 56b similarly in the substrate of each example, and the load-receiving portion 75 corresponds to the bearing portion 56c in the substrate of each example.

In the substrate 56C shown in FIG. 10, the load-receiving portion 75 is constituted by the same bearing portion 75a as the bearing portion 56c in the substrate of each example, and an extension portion 75b that integrally extends from the lower side of the bearing portion 75a. The extension portion 75b has, for example, a rectangular shape as viewed in a vertical cross-section, and has a through-hole 75c formed nearly at the center thereof so as to penetrate in the horizontal direction. Moreover, the actuating portion 73 is constituted by a cantilever (cantilever beam) that extends along the horizontal direction, and has one end 73a continuously integrated with one end side of a lower surface of the extension portion 75b in the load-receiving portion 75. Accordingly, a cutout portion 77 cut in in the horizontal direction from a side is present between the extension portion 75b in the load-receiving portion 75 and the actuating portion 73.

Also, the other end 73b of the actuating portion 73 having the cantilever configuration is continuously integrated with an upper end of the load-transmitting portion 71. Additionally, an elongated hole-like hole portion 58 running along the horizontal direction (running along a length direction of the actuating portion 73) is formed in an intermediate portion 73c of the actuating portion 73 having the cantilever configuration so as to penetrate in the horizontal direction, and enlarged-diameter portions 58a and 58b are formed at both ends of the elongated hole-like hole portion 58. Accordingly, the hole portion 58 constitutes a Roberval mechanism 79. Meanwhile, the load-transmitting portion 71 is formed in the shape of a lengthwise rectangular thick plate or in the shape of a diagonal bar that extends along the vertical direction, and the upper end thereof is continuously integrated with the other end 73b of the actuating portion 73 as described above.

Also, a plurality (two in the illustrated example) of screw holes 81a and 81b are drilled from a side surface side at positions spaced apart in an up-and-down direction in the load-transmitting portion 71. Additionally, the strain gauges R1, R2, R3, and R4 constituting the strain sensor 57 are attached to the positions corresponding to the enlarged-diameter portions 58a and 58b of the Roberval mechanism 79 in a lower surface or an upper surface (lower surface in FIG. 10) of the actuating portion 73.

In incorporating such a load cell 51 shown in FIG. 10 into the bed body, as shown by chain lines in FIG. 10, the portion of the load-receiving portion 75 of the substrate 56C below the extension portion 75b is inserted into the hollow tube portion 103 (for example, the first hollow coupling arm 9a in the aforementioned lifting link mechanism) in the bed body from an upper end thereof, screws 83a and 83b are threaded into the screw holes 81a and 81b of the load-transmitting portion 71 from the outside via the attachment holes 81a and 81b formed in advance in the hollow tube portion 103, and thereby the load-transmitting portion 71 of the substrate 56C is fixed to the hollow tube portion 103 in the bed body. Also, a load application member (for example, the pin (spindle) 13 provided in the second coupling arm 9b in the aforementioned lifting link mechanism) in the bed body is engaged with the bearing portion 75a in the load-receiving portion 75.

In addition, in this case, similarly, as shown by the chain line in FIG. 10, it is desirable to adopt a configuration in which a shaft rod-like coupling pin 85 having a smaller diameter than the through-hole 75c is inserted through the through-hole 75c of the extension portion 75b in the load-receiving portion 75 with play, a coupling hook member 87 made of an elastic material is engaged with the load application member (for example, pin (spindle) 13) and the coupling pin 85, and a resilient force in a direction in which the load application member (for example, pin (spindle) 13) and the coupling pin 85 are brought close to each other is applied to these pins by the coupling clip member 87. By adopting such a configuration, a state where the load application member (for example, the pin (spindle) 13) abuts against the bearing portion 75a in the load-receiving portion 75 of the substrate 56C can be always maintained to prevent rattling from occurring in the substrate 56C.

If a vertically downward load is applied to the bearing portion 75a in the load-receiving portion 75 in a state where the substrate 56C of the load cell 51 is incorporated into the bed body in this way, a force in a direction in which the load-receiving portion 75 is lowered is exerted on the load-receiving portion. Meanwhile, since the load-transmitting portion 71 of the substrate 56C is fixed to the hollow tube portion 103 (for example, the first coupling arm 9a) on the bed body side, the actuating portion 73 having the cantilever configuration is deflected and deformed as a cantilever beam, strain (tension strain/compression strain) occurs on the lower surface and upper surface of the actuating portion, the strain sensor 57 including the strain gauges R1, R2, R3, and R4 detects the strain like the aforementioned respective examples, and consequently, a load W is detected.

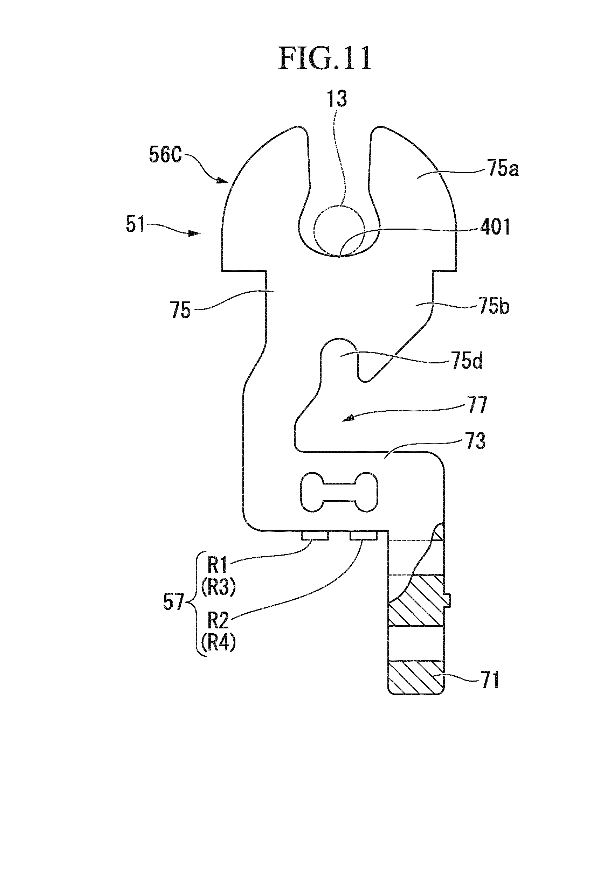

The above-described load cell 51 shown in FIG. 10 has a configuration in which the whole substrate 56C including the load-transmitting portion 71, the actuating portion 73, and the load-receiving portion 75 is integrally and continuously made using the same material, and another example of the load cell 51 in a case where the whole substrate 56C is continuously integrated in this way is shown in FIG. 11.

The substrate 56C of the load cell 51 shown in FIG. 11 is substantially the same as the substrate 56C shown in FIG. 10 in terms of components other than the load-receiving portion 75. Also, the load-receiving portion 75 of the substrate 56C shown in FIG. 11 is constituted by the bearing portion 75a, and the extension portion 75b that integrally extends from the lower side of the bearing portion 75a, the cutout portion 77 between the extension portion 75b and the cantilever (cantilever beam)-like actuating portion 73 is cut in the shape of a substantially triangular shape from a side, and a portion equivalent to the through-hole 75c in FIG. 10 serves as a recessed portion 75d that is recessed from an inclined surface of the cutout portion 77. In the load cell 51 to which such a substrate 56C is applied, strain can be also detected substantially similarly to the load cell shown in FIG. 10.

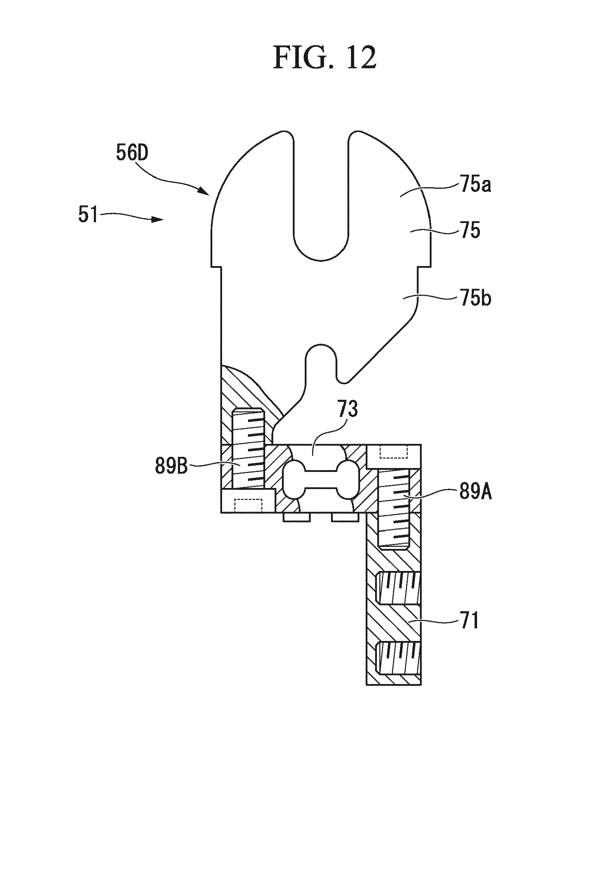

The example shown in the above-described FIG. 10 or 11 has a configuration in which the whole substrate 56C including the load-transmitting portion 71, the actuating portion 73, and the load-receiving portion 75 are integrally and continuously made using the same material. However, depending on the case, a substrate may be configured so as to be split into two pieces or three pieces, and the respective split pieces may be configured so as to be coupled together by proper coupling and anchoring means, such as screw-stopping or welding, and brazing. Examples thereof are shown in FIGS. 12 to 14.

A substrate 56D of the load cell 51 shown in FIG. 12 has a configuration in which the load cell is split into three pieces by splitting the load-transmitting portion 71, the actuating portion 73, and the load-receiving portion 75 therebetween, respectively.

In FIG. 12, the overall shape of the substrate 56D is the same as that of the substrate 56C shown in FIG. 10. However, the load-transmitting portion 71, the actuating portion 73, and the load-receiving portion 75 are separately made, respectively, the load-transmitting portion 71 and the actuating portions 73 are coupled together by a screw 89A, and the actuating portion 73 and the load-receiving portion 75 are coupled together by a screw 89B.

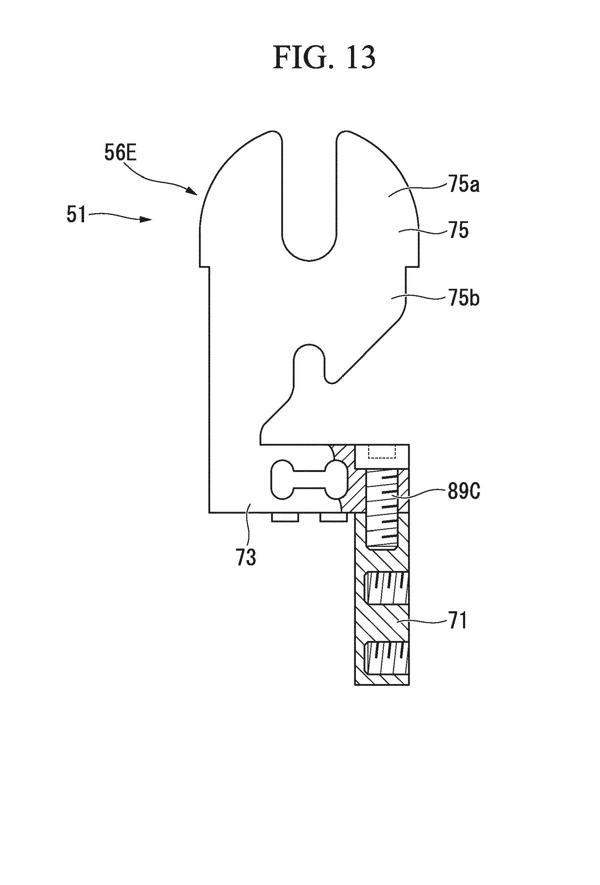

Moreover, the substrate 56E of the load cell 51 shown in FIG. 13 has a configuration in which the load-receiving portion 75 and the actuating portion 73 are integrally made, the load-transmitting portion 71 is made separately from the load-receiving portion 75 and the actuating portion 73, and the actuating portion 73 and the load-transmitting portion 71 are coupled together by a screw 89C, that is, has a two-piece split configuration.

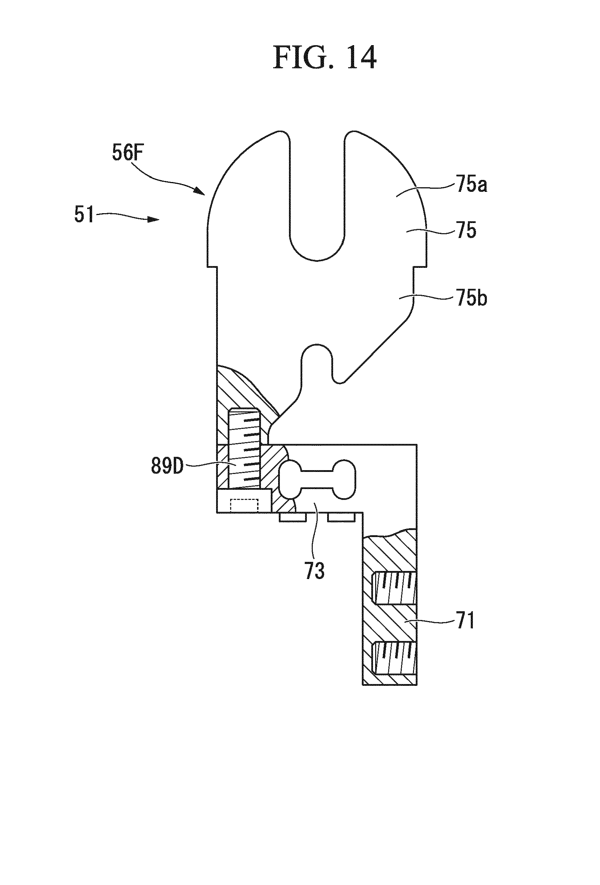

Moreover, a substrate 56F of the load cell 51 shown in FIG. 14 has a configuration in which the load-transmitting portion 71 and the actuating portion 73 are integrally made, the load-receiving portion 75 is made separate from the load-transmitting portion 71 and the actuating portion 73, and the load-receiving portion 75 and the actuating portions 73 are coupled together by a screw 89D, that is, is configured to be split into two pieces.

When the substrate constituting the load cell 51 is split as described above, it is possible to select an optimal material as a structural material of each split portion according to the required characteristics of each split portion in the substrate, for example, the desired workability, yield strength, elongation, or the like of each portion. That is, the load-receiving portion 75 is a portion that receives the load from the load application member and applies a force caused by the load to the actuating portion 73 and that does not directly contribute to occurrence of strain for load detection. Therefore, in short, the load-receiving portion is better if workability is excellent so that the load-receiving portion can be easily machined in an optimal shape that can be machined in order to receive the load from the load application member, and is better even if elongation or yield strength is not so much taken into consideration. Meanwhile, since the actuating portion 73 is a portion that is deflected and deformed by a force given from the load-receiving portion 75, it is desirable that yield strength be high and elongation be small. Moreover, the load-transmitting portion 71 may be a portion of a structure for being fixed to and supported by a member of the bed body. The yield strength may be lower than in the actuating portion 73, but it is desired to have a certain amount of yield strength as the support structure, and it is desired that the elongation be small to some extent.

Here, as the materials of the substrate constituting the load cell 51, metals such as an aluminum alloy, iron, steel, and stainless steel, and other resins such as engineering plastic can be used. Also, when the substrate is configured to be split, as the material of each split portion, an optimal combination may be selected from among these materials.

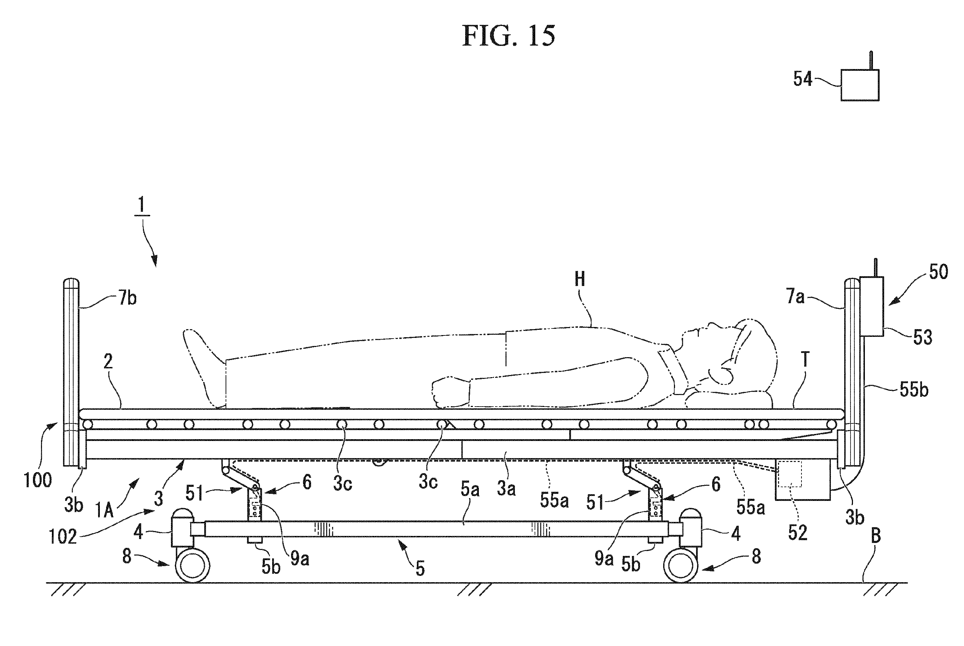

Moreover, a load cell 51 different from the above respective examples, and a head incorporated into the load cell will be described with reference to FIGS. 15 to 20D. In addition, in these drawings, the same elements as the elements shown in the respective drawings that are already described will be designated by the same reference numerals of the respective drawings that are already described, and the detailed description thereof will be omitted.

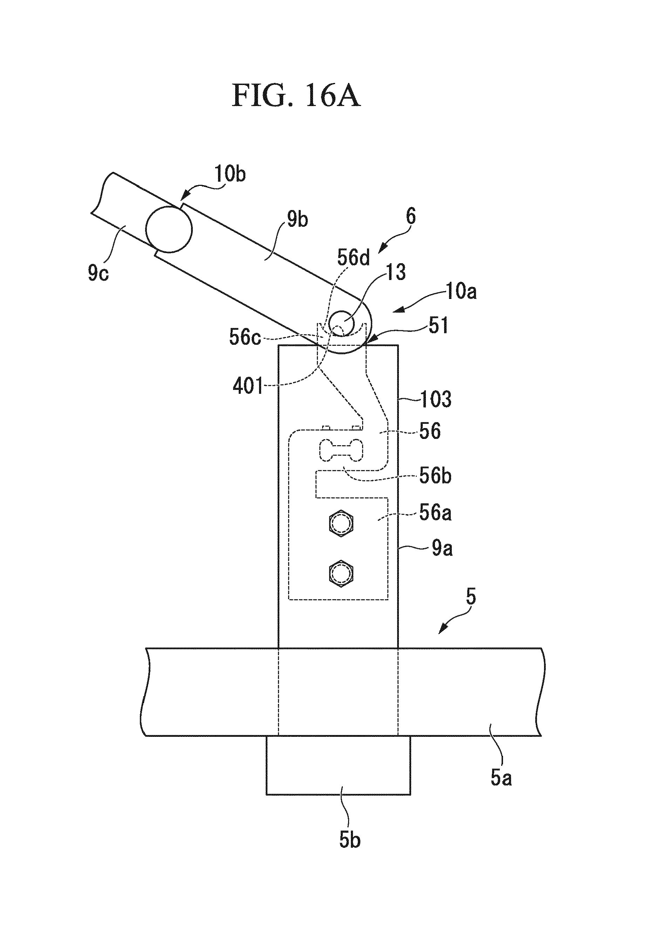

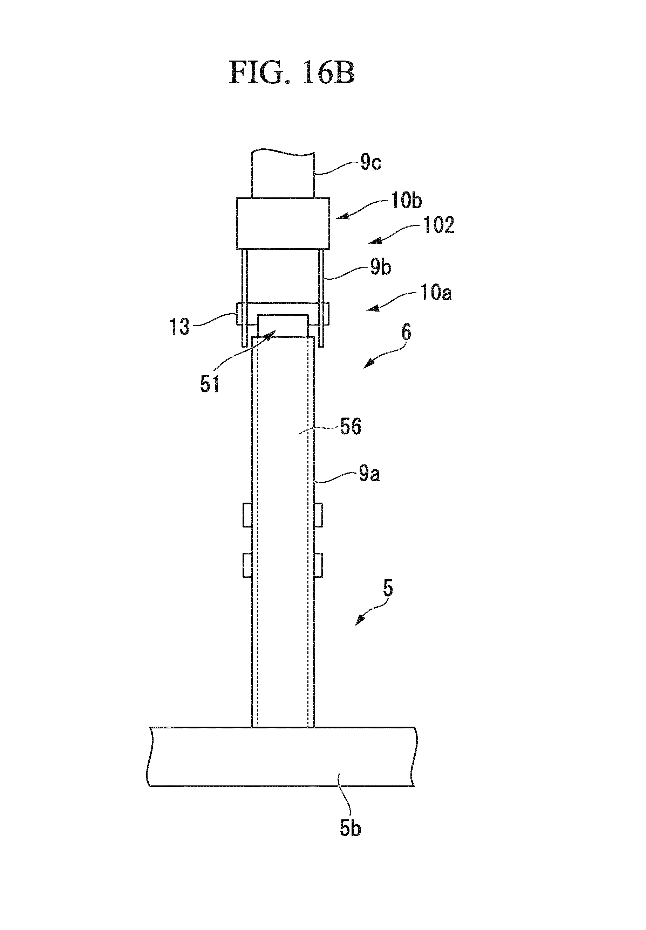

As shown in FIGS. 15, 16A, and 16B, the first coupling arms 9a constituting the lifting link mechanisms 6 are attached so as to become nearly vertical in a state where the lower ends thereof are fixed to the pair of front and rear pipe frames 5b constituting the bottom frame 5. In addition, the hollow tube portion 103 is formed at least on the upper end side (the side far from the bottom frame 5) of the first coupling arm 9a.

Also, as shown in FIGS. 16A and 16B, the load cell is inserted into the hollow tube portion 103 of the first coupling arm 9a, for example, from above, and is fixed to the coupling arm 9a. Also, the supporting surface 401 of the bearing portion 56c in the load cell 51 has the structure in which the load from the bed plate 2 can be detected by receiving the substantially horizontal pin (spindle) 13 provided at the end of the second coupling arm 9b.

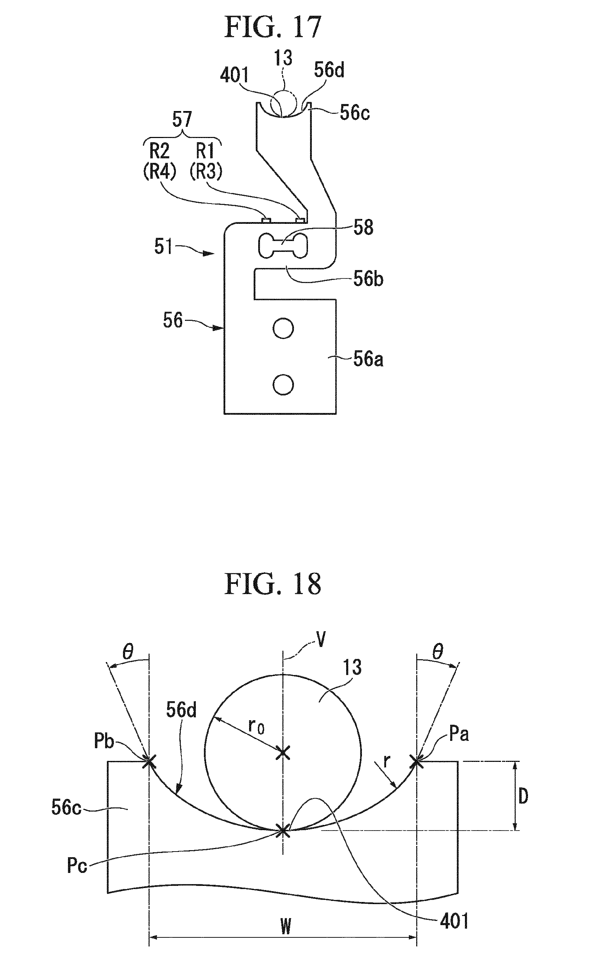

Similar to those already described, the load cell 51 basically has the substrate 56 that generates strain according to the load from the bed surface-forming section 100 side, such as the bed plate 2, and the strain sensor 57 that is attached to the substrate 56 in order to detect the strain of the substrate (refer to FIG. 17A).

The substrate 56 has on one end side the bearing portion 56c formed as the load-receiving portion that receives the load from the bed surface-forming section 100 and has on the other end side the attaching portion 56a to be described below formed as the load-transmitting portion that transmits the load to a structural member on the installation surface side in the bed body. The cantilever portion 56b to be described below is formed between the load-receiving portion (bearing portion 56c) and the load-transmitting portion (attaching portion 56a) as the actuating portion that is deflected by the load, and the strain sensor 57 is attached to the actuating portion (cantilever portion 56b). Also, the bearing portion 56c as the load-receiving portion constitutes a bearing of the first hinge portion 10a. Moreover, the attaching portion 56a as the load-transmitting portion is nearly vertical in the hollow tube portion 103 of the first coupling arm 9a along the length direction of the first coupling arm 9a, and the substrate 56 is attached to the first coupling arm 9a by screw-stopping.

The cantilever portion 56b is provided with the hole portion 58 for constituting the Roberval mechanism. Additionally, the strain sensor 57 is adhered to the cantilever portion 56b. The strain sensor 57, similar to the case of FIGS. 4A to 4C already described, includes, for example, the four strain gauges (strain-sensitive resistors) R1, R2, R3, and R4, and constitutes the same Wheatstone bridge circuit shown in FIG. 5.

Here, the bearing 56c of the substrate 56 has the recessed portion 56d that opens to the spindle 13 side so as to accommodate at least a portion of the outer peripheral surface of the spindle 13. The inner surface, particularly, a bottom surface of the recessed portion 56d receives the spindle 13 as the supporting surface 401, and is made as, for example, a curved surface so that the load from the bed surface-forming section 100 side, such as the bed plate 2, is applied thereto.

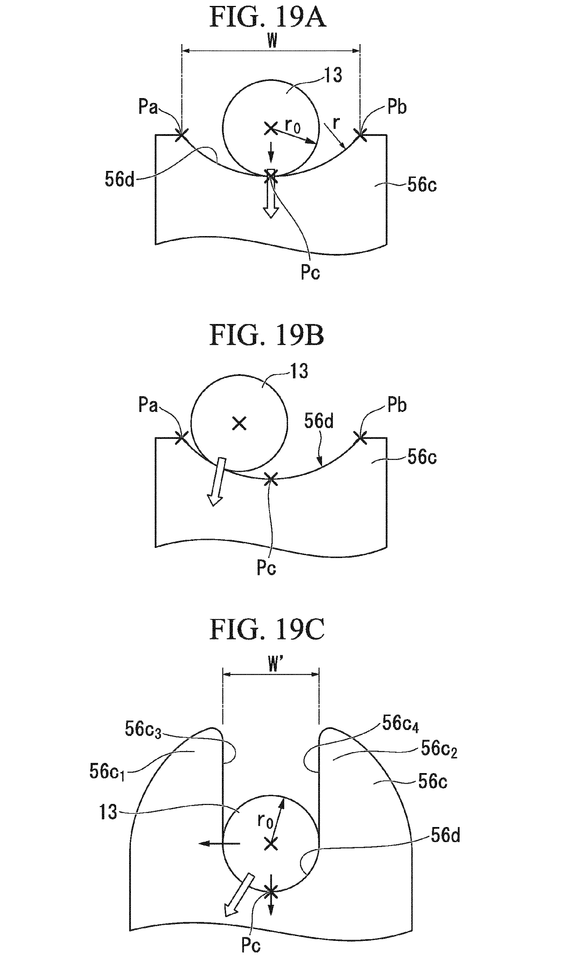

Here, the preferable shape and dimensions of the recessed portion 56d of the bearing portion 56c in the substrate 56 of the load cell 51 shown in FIGS. 16A, 16B, and 17 will be described in more detail with reference to FIGS. 19A and 19B. In addition, FIG. 19C shows shape and dimensions for comparison with the above preferable shape and dimensions.

As shown in FIG. 18, basically, the inner surface of the recessed portion 56d is formed as a surface that is continuously connected to a curvature radius r equal to or more than the radius r₀ of the spindle 13 from a central portion Pc of the recessed portion 56d to both ends Pa and Pb, when viewed in a cross-section orthogonal to an axis of the spindle 13. Moreover, an angle (an angle in a direction in which the recessed portion opens to the outside) θ, at which the inner surface of the recessed portion 56d is formed with respect to a plane (for example, a vertical plane) V passing through the axis of the spindle 13 and a central portion Pc of the recessed portion 56d in a portion ranging from the central portion Pc of the recessed portion 56d to both the ends Pa and Pb, is formed so as not to reach 0° (that is, so as not to be parallel). For example, as shown in FIG. 18, it is meant that, even if the recessed portion 56d is formed so that the curvature radius r of the inner surface thereof becomes gradually large from the center Pc of the recessed portion 56d toward both the ends Pa and Pb, the inner surface angle θ at both the ends Pa and Pb whose curvature radius r becomes the largest does not reach zero. Moreover, the recessed portion 56d is adapted such that the width W between both the ends Pa and Pb is set to a dimension exceeding twice the radius r₀ of the spindle 13 and a clearance is not generated between the outer peripheral surface of the spindle 13 in the recessed portion 56d and both the ends Pa and Pb of the recessed portion 56d.

The reason why the preferable shape and dimensions of the recessed portion 56d of the bearing portion 56c are determined as described above is as follows.

That is, for example, as shown in FIG. 19C for comparison, portions 56c₁ and 56c₂ at both left and right ends of the bearing portion 56c protruded upward largely so as to sandwich the spindle 13 from both sides of the spindle, and the inner surfaces of the recessed portion 56d on both sides are formed as wall surfaces (vertical wall surfaces) 56c₃ and 56c₄ that are parallel to the plane V passing through the axis O of the spindle 13 and the central portion Pc of the recessed portion 56d. Moreover, when the width (the width between the vertical wall surfaces 56c₃ and 56c₄) W' between the portions 56c1 and 56c2 at both the left and right ends of the bearing portion 56c is substantially equal to twice the radius r₀ of the spindle 13, there is a concern that load detection precision may become low if a load offset toward the bed surface 3 of the bed body 1A is applied. Specifically, when a user sits on an end of the bed surface 3 or a user who is sleeping on the bed surface 3 greatly tosses about in bed to an end side of the bed surface 3 and the load applied to the bed surface 3 is largely offset (hereinafter, such a state is referred to as an offset load state), the bed body 1A is strained, though slightly.

At this time, the spindle 13 tends to move towards the direction (lateral direction) of the strain, and a force in a lateral direction is applied to any of the left and right vertical wall surfaces 56c₃ and 56c₄ of the recessed portion 56d of the bearing portion 56c. This means that a force component different from a vertical load (force) that is originally intended to be detected by the load cell 51 is applied to the substrate 56 of the load cell 51. Therefore, in the offset load state, not only the strain caused by the force in the vertical direction but also the strain caused by the lateral force may be superimposed on each other. As a result, there is a concern that the load in the vertical direction cannot be precisely detected and the load detection precision may deteriorate in the actuating portion (cantilever portion) 56b of the substrate 56.

In contrast, as shown in FIG. 18, when the shape and dimensions of the recessed portion 56d of the bearing portion 56c are determined, the load in the vertical direction can be more precisely detected even in the offset load state. That is, for example, as shown in FIGS. 19A and 19B, if the spindle 13 tends to move in the lateral direction depending on the offset load state, the movement of the spindle 13 in the lateral direction is allowed by the clearance S on the side surface side of the spindle 13, and the surface on a side where the spindle 13 touches is not a vertical wall surface. Therefore, the lateral force applied to the surface of the side where the shaft 23 touches is reduced. Therefore, even in the offset load state, the strain of the actuating portion (cantilever portion) 56b of the substrate 56 caused by the lateral force also becomes smaller. As a result, the load in the vertical direction can be more precisely detected.

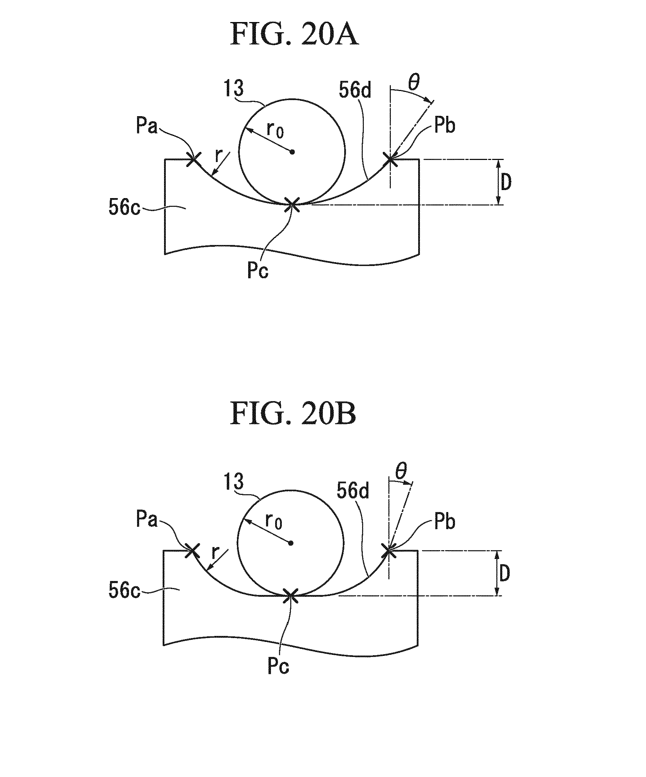

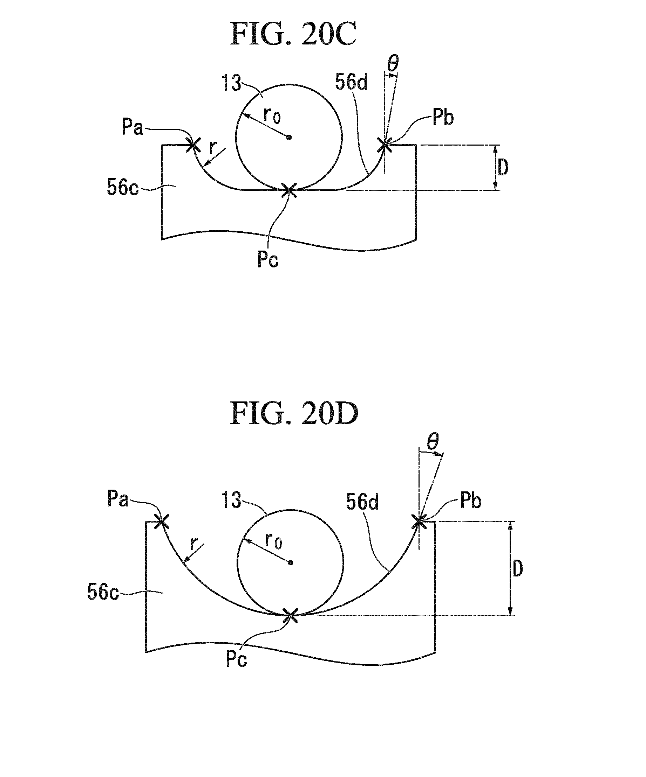

In addition, here, the expression "the inner surface of the recessed portion 56d is formed as a surface that is continuously connected to a curvature radius r equal to or greater than the radius r₀ of the spindle 13 from a central portion Pc of the recessed portion 56d to both ends Pa and Pb, when viewed in a cross-section orthogonal to an axis of the spindle 13" does not mean that all between both the ends Pa and Pb should be continuously connected to a curved surface but includes, for example, a case where the vicinity of the central portion Pc is a plane (that is, a case where the curvature radius in the vicinity of the central portion Pc is infinite). Some typical examples of the inner surface shape of the recessed portion 56d are shown in FIGS. 20A, 20B, 20C, and 20D. FIG. 20A shows an example in which the curvature radius r becomes gradually larger from the central portion Pc of the recessed portion 56d to both the ends Pa and Pb. Additionally, FIG. 20B shows an example in which the vicinity of the central portion Pc is a plane and the curvature radius r becomes gradually larger from the region of the plane toward both the ends Pa and Pb. Moreover, FIG. 20C shows an example in which the vicinity of the central portion Pc is a plane and the portions from the region of the plane to both the ends Pa and Pb are curved with a constant curvature radius r. Moreover, FIG. 20D shows an example in which the portions from the central portion Pc to both the ends Pa and Pb are curved with a constant curvature radius r. Of course, in any case of FIGS. 20A to 20D, it is desirable that the maximum value of the curvature radius r of the inner surface of the recessed portion 56d be smaller than the radius r₀ of the spindle 13.

In addition, the depth D (equivalent to the height from the bottom surface of the central portion Pc in the recessed portion 56d to the positions of both the ends Pa and Pb, that is, the height from the horizontal surface passing through the inner surface central portion Pc in the recessed portion 56d to the positions of both the ends) of the recessed portion 56d is not particularly limited, and is allowed so as to be also greater than the radius r₀ of the spindle 13, for example, particularly as shown in FIG. 20D. Here, if the depth D becomes large, in order to satisfy the inclination condition (θ > 0) of both the ends Pa and Pb, the width W between both the ends Pa and Pb of the recessed portion 56d should often be made large. Accordingly, if the depth W is excessively large, there is a concern that the substrate 56 may be enlarged and incorporation of the substrate into the bed body 1A may become difficult. Therefore, usually, it is desirable that the depth D be less than twice the radius r₀ of the spindle 13, and more preferably, it is preferable that the depth have a value smaller than the radius r₀ of the spindle 13. Moreover, the range where the depth D is optimal is within a range of 0.2 times to 0.8 times the radius r₀ of the spindle 13.

Moreover, although the upper limit of the width W between both the ends Pa and Pb of the recessed portion 56d is not particularly determined, similar to the above, it is desirable that the upper limit be equal to or less than 20 times the radius r₀ of the spindle 13 from a viewpoint of miniaturization. More preferably, the width W is set to be within a range of 2.4 times to 10 times the radius r₀ of the spindle 13.

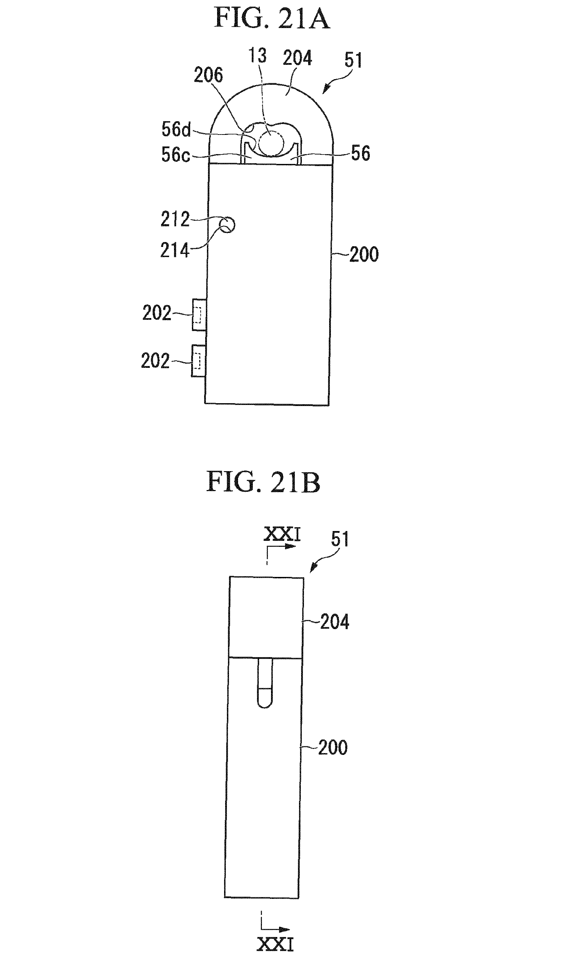

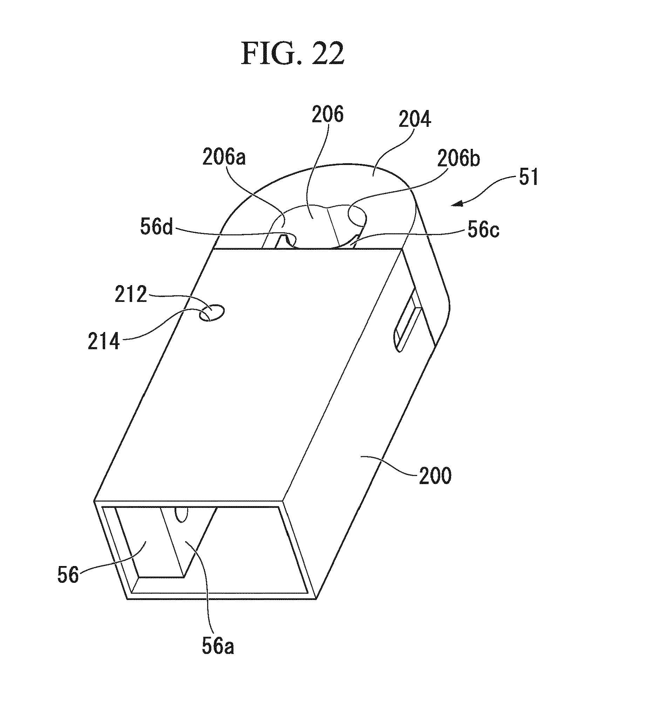

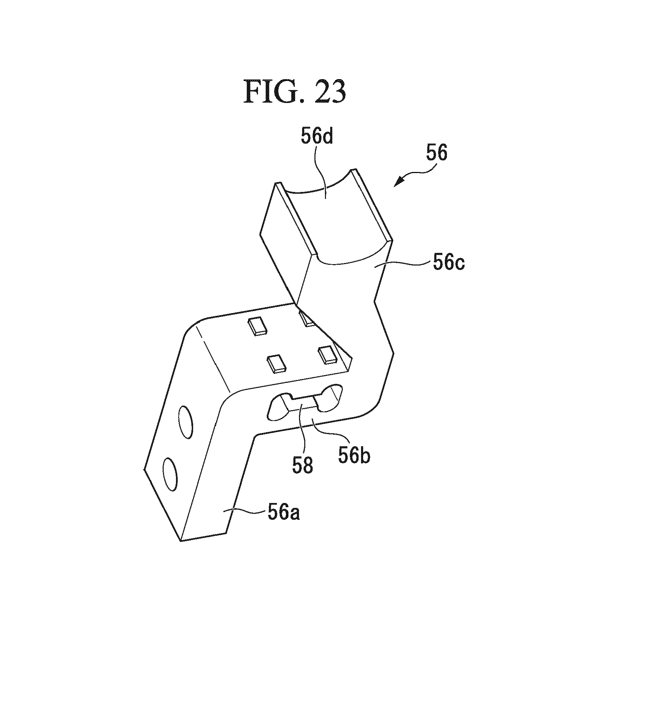

Still another example of the load cell 51 to be used for the load detector for a bed of the invention is shown in FIGS. 21A, 21B, 21C, 22, and 24A, and the substrate 56 to be used for the load cell 51 is shown in FIG. 23.

The configuration of the portion of the substrate 56 in the load cell 51 to be used in this example is substantially the same as that of the substrate 56 of the load cell 51 shown in FIGS. 16A, 16B, and 17. Here, in this example, the orientation of the attaching portion (load-transmitting portion) 56a with respect to the actuating portion (cantilever portion) 56b deviates in a direction that is rotated 90° around the vertical axis from the substrate 56 of the load cell 51 shown in FIGS. 16A, 16B, and 17. However, this is not essential in terms of functions and effects. Therefore, the detailed description of the substrate 56A will be omitted.

In this example, the substrate 56 of the load cell 51 is nearly vertically inserted into an angular tubular casing 200 in which, for example, a horizontal cross-section is a rectangular shape, and the attaching portion (load-transmitting portion) 56a of the substrate 56 is attached to an inner surface of the casing 200 by a screw 202 or the like. In addition, the substrate 56 is attached to a position such that at least the recessed portion 56d of the bearing portion 56c is exposed upward from an upper end of the casing 200.

Also, a cover-like stopper member 204 in which, for example, a vertical cross-section has a downward U shape as a whole is disposed in a state where the stopper member is not mechanically connected with the substrate 56 so as to cover the bearing portion (load-receiving portion) 56c and the spindle (pin) 13 in the substrate 56 of the load cell 51. Here, the cover-like stopper member 204, in short, may cover a space on an opening side in the recessed portion 56d of the bearing portion (load-receiving portion) 56c, and the spindle (pin) 13 that touches the inner surface of the recessed portion 56d. In the illustrated example, the stopper member is made, for example, in a shape surrounding not only a portion above the bearing portion 56c but also the periphery of the bearing portion 56c so as to cover the whole bearing portion (load-receiving portion) 56 from a viewpoint of attachment, and is configured so as to include also the spindle 13 therein.

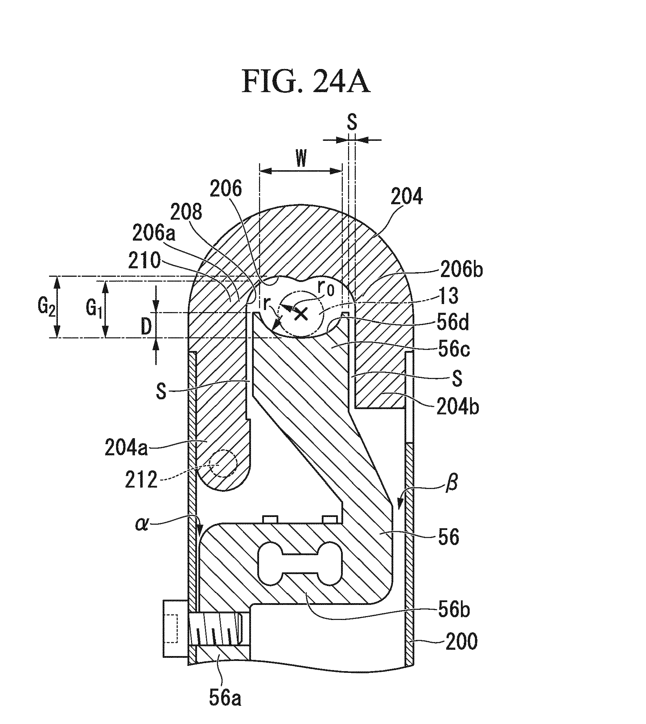

Here, an upper portion of an inside space of the cover-like stopper member 204 is formed as a downward recess 206 that faces the recessed portion 56d of the bearing portion 56c, and both side portions 206a and 206b of the downward recess 206 are located outside both the ends Pa and Pb of the recessed portion 56d of the bearing portion 56c. Here, both the side portions 206a and 206b of the downward recess 206 constitute a wall portion 210 having an inward (direction facing the spindle 13 supported by the recessed portion 56d) wall surface 208 as will be described below later with reference to FIG. 24A. Moreover, a protruding portion 206c protruding downward is formed nearly at a central portion of an inner surface of the downward recess 206 of the stopper member 204. Also, the inner surface shape of the recess 206 is divided into left and right portions by the protruding portion 206c, and the left and right portions are formed in the shape of a concave curve, respectively. In addition, the concavely curved surfaces of the left and right portions are formed in a shape nearly corresponding to the inner surface shape of the recessed portion 56d of the bearing portion 56c, respectively. For example, a concavely curved surface shape equivalent to a track when the spindle 13 is moved in the lateral direction may be taken in the state where the vertical distance from the bottom surface of the recessed portion 56d of the bearing portion 56c to the inner surface of the recess 206 of the stopper member 204 is kept constant at the same distance as a distance G₁ (refer to FIG. 24A) from the bottom surface of the recessed portion 56d of the bearing portion 56c to a tip of the protruding portion 206c in the recess 206 of the stopper member 204.

Both the side portions 206a and 206b of the recess 206 in the cover-like stopper member 204 extend further downward, and the portions (lower ends 204a and 204b) thereof on the tip side are inserted into an upper portion of the casing 200. At least one of the lower ends 204a and 204b is attached to the casing 200 by arbitrary attachment means. In the case of the present embodiment, a projection-like (boss-like) attachment portion 212 is formed on an external surface of one lower end 204a so that attachment is easily enabled, a fitting hole 214 is formed in a side wall of the casing 200, and the lower ends 204a and 204b are inserted into the casing 200 from above to fit the projection-shaped attachment portion 212 into the fitting hole 214 of the casing 200 and thereby the attachment portion is attached to the casing 200. Here, it is also possible to use a screw or the like as the attachment means.

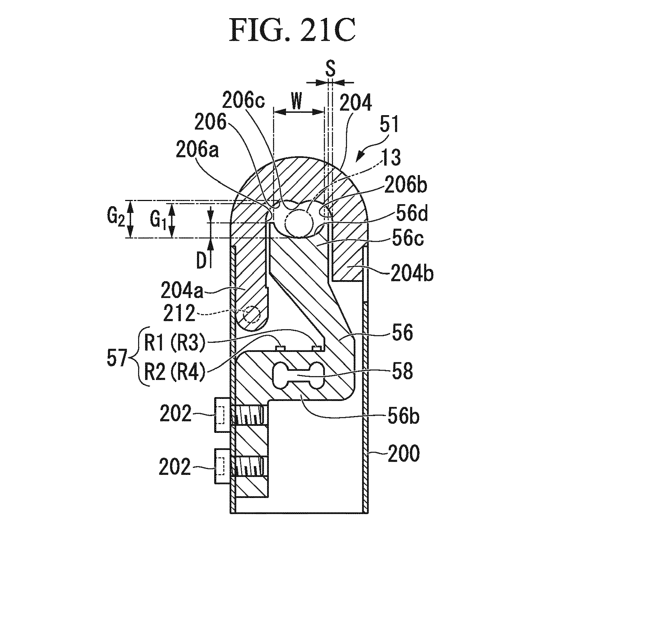

The stopper member 204 is brought into a state where the stopper member is not mechanically coupled with the substrate 56 as mentioned above. Here, the state where the stopper member is not mechanically coupled, in short, may be a state where a force applied to the stopper member 204 is not transmitted to the bearing portion 56c of the substrate 56 and the portion of the cantilever portion (actuating portion) 56b on the bearing portion 56c side so that strain is not caused in the cantilever portion (actuating portion) 56b by the force applied to the stopper member 204 (accordingly, so that the strain caused by the force is not detected by the strain sensor). In practice, as shown in FIG. 21C, it is desirable that the inner surface of the stopper member 204 be spatially separated from the substrate 56, that is, to adopt an aspect in which a spatial gap (clearance) is present between the stopper member 204 and the substrate 56.

In incorporating the load cell 51 as described above into the bed body 1A, the load cell is inserted into the hollow tube portion 103 of an upper portion of, for example, the first coupling arm 9a in the bed body 1 A from above together with the casing 200 and the casing 200 is fixed to the first coupling arm 9a, in a state where the substrate 56 of the load cell 51 is attached to the casing 200 (here, usually, in a state where the stopper member 204 is not yet attached to the casing 200). Then, the spindle (pin) 13 in the bed body 1A is located on the recessed portion 56d of the bearing portion 56c, and the cover-like stopper member 204 is attached in that state. That is, the stopper member 204 is attached to the casing 200 by inserting the lower ends 204a and 204b of the cover-like stopper member 204 into the casing 200 from above to fit the projection-shaped attachment portion 212 into the fitting hole 214 of the casing 200, as mentioned above.

If the load cell 51 is incorporated into the bed body in this way, the strain caused in the actuating portion (cantilever portion) 56b changes similar to the respective examples already described, depending on a change in the load applied from the bed surface-forming section, the change is detected by the strain sensor 57, and consequently, the change of the load is detected.

In addition, in the above description, the load cell 51 of the load detector is a load cell equipped with the casing 200, that is, a load cell in which the substrate 56 is inserted into the casing 200 and the substrate 56 is inserted into the hollow tube portion 103 of the upper portion of the first coupling arm 9a together with the casing 200. However, the casing may not be provided depending on the case. That is, the substrate 56 may be directly inserted into the hollow tube portion 103 of the upper portion of the first coupling arm 9a and fixed the first coupling arm 9a, and the lower portion of the cover-like stopper member 204 may be inserted into the hollow tube portion 103 of the upper portion of the first coupling arm 9a to attach the stopper member 204 to the first coupling arm 9a. The fixing means and the attachment means in that case are not particularly limited.

Here, the functions of the stopper member 204 will next be described with reference to FIG. 24A.

The stopper member 204 has the separation preventing function (first function) of preventing the spindle 13 (pin) 13 from separating from the bearing portion 56c of the load cell substrate 56, for example, in the offset load state as mentioned above, or when a load is abruptly applied onto the bed surface 100, and the function (second function) of preventing a situation in which strain is added to the cantilever portion (actuating portion) 56b by the force (forces other than a vertical load to be originally detected) applied to the spindle 13 in the offset load state, and thus, an abnormality signal is generated or load detection precision deteriorates.

Here, as the shape and dimensions of the recessed portion 56d of the bearing portion 56c of the substrate 56, a surface that is continuously connected to the curvature radius r equal to or more than radius r₀ of the spindle 13 is formed, the width W of the recessed portion 56d is set to a dimension exceeding twice the radius r₀ of the spindle 13, and the inner surface of the recessed portion 56d is made so as not to become a wall surface vertical up to both ends (that is, θ > 0). Therefore, if the stopper member 204 is not provided when an offset load is applied from the bed surface-forming section 100 and the spindle 13 is moved in the lateral direction by the lateral force, there is a concern that the spindle 13 may separate laterally from the recessed portion 56d. Additionally, when the offset load as mentioned above is applied in addition to this, the spindle 13 may be lifted upward in a portion opposite to the portion of the bed body 1A to which the offset load is applied. Additionally, even when the load from the bed surface-forming section 100 changes abruptly, the spindle 13 may be moved upward. In that case, if the stopper member 204 is not provided, there is also a concern that the spindle 13 may separate above the bearing portion 56c.

However, by providing the stopper member 204, the space on the opening side of the recessed portion 56d in the bearing portion 56c is surrounded by the inner surface of the recess 206 of the stopper member 204. Therefore, for example, even if the spindle 13 tends to separate upwardly and laterally from the recessed portion 56d of the bearing portion 56c, for example, as shown by the chain line in FIG. 24A, the movement of the spindle 13 in a separating direction is prevented by the inner surface of the recessed portion 206 of the stopper member 204. That is, the aforementioned first function (separation preventing function) is exhibited. In addition, here, with respect to the lateral movement of the spindle 13 caused by the lateral force in the offset load state, the lateral separation of the spindle is prevented by the inward (direction facing the spindle 13) wall surface 208 formed by the wall portion 210 including both the side portions 206a and 206b of the downward recess 206 of the stopper member 204.

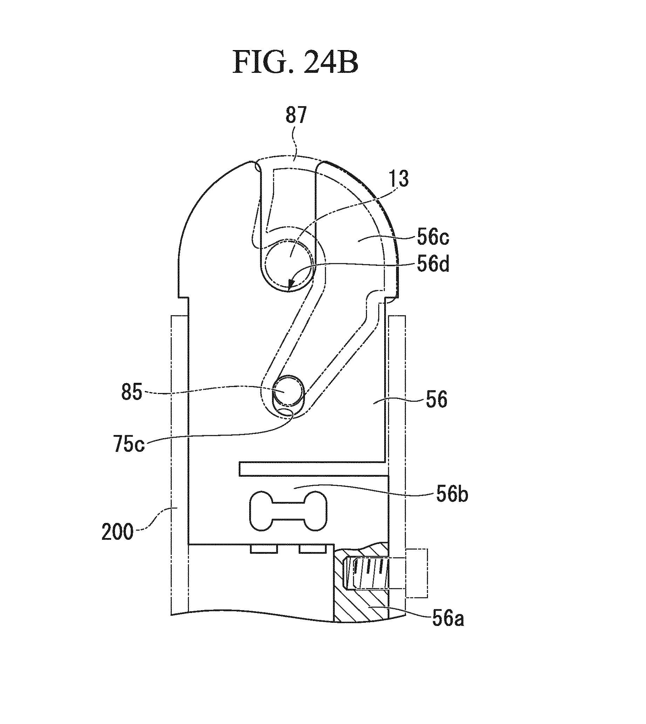

Here, as a measure for preventing the spindle 13 from separating from the bearing portion 56c upwardly and laterally, for example, a structure as shown in 24B is considered in addition to the structure shown in FIG. 24A. That is, a configuration is considered in which not only the portions on both sides of the recessed portion 56d of the bearing portion 56c are made to extend upward greatly, but also the through-hole 75c is formed in the portion. That is, the portion (portion that does not lead to the cantilever portion (actuating portion) 56b) below the bearing portion (load-receiving portion) 56c in the substrate 56, the shaft rod-like coupling pin 85 having a smaller diameter than the through-hole 75c, is inserted through the through-hole with play, the coupling pin 85 is fixed to the tubular portion of the casing 200 or the first coupling arm 9a, the coupling hook member 87 made of an elastic material is engaged with the spindle (pin) 13 and the coupling pin 85, and a resilient force in the direction in which the spindle 13 and the coupling pin 85 are brought close to each other is applied to the spindle and the coupling pin by the coupling hook member 87. If such a configuration is adopted, it is possible to always maintain a state where the spindle 13 abuts against the bottom surface of the recessed portion 56d of the bearing portion 56c of the substrate 56. That is, the separation of the spindle 13 from the recessed portion 56d of the bearing portion 56c can be prevented. Accordingly, application of the structure as shown in FIG. 24B is also allowed.

However, in such a structure as that shown in FIG. 24B, when an offset load is applied as mentioned above, the force to lift the spindle 13 upward is exerted in the portion opposite to the portion of the bed body 1A to which the offset load is applied. That is, in the structure as shown in FIG. 24B, a pulling force is always exerted between the spindle 13 and the coupling pin 85 by the coupling hook member 87. Therefore, with the lifting of the spindle 13, the coupling pin 85 may be lifted upward, and the coupling pin 85 may push up an upper end edge of the through-hole 75c. This lifting force may cause the strain (the strain on the negative side) in a direction opposite to the strain caused by the normal vertical load, in the cantilever portion (actuating portion) 56b of the substrate 56. As a result, the original precision of load detection may deteriorate.

In contrast, in the case of the structure shown in FIG. 24A, when the force to lift the spindle 13 upward as mentioned above is exerted, the spindle 13 tends to move upward slightly, touch a top inner surface of the recess 206 of the stopper member 204, and lift the stopper member 204. However, the stopper member 204 is not mechanically coupled with the substrate 56, and particularly, the bearing portion 56c of the substrate 56 and the portion of the cantilever portion (actuating portion) 56b on the bearing portion 56c side is spatially separated from the stopper member 204. Therefore, the force to lift the stopper member 204 is not applied to the bearing portion 56c of the substrate 56 and the portion of the cantilever portion (actuating portion) 56b on the bearing portion 56c side. Therefore, a situation in which the strain on the negative side is caused in the cantilever portion (actuating portion) 56b of the substrate 56 due to the above lifting force is avoided. As a result, it is possible to improve the load detection precision.

Here, the dimensions of the respective portions in the stopper member 204 and in the bearing portion 56c of the substrate 56 and the mutual relationship between the dimensions, in short, is determined so that the movement of the spindle 13 in the up-and-down direction and the lateral direction is allowed in a space between the recessed portion 56d of the bearing portion 56c and the recess 206 of the stopper member 204, and the force is not immediately transmitted to the substrate 56 when the spindle 13 abuts against the stopper member 204, and the bearing portion 56c and the stopper member 204 are brought into a state where the bearing portion and the stopper member are not mechanically coupled with each other. The preferable dimensions of the respective portions and the preferable mutual relationship between the dimensions are as follows (refer to FIG. 24A).

First, similar to that already described, it is preferable that the width W of the recessed portion 56d of the bearing portion 56c exceed 2.2 times the radius r₀ of the spindle 13 and be equal to or less than 20 times the radius r₀.

The depth D of the recessed portion 56d of the bearing portion 56c, similar to that already described, is also less than 2 times the radius r₀ of a spindle 13, preferably, less than 1 times the radius ro of the spindle 13. Moreover, the depth of the recessed portion is more preferably within a range of 0.2 times to 0.8 times the radius r₀.

Meanwhile, the distance G₁ from the bottom surface of the recessed portion 56d of the bearing portion 56c to the tip of the protruding portion 206c in the recess 206 of the stopper member 204 exceeds twice the radius r₀ of the spindle 13 and is equal to or less than 10 times the radius, and is more preferably within a range of 2.2 to 4 times the radius r₀ of the spindle 13.

Moreover, the distance G₂ from the bottom surface of the recessed portion 56d of the bearing portion 56c to topmost portions of the concavely curved surfaces on both the left and right sides of the protruding portion 206c in the recess 206 of the stopper member 204 may be determined depending on a track when the spindle 13 is moved in the lateral direction with the distance G₁ being made constant within a range where the relationship of the distance G₁ with respect to the spindle 13 is satisfied.

Additionally, it is preferable that the gap S between an outside surface of the bearing portion 56c and an inside surface of the stopper member 204 be equal to or more than 0.5 mm and less than 2 times the radius r₀ of the spindle 13.

In addition to this, it is preferable that the gap α between an outside surface of the portion of the actuating portion (cantilever portion) 56b, which leads to the load-transmitting portion (attaching portion) 56a, and an inside surface of the casing 200 out of the gaps α and β between an outside surface of the substrate 56 and the inside surface of the casing 200 be within a range of 0.1 to 5 mm, and it is preferable that the gap β between the outside surface of the portion of the actuating portion (cantilever portion) 56b, which leads to the bearing portion 56c, and the inside surfaces of the casing 200 be within a range of 2 to 10 mm.

Here, as for the gap α, the actuating portion (cantilever portion) 56b can be strained smoothly due to the presence of the gap when a load is applied from the bed body 1A. Additionally, as for the gap β, not only can the actuating portion (cantilever portion) 56b be strained smoothly similar to the above due to the presence of the gap, but also, a cable or the like for signal extraction from the strain sensor 57 can be passed through the gap.

In addition, the materials of the stopper member 204 are not particularly limited, and similar to the materials of the substrate 56 of the load cell 51, metals such as an aluminum alloy, iron, steel, and stainless steel, and other resins such as engineering plastic can be used. Here, it is desirable to select a material that can easily absorb a force applied to the stopper member 204 or to select a material capable of being slightly deformed during attachment and detachment so that attachment and detachment are easily enabled, and it is desirable to use a resin material from these viewpoints. Moreover, the materials of the stopper member 204 do not need to be the same as the materials of the substrate 56 of the load cell 51, and it is desirable to select a material different from the materials of the substrate 56 of the load cell 51 from the above viewpoints.

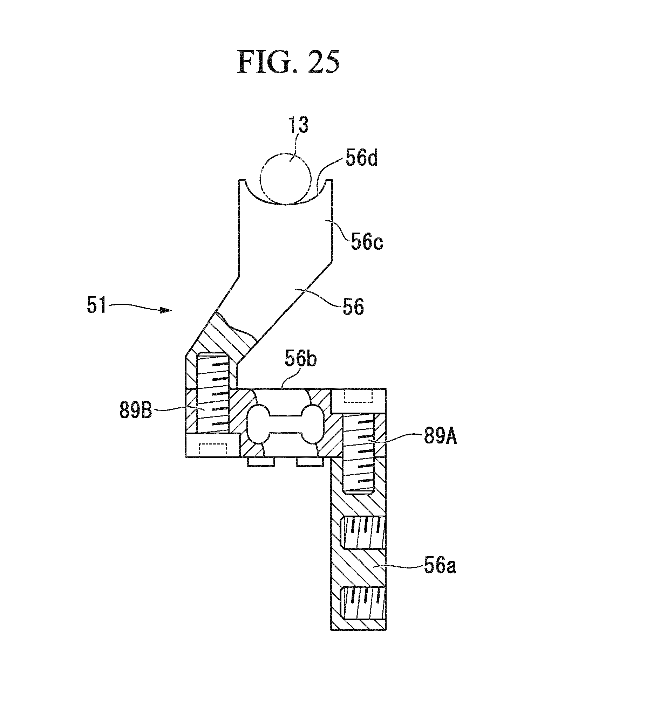

The substrate 56 to be used for the load cell 51 shown in FIGS. 21A, 21B, 21C, 22, and 24A, that is, the substrate 56 shown in FIG. 23 may not have an integrally continuous configuration in its entirety. That is, depending on the case, the substrate 56 may be configured to be split into two pieces or three pieces, and the respective split pieces may be configured to be connected together by proper coupling and anchoring means, such as screw-stopping, welding, or brazing. Examples thereof are shown in FIGS. 25 to 27.

The substrate 56 of the load cell 51 shown in FIG. 25 has a configuration in which the load cell is split into three pieces by splitting the load-transmitting portion (attaching portion) 56a, the actuating portion (cantilever portion) 56b, and the load-receiving portion (bearing portion) 56c therebetween, respectively.

In FIG. 25, the load-transmitting portion (attaching portion) 56a, the actuating portion (cantilever portion) 56b, and the load-receiving portion (bearing portion) 56c are separately made, respectively, the load-transmitting portion (attaching portion) 56a and the actuating portion (cantilever portion) 56b are coupled together by the screw 89A, and the actuating portion (cantilever portion) 56b and the load-receiving portion (bearing portion) 56c are coupled together by the screw 89B.

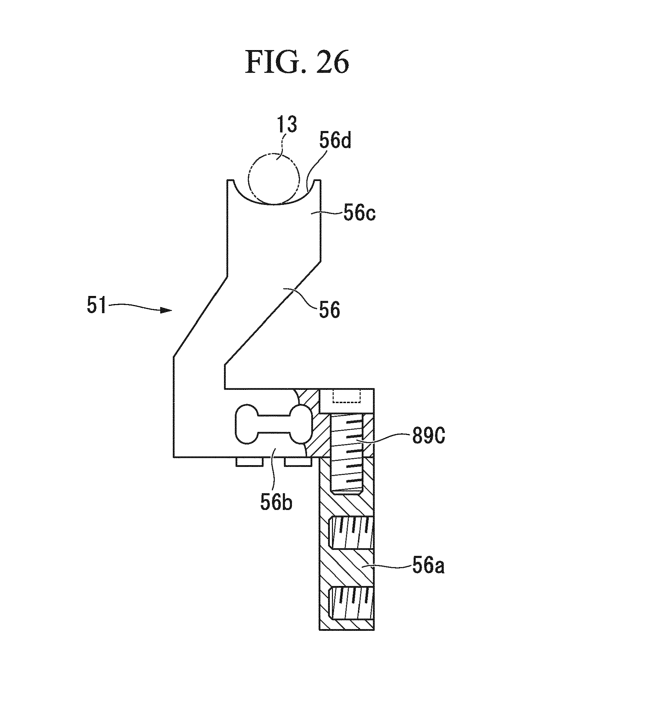

Additionally, the substrate 56 of the load cell 51 shown in FIG. 26 has a configuration in which the load-receiving portion (bearing portion) 56c and the actuating portion (cantilever portion) 56b are integrally made, the load-transmitting portion (attaching portion) 56a is separately made from the load-receiving portion (bearing portion) 56c and the actuating portion (cantilever portion) 56b, and the actuating portion (cantilever portion) 56b and the load-transmitting portion (attaching portion) 56a are coupled together by the screw 89C, that is, has a two-piece split configuration.

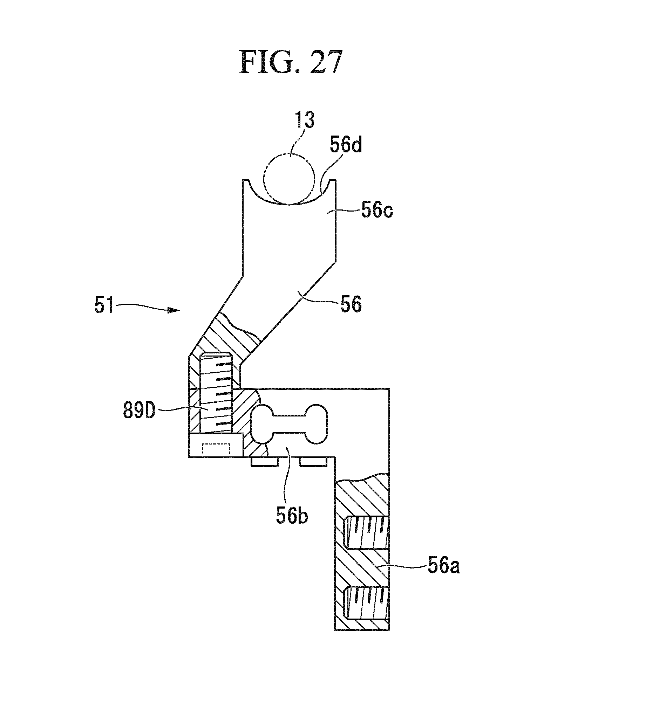

Moreover, the substrate 56 of the load cell 51 shown in FIG. 27 has a configuration in which the load-transmitting portion (attaching portion) 56a and the actuating portion (cantilever portion) 56b are integrally made, the load-receiving portion (bearing portion) 56c is separately made from the load-transmitting portion (attaching portion) 56a and the actuating portion (cantilever portion) 56b, and the actuating portion (cantilever portion) 56b and the load-receiving portion (bearing portion) 56c are coupled together by the screw 89D, that is, has a two-piece split configuration.

As shown in FIG. 25, 26, or 27, when the substrate 56 of the load cell 51 is split, as already described, it is possible to select an optimal material as a structural material of each split portion according to the required characteristics of each split portion in the substrate, for example, the desired workability, yield strength, elongation, or the like of each portion.

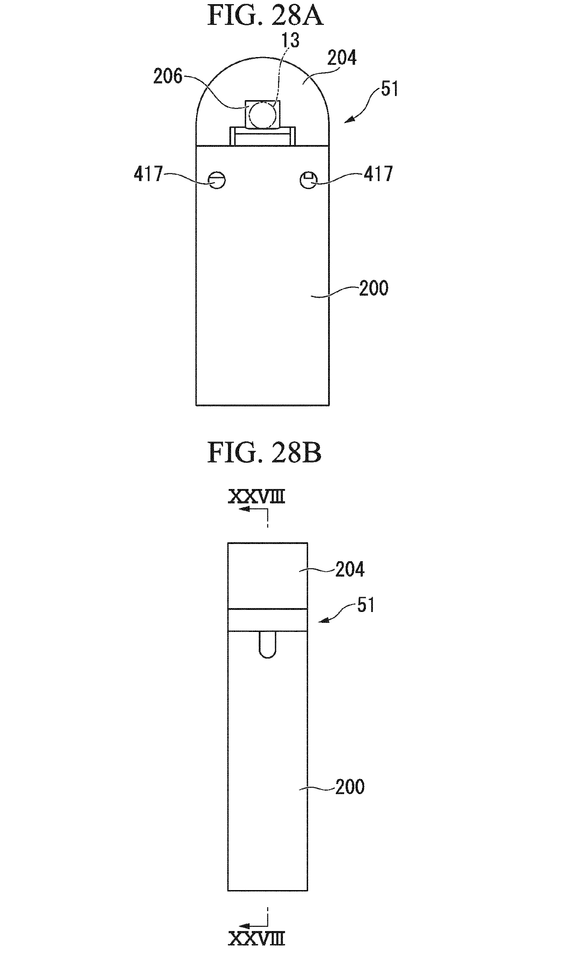

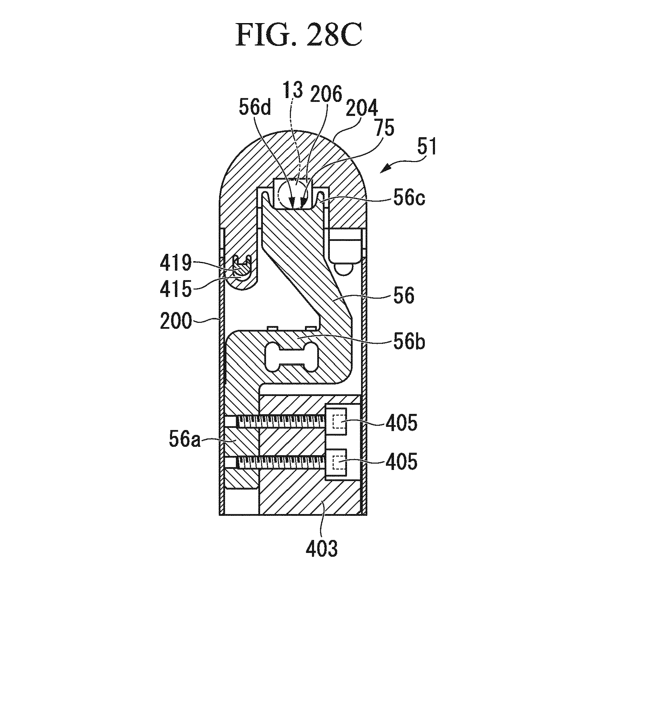

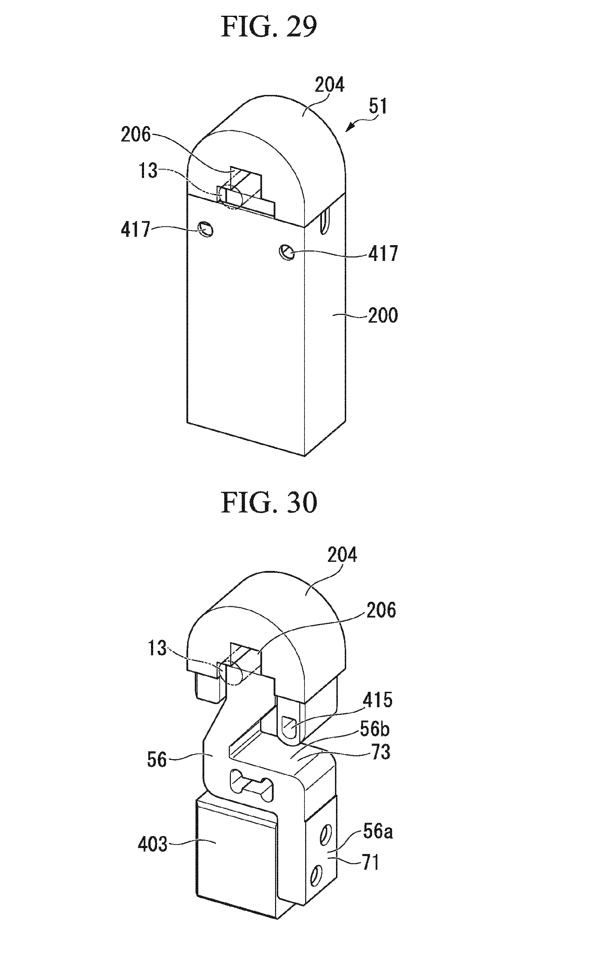

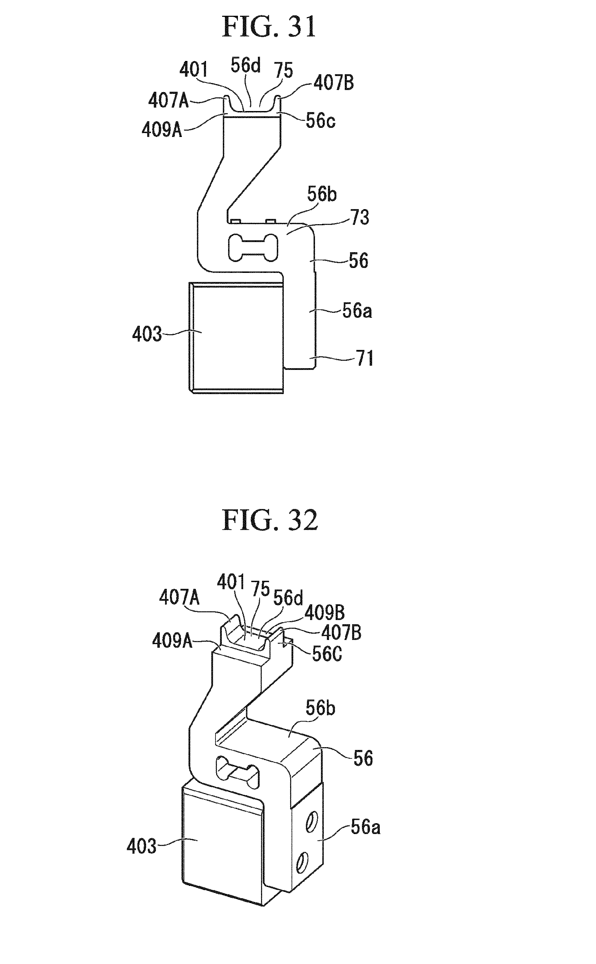

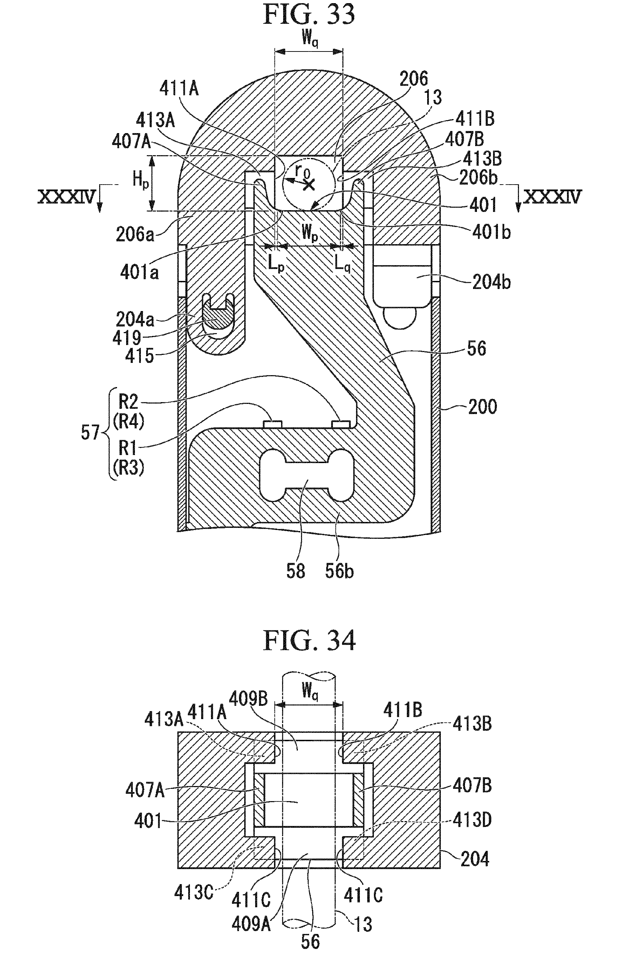

A still further example of the load cell 51 to be used for the load detector for a bed of the invention is shown in FIGS. 28A, 28B, 28C, and 29. Additionally, the load cell 51 in a state where the casing 200 is omitted in the example is shown in FIG. 30, and the substrate 56 to be used for the load cell 51 is shown in FIGS. 31 and 32. An enlarged cross-section of main portions of the load cell 51 of the example is shown in FIGS. 33 and 34.

In the example shown in FIGS. 28A to 34, similar to the example shown in FIGS. 21A to 24A, the cover-like stopper member 204 in which, for example, the vertical cross-section has the downward U shape as a whole is disposed in a state where the stopper member is not mechanically coupled with the substrate 56 so as to cover the bearing portion (load-receiving portion) 56c and the spindle (pin) 13 in the substrate 56 of the load cell 51. Here, the shape, particularly, inside shape of the stopper member 204 is different from that of the example shown in FIGS. 21A to 24A. Additionally, the shape of the substrate 56 to be used for the load cell 51 is also slightly different from the shape of the substrate 51 in the example shown in FIGS. 21A to 24A. Thus, in the following, the example shown in FIGS. 28A to 34 will be described focusing on points different from the example shown in FIGS. 21A to 24A.

The substrate 56 of the load cell 51, similar to the respective examples already described, has on one end side (upper side of the drawing) the bearing portion 56c formed as the load-receiving portion 75 that receives the load from the bed surface-forming section 100 and has on the other end side (lower side of the drawing) the attaching portion 56a as the load-transmitting portion 71 that transmits the load to a structural member on the installation surface side in the bed body. The cantilever portion 56b is formed between the load-receiving portion 75 (bearing portion 56c) and the load-transmitting portion 71 (attaching portion 56a) as the actuating portion 73 that is deflected by the load, and the strain sensor 57 is attached to the actuating portion 73 (cantilever portion 56b).

An attachment block 403 forming, for example, a rectangular parallelepiped shape is fixed to the attaching portion 56a of the substrate 56 by, for example, a screw 405. Thus, the substrate 56 to which such an attachment block 403 is fixed is nearly vertically inserted into the angular tubular, that is, bottomed or non-bottomed casing 200 in which, for example, a horizontal cross-section has a rectangular shape. In addition, at least the recessed portion 56d of the bearing portion 56c of the substrate 56 is exposed upward from the upper end of the casing 200.

It is preferable that the casing 200 serve as the first coupling arm 9a (refer to FIG. 15) in the bed body 1 A. In other words, it is desirable that the casing 200 has a lower end directly fixed to the bottom frame 5 in the bed body 1 A by welding or the like. In this case, although the substrate 56 to which the attachment block 403 is fixed may be fixed to the casing 200, in the case of the present example, the substrate is inserted only. Here, it is desirable that the gap between the substrate 56 to which the attachment block 403 is fixed, and the casing 200 be suppressed to the minimum to such a degree that severe mechanical rattling does not occur between the substrate 56 to which the attachment block 403 is fixed, and the casing 200.

The recessed portion 56d formed in the bearing portion 56c of the substrate 56 has the supporting surface 401 that receives the spindle 13 in contact with the spindle 13. The supporting surface 401 is formed as a substantially horizontal plane unlike the example shown in FIGS. 21A to 21C. Also, both side portions (portions on both sides in a horizontal direction orthogonal to a length direction of the spindle 13) 407A and 407B of the supporting surface 401 forming the plane have risen in the shape of a curve. In other words, the portion between rising portions 407A and 407B serves as the supporting surface 401 forming the plane. The width (width of a region that maintains the plane without rising) of the supporting surface that is the plane is defined as Wp. In addition, the portions on both sides of the bearing portion 56c along the length of the spindle 13 in the horizontal direction are cut out in the shape of steps (cutout portions 409A and 409B).

The outside shape of the cover-like stopper member 204, similar to the example shown in FIGS. 21A to 24A, is made so that, for example, the vertical cross-section has the downward U shape, and has the downward recess 206. The inside shape (inner surface shape of recess 206) of the stopper member 204 is different from the example shown in FIGS. 21A to 24A. That is, step portions 413A to 413D having wall surfaces 411A to 411D that face the outer peripheral surface of the spindle 13 are formed in four symmetrical places on the inner surface side of the stopper member 204. Here, the wall surfaces 411 A to 411 D are formed as vertical wall surfaces that face the outer peripheral surface of the spindle 13 from both sides in the horizontal direction along the length of the spindle 13. The spacing (the spacing between the wall surface 411C and the wall surface 411 D is also the same) between the wall surface 411 A and the wall surface 411B is defined as Wq for convenience.

Both of the end portions 206a and 206b of the recess 206 in the cover-like stopper member 204 extend further downward, and the portions (lower ends 204a and 204b) thereof on the tip side are inserted into the upper portion of the casing 200. At least one of the lower ends 204a and 204b is attached to the casing 200 by arbitrary attachment means.

In case of the present example, one lower end 204a of the stopper member 204 is formed with a pin hole 415 passing therethrough, and insertion holes 417 are formed in both side walls of the casing 200 so that attachment is easily enabled. Also, the stopper member 204 is configured to be attached to the casing 200 by inserting the lower ends 204a and 204b into the casing 200 from above and inserting a pin 419 made of metal or synthetic resin into the pin hole 415 from the insertion holes 417. Here, the attachment means of the stopper member 204 is not limited to the above, and it is possible to use a screw or the like.

Moreover, in the example shown in FIGS. 28A to 34, the preferable conditions of the dimensional relationship between the respective portions will be described.

The spacing between the wall surface 411 A and the wall surface 411B and the spacing Wq between the wall surface 411C and the wall surface 411D are determined as follows.

That is, as a first condition, the spacing Wq is determined to such a degree such that the spacing is slightly greater than the diameter (2 x r₀) of the spindle 13, the spindle 13 is settled between the wall surfaces 411 A and 411C and the wall surfaces 411 B and 411D, and a slight gap is present (for example, a gap of about 2 mm of one side when the diameter of the spindle 13 is 12 mm) between the wall surfaces 411A and 411C and the outer peripheral surface of the spindle 13 and between the wall surfaces 411B and 411 D and the outer peripheral surface of the spindle 13. Accordingly, the spindle 13 is allowed to slightly move left and right while touching the planar supporting surface 401.

As a second condition, it is desirable to determine that a horizontal distance Lp from the positions of the wall surfaces 411 A and 411C to an end position 401 a of the aforementioned planar supporting surface 401, and a horizontal distance Lq from the positions of the wall surfaces 411 B and 411 D to an end position 401b of the planar supporting surface 401 become smaller than the radius r₀ of the spindle 13, respectively. In the case of the illustrated example, the second preferable condition is satisfied by determining the spacing Wq between the wall surface 411 A and the wall surface 411B and the spacing Wq between the wall surface 411C and the wall surface 411 D so as to become nearly equal to the width Wp of the planar supporting surface 401.

The second condition prevents a situation in which the spindle 13 abuts against the wall surfaces 411 A and 411C or the wall surfaces 411 B and 411 D before riding on (or colliding against) the rising portions 407A and 407B on both sides of the planar supporting surface 401, and the spindle 13 moves further, when the spindle 13 slightly moves (rolls) left and right while touching the planar supporting surface 401.

The height Hp from the planar supporting surface 401 to an internal bottom surface of the recess 206 in the stopper member 204 is made slightly greater than the diameter (twice the radius r₀) of the spindle 13. For example, if the diameter of the spindle 13 is 12 mm, the height Hp is about 13 mm to 15 mm.

In the example shown in FIGS. 28A to 34 described above, similar to the example shown in FIGS. 21A to 24A, the cover-like stopper member 204 can be provided to prevent the spindle 13 from separating upwardly and laterally from the bearing portion 56c. Moreover, in the example shown in FIGS. 28A to 34, the function (second function) of preventing that strain is applied to the cantilever portion (actuating portion) 56b by the force (force other than the vertical load to be originally detected) applied to the spindle 13 in the offset load state and thus an abnormality signal occurs or the load detection precision deteriorates, is more excellent than the example shown in FIGS. 21A to 24A.

That is, even if there is a lateral component as a component of the load applied to the spindle 13 in the offset load state, the lateral component is not applied to the supporting surface 401 that is the horizontal plane in the bearing portion 56c of the substrate 56. Also, when the spindle 13 is moved in the lateral direction by the lateral load, the spindle 13 abuts against or collides against the wall surfaces 411 A and 411C or the wall surfaces 411B and 411 D of the stopper member 204. However, since the stopper member 204 is mechanically (physically) separated from the substrate 56, the lateral load is not applied to the bearing portion 56c of the substrate 56 at all. Accordingly, only the vertical load to be detected can be reliably detected, and the load detection precision can be further enhanced.

In addition, the portions on both sides of the bearing portion 56c, which are cut out in the shape of steps (cutout portions 409A and 409B) along the length of the spindle 13 in the horizontal direction, are formed in order to achieve the miniaturization of the load cell so that the stopper member 204 does not protrude to the outside from an extending surface of the external surface of the casing 200. That is, when the bearing portion 56c is not formed with the cutouts as described above, the stopper member 204 needs to be made to protrude to the outside of the bearing portion 56c in order to form the wall surfaces 411 A to 411 D in the stopper member 204. Also, in that case, in order to prevent the stopper member 204 from protruding to the outside from the extending surface of the external surface of the casing 200, it is necessary to make the thickness of the whole substrate 56 having the bearing portion 56c smaller, and there is a probability that a strength problem may occur in the substrate 56. In contrast, if the bearing portion 56c is formed with the cutout portions 409A and 409B, it is unnecessary to make the whole thickness of the substrate 56 smaller.

In addition, the substrate 56 in the example shown in FIGS. 28A to 34 may be changed to a three-piece split structure according to FIG. 25, or may be a two-piece split structure according to FIG. 26 or 27.

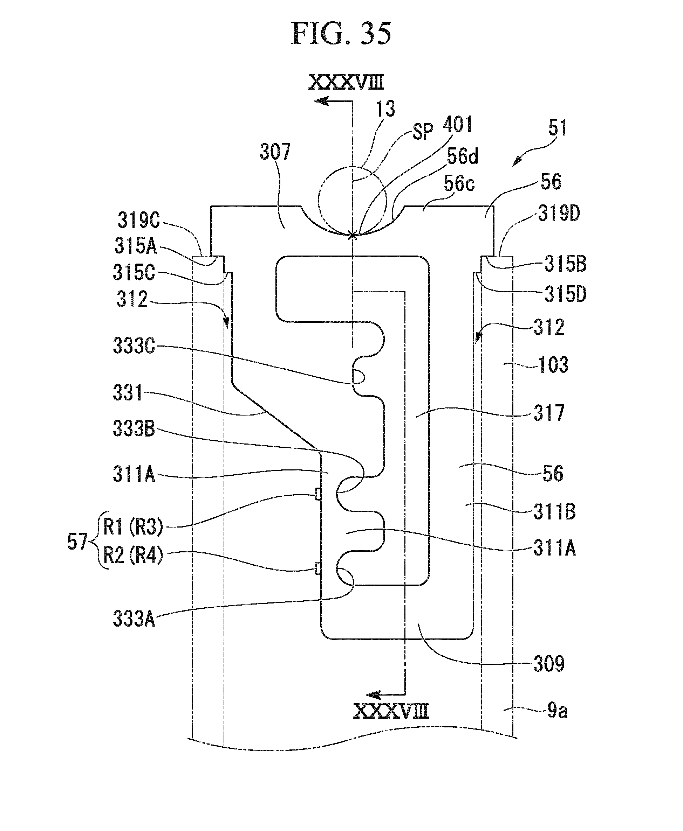

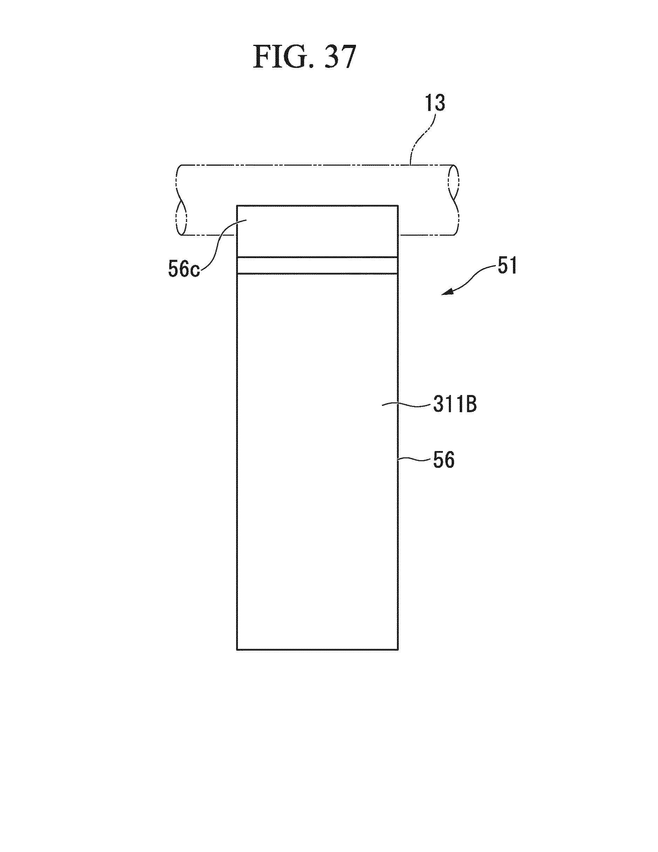

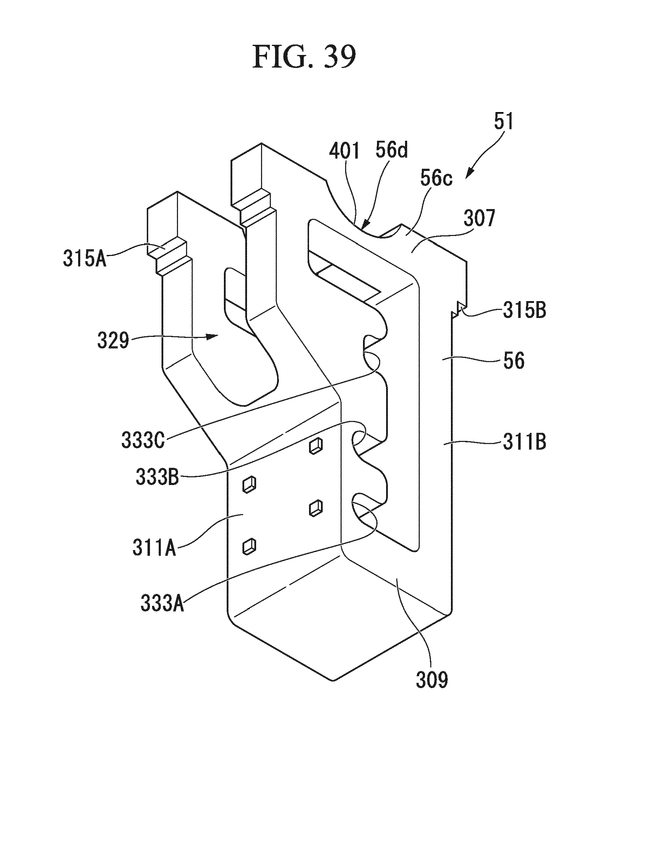

Moreover, a still further example of the load cell 51 in the load detector of the invention is shown in FIGS. 35 to 39.

The load cell 51 of the embodiment shown in FIGS. 35 to 39 is basically configured by the substrate 56 and the strain sensor 57 (the strain gauge R1, R2, R3, R4) to which loads are added, similar to the respective examples already described. Also, the substrate 56 includes a load-receiving beam portion 307 having the bearing portion 56c as the load-receiving portion and a pair of fulcrum-receiving regions 315A and 315B as the load-transmitting portion, a pair of actuating arm portions 311A and 311 B that protrude from the left and right positions in the load-receiving beam portion 307 as the actuating portion, and a coupling portion 309 that connects the portions of the actuating arm portions 311A and 311B on the tip side together, and these respective portions are configured so as to continuously surround the hollow portion 317 as a whole. Also, in the case of the present embodiment, the right and left actuating arm portions 311B and 311A are configured so that the rigidity thereof becomes unbalanced. For example, in the illustrated example, the right and left actuating arm portions are configured so that the rigidity of the right actuating arm portion 311 B is greater than that of the left actuating arm portion 311A.

In addition, the spindle 13 to which the load from the bed surface-forming section of the bed body 1A is added is formed to have a substantially horizontal axis similar to the above. Also, the substrate 56 is nearly vertically inserted into, for example, the hollow tube portion 103 of the first coupling arm 9a (refer to FIG. 15) from above, and the end edge portions of the hollow tube portion 103 at symmetrical positions with the axis as a center, are formed as supporting portions 319C and 319D that support the substrate 56. In addition, the portions inserted into the hollow tube portion 103 are portions of the actuating arm portions 311A and 311B of the substrate 56.

In the load detector of the example shown in FIGS. 35 to 39, the bearing portion 56c as the load-receiving portion in the load-receiving beam portion 307 of the substrate 56 is formed with the recessed portion 56d, and the bottom surface (supporting surface 401) of the recessed portion 56d is configured to receive the spindle 13.

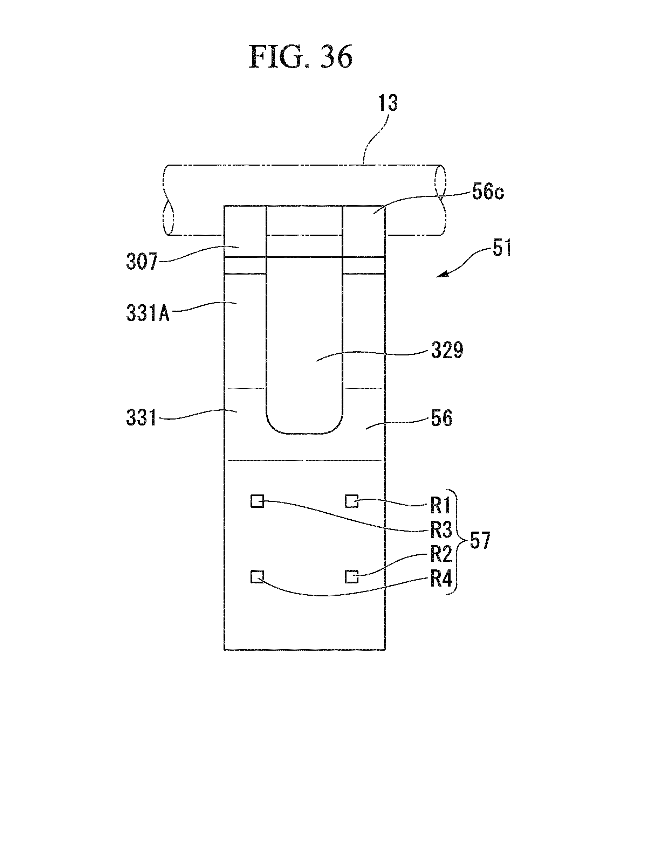

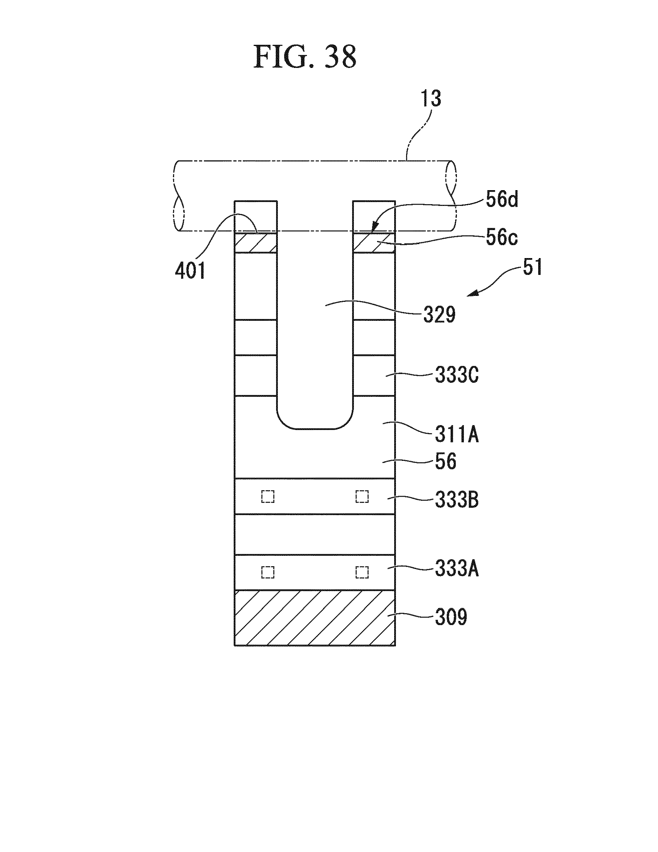

Moreover, if a plane orthogonal to the length direction of the load-receiving beam portion 307 through, for example, the central position (center of the bottom surface of the recessed portion 56d) of the load-receiving beam portion 307 is defined as a reference plane SP, an upper portion in a left portion of the substrate 56 (portion on a side where the strain sensor 57 is located), is cut in from a side along the vertical plane orthogonal to the reference plane SP to form a cut-in portion 329 (refer to FIGS. 36 and 38), and is formed in a shape that is bifurcated by the cut-in portion 329. Specifically, the cut-in portion 329 is formed from the left portion including the bearing portion 56c (load-receiving portion) in the load-receiving beam portion 307 to the middle of the left actuating arm portion 311 A in the length direction out of the right and left actuating arm portions 311B and 311A, and the portion from the bearing portion 56c (load-receiving portion) in the load-receiving beam portion 307 to the portion on one fulcrum-receiving region 315A side and the intermediate position of the one actuating arm portion 311 A continuously connected to the portion is bifurcated. In addition, as described above, the reference plane SP passes through the central position of the load-receiving beam portion 307 in the length direction. However, this is given in order to make explanation easily understood, and the position of the reference plane is not limited to the central position.

Additionally, as shown in FIGS. 35 to 37, an inclined surface 331 formed on the outside surface the actuating arm portion 311 A ranging from the upper portion thereof to the intermediate portion, is made so that the width (it is the width in a direction orthogonal to the thickness direction) thereof becomes smaller in the portion from the upper portion to the intermediate portion. Moreover, a plurality of recessed portions 333A, 333B, and 333C recessed toward an external surface side of the actuating arm portion 311A are formed at intervals in the length direction (up-and-down direction) of the actuating arm portion 311A on an inner surface side of the actuating arm portion 311 A. That is, the first recessed portion 333A is formed near a lower end (portion leading to the coupling portion 309) in the actuating arm portion 311A, the second recessed portion 333B is formed at a position slightly above the first recessed portion 333A, and the third recessed portion 333C is formed at a position (position above the center of the actuating arm portion 311 A in the length direction) above the second recessed portion 333B.

Also, the strain gauges R1 and R3 among the four strain gauges R1, R2, R3, and R4 constituting the strain sensor 57 are adhered to, for example, an external surface at a position corresponding to the second recessed portion 333B, and the strain gauges R2 and R4 are adhered to, for example, an external surface at a position corresponding to the first recessed portion 333A.

Also, the strain gauges R1, R2, R3, and R4 are assembled into the Wheatstone bridge circuit shown in FIG. 5, similar to the example already described.

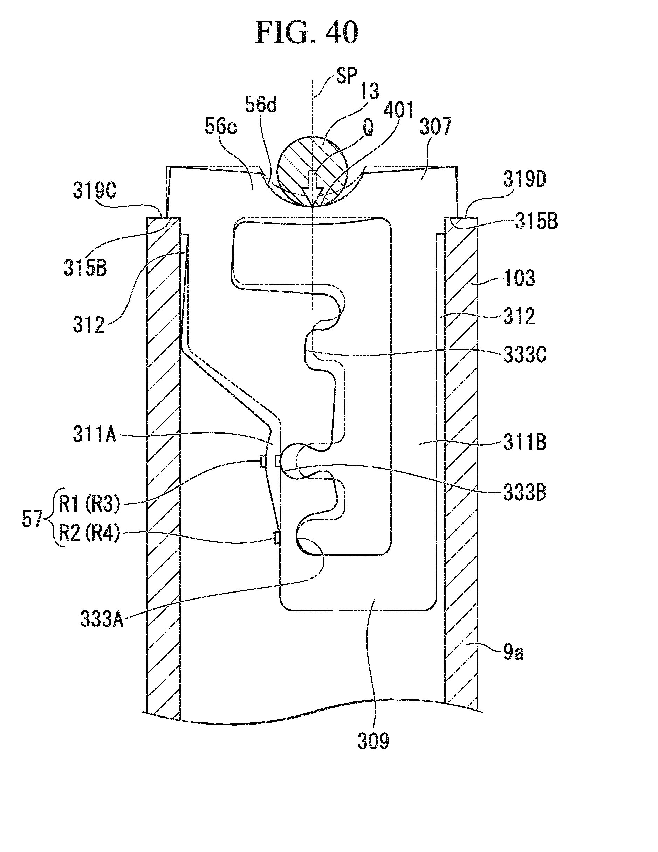

A situation when a load is applied to the load cell 51 shown in FIGS. 35 to 39 is shown in FIG. 40.

In FIG. 40, the actuating arm portions 311 A and 311B are inserted into, for example, the hollow tube portion 103 of the first coupling arm 9a in the bed body 1A from above, and the fulcrum-receiving regions 315A and 315B of the both end bottom surfaces (step surfaces) of the load-receiving beam portion 307 are supported by the end edges (supporting portions 319C and 319D) of the hollow tube portion 103 of the first coupling arm 9a. If a load is added from the spindle 13 located in the recessed portion 56d of the bearing portion 56c as the load-receiving portion to the supporting surface 401 of the bearing portion 56c in this state, the load-receiving beam portion 307 is deflected and deformed such that a central portion thereof falls downward. Here, since the right actuating arm portion 311 B out of the pair of actuating arm portions 311 A and 311 B is not formed with the cut-in portion 329 (refer to FIG. 35) and is also not formed with the recessed portions 333A, 333B, and 333C, the rigidity thereof is markedly large as compared to the left actuating arm portion 311A. Therefore, the right actuating arm portion 311B is not substantially deformed, and the left actuating arm portion 311A is solely deflected and deformed. That is, only the left actuating arm portion 311A is deformed so as to overhang to the left (outward). The deflection and deformation of the actuating arm portion 311A are locally promoted by the recessed portions 333A and 333B formed inside the actuating arm portion 311A, and the promoted strain is effectively detected by the strain sensor 57.

That is, as shown in FIG. 35, the thickness of the actuating arm portion 311A at the first recessed portion 333A becomes locally small and the rigidity thereof becomes smaller, due to the first recessed portion 333A of the end of the actuating arm portion 311A on the coupling portion 309 side, and the lower portion of the first recessed portion 333A is constricted by the coupling portion 309. Therefore, the actuating arm portion 311A is deflected and deformed in a curved shape from a position very near the coupling portion 309 to the outside. Therefore, compression strain occurs in a concentrated manner on the external surface near a portion corresponding to the first recessed portion 333A, and a large resistance change occurs in the strain gauge R2 (R4) of the strain sensor 57 due to the compression strain.

Moreover, also in the vicinity of the second recessed portion 333B formed close to the first recessed portion 333A and above the first recessed portion 333A, the thickness of the actuating arm portion 311A at the second recessed portion 333B becomes locally small and the rigidity thereof becomes small. Therefore, in the vicinity of the second recessed portion 333B, the actuating arm portion is elastically deformed in a direction opposite to the direction of elastic deformation in the vicinity of the first recessed portion 333A, and tension strain occurs in a concentrated manner on the external surface in the vicinity of the portion. Also, a large resistance change occurs in the strain gauge R1 (R3) of the strain sensor 57 due to this tension strain.

Here, the load cell shown in FIG. 35 includes the Roberval mechanism of a type having the shape of the half of a spectacle type Roberval mechanism. That is, the shape of the vicinity of the portion where the first recessed portion 333A and the second recessed portion 333B are formed adjacent to each other is the same as the shape of one side in the spectacle type Roberval mechanism. Also, when the actuating arm portion 311A is deformed, the same strain concentration effect as in a case where the spectacle type Roberval mechanism is formed is obtained in the vicinity of the portion where the first recessed portion 333A is formed and in the vicinity of the portion where the second recessed portion 333B is formed. Moreover, strain (deformation) in an opposite direction occurs in the vicinity of the first recessed portion 333A and in the vicinity of the second recessed portion 333B.

By forming the recessed portions 333A and 333B in this way, the compression strain of the portion of the strain gauge R2 (R4) and the tension strain of the portion of the strain gauge R1 (R3) become markedly large as compared to a case where the recessed portions 333A and 333B are not formed. As a result, a large output can be obtained from the aforementioned Wheatstone bridge circuit. Accordingly, it is possible to detect a load with high precision, and it is possible to reliably recognize a load fluctuation even if the load fluctuation is slight.

Moreover, in such a configuration, the strain gauge R2 (R4) that detects the compression strain, and the strain gauge R1 (R3) that detects the tension strain are stuck close to each other. Therefore, handling of wiring lines between these strain gauges or lead wires for input/output between the strain gauges and the outside also becomes easy.

In addition, in the present example, the third recessed portion 333C is formed for the sake of design convenience and is not directly related with the amplification of strain. Therefore, the third recessed portion may not be necessarily formed in the actuating arm portion 311 A.

Additionally, in the present example, as shown in FIGS. 35 and FIG. 40, when the actuating arm portions 311A and 311 B are inserted into the hollow tube portion 103 of the first coupling arm 9a in the bed body 1A, second step surfaces 315C and 315D are formed on the lower side of the fulcrum-receiving regions (step surfaces) 315A and 315B so that gaps 312 are formed between an inner surface 103E of the hollow tube portion 103 and the external surfaces of the actuating arm members 311A and 311B. That is, the formation of the gap 312 is adjusted by setting the distance between the external surfaces (the maximum distance between a left side surface of the actuating arm portion 311A and a right side surface of the actuating arm portion 311B in FIGS. 35 and 40) of the portions of the actuating arm portions 311 A and 311B inserted into the hollow tube portion 103 of the first coupling arm 9a so as to be smaller than the internal diameter of the hollow tube portion 103.

By adjusting shape and dimensions in this way so that the gap 312 is formed, thereby allowing, within the gap 312, the deformation of the actuating arm portion 311A when a load is applied, it is possible to reliably detect strain.

Additionally, also in the present example, if a load Q is applied to the load-receiving beam portion 307 in the vertical direction, not only is the actuating arm portion 311A on a side (left side) with small rigidity out of the pair of actuating arm portions 311 A and 311B shown in FIG. 40 deflected and deformed, but also the actuating arm portion 311B on a side (right side) with large rigidity is inclined counterclockwise slightly from the vertical direction on the basis of the fulcrum-receiving region 215B (is inclined so that a lower end side thereof moves to the right), and simultaneously, the position of the coupling portion 309 slightly moves to the right. However, the inclination angle and movement distance of the actuating arm portion and the coupling portion are only very slight, and thus, the inclination and movement thereof are neglected in FIG. 40, and are not particularly shown on the drawing. Here, the above inclination and movement can be allowed by adjusting shape and dimensions so that the gap 312 is formed also on the right actuating arm portion 211B side as mentioned above.

In addition, the functions according to the shape and dimensions of the recessed portion 56d of the bearing portion 56c may be the same as those of the respective examples already described, and the description thereof will be omitted.

In addition, in the present example, the substrate 56 of the load cell 51 is directly inserted into the hollow tube portion 103 of the first coupling arm 9a that is a structural member of the load transmission path of the bed body 1A. However, the substrate 56 may be inserted into a casing that is separate from the structural member of the load transmission path of the bed body 1A and may be inserted into the structural member of the load transmission path of the bed body 1 A together with the casing, for example, the hollow tube portion 103 of the first coupling arm 9a.

Additionally, also in the present example, it is not necessary to adopt strain gauges (strain-sensitive resistors) for all of the four resistors R1, R2, R3, and R4 constituting the Wheatstone bridge circuit, and it is needless to say that a strain gauge as at least one resistor may be adhered to a position corresponding to at least one of the first recessed portion 333A and the second recessed portion 333B in the actuating arm portion 311A, and dummy resistors may be used for the rest.

Moreover, in the example shown in FIGS. 36 to 39, the strain gauges R1 to R4 are adhered to the external surface of the actuating arm portion 311 A. However, strain is generated also on an inner surface side of the actuating arm portion 311A and large strain occurs particularly on the surfaces (bottom surface) of the first recessed portion 333A and the second recessed portion 333B on the deep side. Therefore, depending on the case, a load can be detected even if some or all of the strain gauges R1 to R4 are adhered to the inner surface side of the actuating arm portion 311 A, particularly, the bottom surface of one or both of the recessed portions 333A and 333B. For example, the strain gauges R1 and R3 may be adhered to the bottom surface of the first recessed portion 333A, and the strain gauges R2 and R4 may be struck on the bottom surface of the second recessed portion 333B.

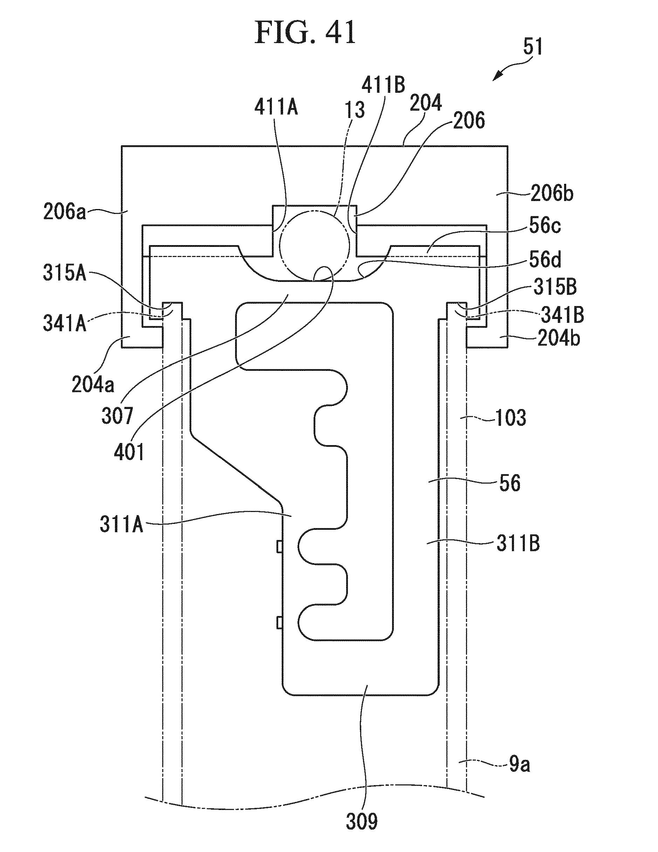

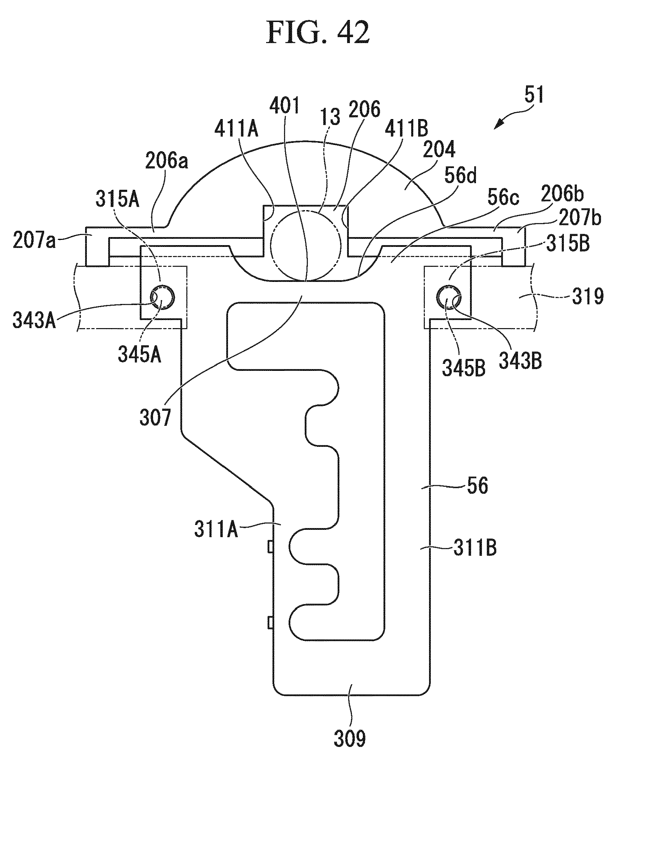

Moreover, as for the load cell 51 of the example shown in FIGS. 36 to 39, it is also desirable to provide the cover-like stopper member 204. An example of the load cell 51 in which the stopper member 204 is provided in this way is shown in FIG. 41, and another example of the load cell is shown in FIG. 42.

In the example shown in FIG. 41, the configuration of the portion of the substrate 56 in the load cell 51 is nearly the same as that of the substrate 56 of the load cell 51 in the example shown in FIGS. 36 to 39.

Also, recessed grooves 341 A and 341 B are formed in a lower surface near both ends of the load-receiving beam portion 307, and the recessed grooves 341A and 341B are configured so as to be fitted into, for example, an upper end of the hollow tube portion 103 of the first coupling arm 9a. Accordingly, in this case, inner bottom surfaces of the recessed grooves 341A and 341B are equivalent to the fulcrum-receiving regions 315A and 315B. In addition, in this case, the recessed grooves 341A and 341 B do not need to be closely fitted into the upper end of the hollow tube portion 103, and may be loosely fitted with slight play.

Also, the substrate 56 in the load cell 51 is nearly vertically inserted into the hollow tube portion 103 of the first coupling arm 9a from above, and the end edge portions of the hollow tube portion 103 at symmetrical positions with the axis as a center, are formed as supporting portions that support the substrate 56.

Moreover, the cover-like stopper member 204 in which, for example, a vertical cross-section has a substantially downward U shape as a whole is disposed in a state where the stopper member is not mechanically coupled with the substrate 56A so as to cover the bearing portion (load-receiving portion) 56c and the spindle (pin) 13 in the substrate 56A of the load cell 51. That is, both the side portions 206a and 206b of the inner surface of the inside space (recess 206) of the stopper member 204 are formed with the wall surfaces 411 A and 411 B that face the outer peripheral surface of the spindle 13, similar to the example shown in FIGS. 28A to 34. The wall surfaces 411A to 411D are formed as vertical wall surfaces that face the outer peripheral surface of the spindle 13 from both sides in the horizontal direction along the length of the spindle 13. Here, although the above wall surfaces are formed at a total of four symmetrical positions, only two wall surfaces 411 A and 411 A are shown for the sake of illustration in FIG. 41.

Both the side portions 206a and 206b of the recess 206 in the cover-like stopper member 204 extend downward to the position on the external surface side of the upper end of the first coupling arm 9a, and the portions (lower ends) 204a and 204b on the tip side thereof are fixed to the external surface side of the upper end of the first coupling arm 9a. In addition, this fixing means is not particularly limited, and arbitrary means can be applied if easily detachable means, such as screw-stopping or fitting, are adopted.

In addition, here, it is desirable that the portion that fixes the stopper member 204 to the first coupling arm 9a be located below the bottom surface of the recessed portion 56d of the bearing portion 56c so that the force when the spindle 13 moves and abuts against the wall surfaces 411 A and 411 B of the recess 206 of the stopper member 204 due to an offset load or the like in the bed surface-forming section of the bed body does not influence the strain (deflection deformation) of the load-receiving beam portion 307.

In addition, in the load cell 51 shown in FIG. 41, the substrate 56 of the load cell is directly inserted into the hollow tube portion 103 of the first coupling arm 9a that is a structural member of the load transmission path of the bed body 1 A, and the stopper member 204 is attached to the coupling arm 9a. However, the substrate 56 may be inserted into a casing that is separate from the structural member of the load transmission path of the bed body 1A, and the stopper member 204 may be attached to or inserted into the casing and may be inserted into the structural member of the load transmission path of the bed body 1A together with the casing, for example, the hollow tube portion 103 of the first coupling arm 9a. Otherwise, similar to the example shown in FIGS. 28A to 34, the casing may be made to serve as the first coupling arm 9a, and the casing may be directly fixed to the bottom frame 5 of the bed body.

Also in the example shown in FIG. 42, the configuration of the portion of the substrate 56 in the load cell 51, and the configuration of the portion of the substrate 56 in the load cell 51 is nearly the same as those of the substrates 56 of the load cells 51 in the example shown in FIGS. 36 to 39 and the example shown in FIG. 41.

Also, attachment holes 343A and 343B passing through the load-receiving beam portion 307 are formed near both the ends of the load-receiving beam portion 307. When the substrate 56 of such a load detector is supported, pin-like members 345A and 345B may be inserted through the attachment hole 343A and 343B, and the pin-like members 345A and 345B may be fixed or locked to the supporting member 319 that constitutes any place in the load transmission path in the bed body 1A. In such a configuration, the attachment holes 343A and 343B are equivalent to the fulcrum-receiving regions 315A and 315B.

Moreover, also by the example of FIG. 42, the cover-like stopper member 204 is disposed in a state where the stopper member is not mechanically coupled with the substrate 56A so as to cover the bearing portion (load-receiving portion) 56c and the spindle (pin) 13 in the substrate 56A of the load cell 51.

Both the side portions 206a and 206b of the inner surface of the inside space (recess 206) of the stopper member 204 are formed with the wall surfaces 411A and 411B that face the outer peripheral surface of the spindle 13, similar to the example shown in FIGS. 28A to 34 and similar to the example shown in FIG. 41. The wall surfaces 411 A to 411 D are formed as vertical wall surfaces that face the outer peripheral surface of the spindle 13 from both sides in the horizontal direction along the length of the spindle 13. Here, although the above wall surfaces are formed at a total of four symmetrical positions, only the two wall surfaces 411A and 411B are shown for the sake of illustration in FIG. 42.

Also, both of the side portions 206a and 206b of the recess 206 in the cover-like stopper member 204 extend outward within the horizontal surface, and extending ends 207a and 207b are fixed to the upper surface of the supporting member 319 that constitutes any place in the load transmission path in the bed body 1 A. This fixing means is not particularly limited, and arbitrary means can be applied if easily detachable means, such as screw-stopping or fitting, are adopted.

In addition, also in the case of this example, it is desirable that the portion that fixes the stopper member 204 to the first coupling arm 9a be located outside the attachment holes 343A and 343B of the substrate 56 so that the force when the spindle 13 moves and abuts against the wall surfaces 411 A and 411 B of the stopper member 204 due to an offset load or the like in the bed surface-forming section 100 of the bed body 1A does not influence the strain (deflection deformation) of the load-receiving beam portion 307.

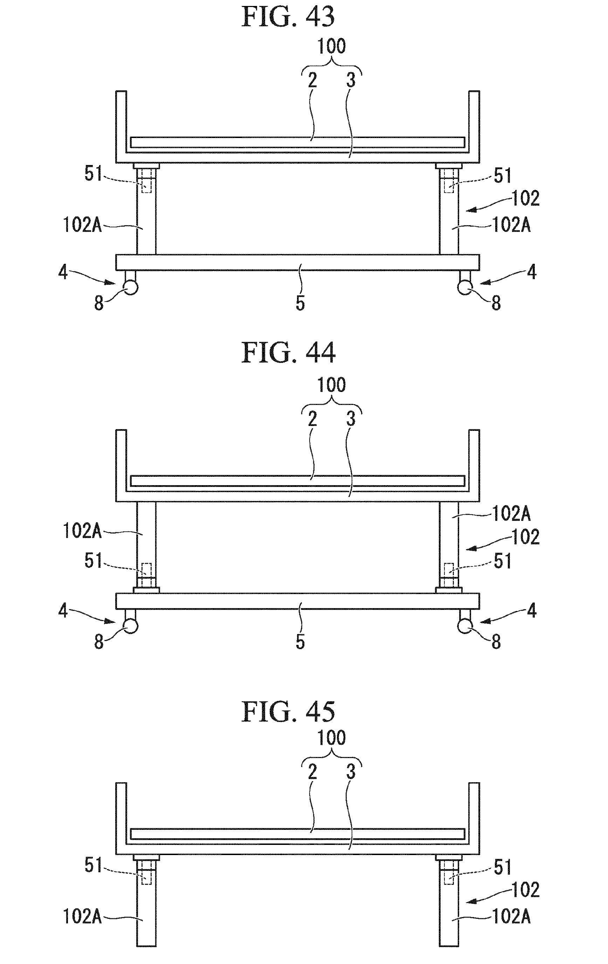

In addition, in the aforementioned example shown in FIGS. 1, 2A, and 2B or the aforementioned example shown in FIG. 7A or 7B, the lifting link mechanism 6 is configured to be provided at the connecting and supporting section 102 between the top frame 3 and the bottom frame 5. However, the invention can also be applied to a case where the lifting link mechanism 6 is not provided at the connecting and supporting section 102. An example thereof is shown in FIG. 43.

In the example shown in FIG. 43, the top frame 3 and the bottom frame 5 are configured to be coupled together by, for example, a plurality of (usually, four) hollow pipe-like vertical posts 102A as the connecting and supporting section 102, and the load cell 51 is interposed between an upper end of each post 102A and the top frame 3.

Additionally, another example in a case where the lifting link mechanism 6 is not provided at the connecting and supporting section 102 is shown in FIG. 44.

In the example shown in FIG. 44, similar to the example shown in FIG. 43, the top frame 3 and the bottom frame 5 are configured to be coupled together by, for example, the plurality of (usually, four) hollow pipe-like vertical posts 102A as the connecting and supporting section 102. In this case, however, the load cell 51 is interposed between a lower end of each post 102A and the bottom frame 5.

In addition, both the example shown in FIG. 43 and the example shown in FIG. 44 are described as the example of the case where the lifting link mechanism is not provided at the connecting and supporting section 102 between the top frame 3 and the bottom frame 5. However, even when the lifting link mechanism is provided at the connecting and supporting section 102, the load cell 51 can be interposed between the top frame 3 and the connecting and supporting section 102 (for example, between the top frame 3 and the lifting link mechanism) in imitation of the example shown in FIG. 43. Additionally, similarly, even when the lifting link mechanism is provided at the connecting and supporting section 102, the load cell 51 can be interposed between the connecting and supporting section 102 and the bottom frame 5 (for example, between the lifting link mechanism and the bottom frames 5) in imitation of the example shown in FIG. 44.

Moreover, when the lifting link mechanism is not provided at the connecting and supporting section 102 between the top frame 3 and the bottom frame 5 as in the example shown in FIG. 43 or the example shown in FIG. 44, the load cell 51 can also be interposed in an intermediate portion of each post 102A that constitutes the connecting and supporting section 102.

Meanwhile, in the invention, the load cells 51 that detect the load of the bed body can also be arranged at the leg sections 4 of the four corners of the bed body 1 A. That is, generally, in this type of bed body 1A, it is usual to provide the caster mechanism 8 for facilitating the movement of the bed body 1 A at the leg section 4. However, the load cell 51 may be interposed inside a portion receiving the caster mechanism 8 or inside the caster mechanism 8.

In addition, when the lifting link mechanism 6 (for example, refer to the example shown in FIG. 43) is not provided, the bottom frame 5 may also be omitted. In this case, the caster mechanism 8 as the leg section 4 may be directly provided at a lower end of each post 102A. Also in the bed body having such a configuration, the load cell 51 may be interposed between the top frame 3 and each post 102A in imitation of the example shown in FIG. 43, or the load cell 51 may be interposed in the leg section 4 (for example, the caster mechanism 8).

Moreover, the invention can also be applied to the bed body without both the lifting link mechanisms and the caster mechanisms. An example in that case is shown in FIG. 45. In this example, the load cell 51 is interposed between the top frame 3 and each post 102A equivalent to the leg section.

As described above, in the bed 1 with a load detection function to which the invention is applied, the load cell 51 may be incorporated into the portion that receives the load from the bed surface-forming section 100 side and transmits the load to the installation surface B side in any place in the load transmission path that leads from the bed surface-forming section (portion constituted from the bed plate 2 and the top frame 3 in the aforementioned respective embodiments) 100 via the connecting and supporting section 102 (irrespective of the presence/absence of the lifting link mechanism 6 or the bottom frame 5) to the leg section 4. Accordingly, the load cell 51 may be interposed between the bed surface-forming section 100 and the connecting and supporting sections 102, in the intermediate portion of the connecting and supporting section 102, between the connecting and supporting section 102 and the leg section 4, or in the portion of the leg section 4.

In addition, in the aforementioned respective examples, the bed surface-forming section 100 that forms the bed surface T in the bed body 1A is constituted by the bed plate 2, and the top frame 3 that supports the bed plate 2. However, depending on the case, the bed surface-forming section 100 may be one that does not have the top frame 3, that is, one including only the bed plate 2. It goes without saying that the invention can also be applied to this case. For example, the load cell 51 may be interposed between the bed plate 2 and the connecting and supporting section (for example, the post 102A) for supporting the bed plate.

Additionally, similar to the above, when the bed surface-forming section 100 is one that does not have the top frame 3, that is, one including only the bed plate 2, there is also a bed body configured such that the lifting link mechanism 6 is provided at the connecting and supporting section 102 for supporting the bed plate 2 so as to directly lift and lower the bed plate 2. It goes without saying that the invention can also be applied to such a case.

Moreover, there is also a bed body configured such that the lifting link mechanism 6 directly lifts and lowers the bed plate 2 by the top frame 3 functioning as a simple enclosure even if the bed surface-forming section 100 includes the top frame 3. In such a case, since the top frame 3 does not substantially support a load, the top frame 3 deviates from the load transmission path that leads from the bed surface-forming section 100 via the connecting and supporting section 102 to the leg section 4. In that case, the load cell 51 may be interposed in any place in the load transmission path that leads from the bed plate 2 via the connecting and supporting section 102 to the leg section 4.

In addition, in the above description, the link mechanism is applied as the mechanism for lifting and lowering the bed surface-forming section 100. However, depending on the case, lifting mechanisms that do not use the link mechanism, for example, lifting mechanisms of a manual or electric rotary screw type (screw type), a jack type, or the like are used. It goes without saying that the invention can also be applied to bed bodies having lifting mechanisms other than such a link mechanism.

Moreover, in the load detector for a bed of the invention, the portions other than the bearing portion as the load-receiving portion in the load cell, in short, may have the actuating portion that has the strain sensor attached thereto and is deflected by a load, and the load-transmitting portion that transmits the load to the structural member on the installation surface side in the bed body, and it goes without saying that the load detector for a bead of the invention is not limited to the configurations shown in the respective embodiments.

### INDUSTRIAL APPLICABILITY

The bed with a load detection function according to the invention can be used in medical facilities (examples: hospitals, clinics, or the like), nursing facilities, child care institutions, other lodging facilities (examples: hotels, inns, or the like), ordinary homes (examples: home care or the like), or the like. In that case, it is possible to detect the loads applied to the bed, thereby monitoring, for example, the state (situation while staying in bed) of a bed user, such as getting into bed (going to bed), getting out of bed (rising), a staying-in-bed position, body motions (examples: tossing about in bed or the like), or postures (examples: lying on one's back, lying on one's stomach, lying on one's side, or the like). Additionally, the load detector for a bed according to the invention can be incorporated not only into a new bed but also into an existing bed. Even in such a case, the above functions can be exhibited.

### REFERENCE SIGNS LIST

- 1:: BED WITH LOAD DETECTION FUNCTION
- 1A:: BED BODY
- 2:: BED PLATE
- 3:: TOP FRAME
- 4:: LEG SECTION
- 5:: BOTTOM FRAME
- 6:: LIFTING LINK MECHANISM
- 8:: CASTER MECHANISM
- 9a:: FIRST COUPLING ARM
- 9b:: SECOND COUPLING ARM
- 9c:: THIRD COUPLING ARM
- 9d:: FOURTH COUPLING ARM
- 11:: ACTUATOR
- 12:: GUIDE SLIT (BEARING)
- 13:: PIN (SPINDLE)
- 50:: LOAD DETECTOR
- 51:: LOAD CELL
- 52:: COMPUTING UNIT
- 53:: TRANSMITTING UNIT
- 54:: RECEIVING UNIT
- 56, 56A, 56B:: SUBSTRATE
- 56a:: ATTACHING PORTION (LOAD-TRANSMITTING PORTION)
- 56b:: CANTILEVER PORTION 5 (ACTUATING PORTION)
- 56c:: BEARING PORTION (LOAD-RECEIVING PORTION)
- 57:: STRAIN SENSOR
- 58:: HOLE PORTION (ROBERVAL MECHANISM)
- 59a:: FIRST EXTENSION PORTION
- 59b:: SECOND EXTENSION PORTION
- 71:: LOAD-TRANSMITTING PORTION
- 73:: ACTUATING PORTION
- 75:: LOAD-RECEIVING PORTION
- 100:: BED SURFACE-FORMING SECTION
- 102:: CONNECTING AND SUPPORTING SECTION
- 103:: HOLLOW TUBE PORTION
- 204:: STOPPER MEMBER
- 206:: RECESS
- 208:: WALL SURFACE
- 307:: LOAD-RECEIVING BEAM PORTION
- 311 A, 311B:: ACTUATING ARM PORTION
- 315A, 315B:: FULCRUM-RECEIVING REGION
- 309:: COUPLING PORTION
- 317:: HOLLOW PORTION
- 401:: SUPPORTING SURFACE
- 411A, 411B, 411C, 411D:: WALL SURFACE
- B:: INSTALLATION SURFACE
- T:: BED SURFACE
- H:: USER
- R1, R2, R3, R4:: STRAIN GAUGE (STRAIN-SENSITIVE RESISTOR)

## Claims

1. A bed with a load detection function that detects a change in a load applied to a bed body, using a load detector attached to the bed body, and detects the state of a user on a bed surface of the bed body,
wherein the bed body is configured to have a bed surface-forming section that forms the bed surface, a leg section that touches an installation surface on which the bed body is to be installed, and a connecting and supporting section that connects the bed surface-forming section and the leg section together and transmits a load from the bed surface-forming section toward the leg section so that the bed surface-forming section is located above the installation surface,
wherein the load detector has a load cell that measures a strain generated by the load being applied to the bed body, and
wherein the load cell is provided in a portion that receives the load from the bed surface-forming section side and transmits the load to the installation surface side, in any place in a load transmission path that leads from the bed surface-forming section via the connecting and supporting section to the installation surface.

2. The bed with a load detection function according to Claim 1,
wherein the load cell has a substrate that generates a strain according to the load from the bed surface-forming section side, and a strain sensor that is attached to the substrate in order to detect the strain of the substrate, the substrate has a load-receiving portion receiving the load from the bed surface-forming section formed on one end side and has a load-transmitting portion transmitting the load to a structural member on the installation surface side in the bed body formed on the other end side, the actuating portion deflected by the load is formed between the load-receiving portion and the load-transmitting portion, and the strain sensor is attached to the actuating portion.

3. The bed with a load detection function according to Claim 2,
wherein the actuating portion in the substrate constituting the load cell is constituted by a cantilever portion that has one end continuously connected to the load-receiving portion and has the other end continuously connected to the load-transmitting portion.

4. The bed with a load detection function according to Claim 2,
wherein a spindle having a substantially horizontal axis is interposed in the load transmission path of the bed body, and any one of the load-receiving portion and the load-transmitting portion of the substrate is formed with a bearing portion equipped with a supporting surface that touches a portion on a lower surface side of an outer peripheral surface of the spindle.

5. The bed with a load detection function according to Claim 4,
wherein the bearing portion has a recessed portion opening to the spindle side so as to accommodate at least a portion of the outer peripheral surface of the spindle, and at least a portion of an inner surface of the recessed portion forms the supporting surface.

6. The bed with a load detection function according to Claim 4,
wherein the supporting surface of the bearing portion is formed by an upward, substantially horizontal plane.

7. The bed with a load detection function according to Claim 4,
wherein a stopper member, which covers the spindle with a gap from the spindle and is not mechanically coupled with at least the load-receiving portion and the actuating portion of the substrate, is provided on the side of the spindle that faces the bearing portion.

8. The bed with a load detection function according to Claim 4,
wherein the substrate is configured so that at least the load-transmitting portion of the substrate is inserted into a tubular member fixed to the structural member on the installation surface side of the bed body, and the stopper member is configured so as to be supported by the tubular member.

9. The bed with a load detection function according to any one of Claims 1 to 3,
wherein the substrate constituting the load cell is interposed in the middle of the connecting and supporting section.

10. The bed with a load detection function according to Claim 9,
wherein the connecting and supporting section includes a lifting link mechanism that lifts and lowers the bed surface-forming section, and
wherein the substrate constituting the load cell is incorporated into the lifting link mechanism.

11. The bed with a load detection function according to Claim 10,
wherein the connecting and supporting section includes a bottom frame that is supported via the leg section above the installation surface in addition to the lifting link mechanism, and
wherein the lifting link mechanism has at least a first arm and a second arm as arms that connect the bed surface-forming section and the bottom arm together, the first arm is connected to the bed surface-forming section side, the second arm is connected to the bottom frame side, and the substrate constituting the load cell is interposed between the bed surface-forming section and the bottom frame.

12. The bed with a load detection function according to Claim 11,
wherein the bed surface-forming section has a bed plate, and a top frame that supports the bed plate, and
wherein the lifting link mechanism has at least a first arm and a second arm as arms that connect the top frame and the bottom arm together, the first arm is connected to the top frame side, the second arm is connected to the bottom frame side, and the substrate constituting the load cell is interposed between the top frame and the bottom arm.

13. The bed with a load detection function according to Claim 12,
wherein the spindle is provided at the end of any one arm of the top arm and the bottom arm where the substrate constituting the load cell is located, and the bearing portion that receives the spindle is formed in any one of the load-receiving portion and load-transmitting portion in the substrate.

14. The bed with a load detection function according to Claim 13,
wherein the load-transmitting portion in the substrate constituting the load cell is an attachment portion that is attached to the other arm of the top arm and the bottom arm.

15. The bed with a load detection function according to Claim 14,
wherein a hollow tube portion is formed at the end of the other arm on the substrate side, the attachment portion of the substrate is inserted into the hollow tube portion, and the attachment portion is configured so as to be supported by the hollow tube portion.

16. The bed with a load detection function according to any one of Claims 1 to 3,
wherein the substrate constituting the load cell is interposed between the bed surface-forming section and the connecting and supporting section.

17. The bed with a load detection function according to any one of Claims 1 to 3,
wherein the substrate constituting the load cell is interposed between the connecting and supporting section and the leg section.

18. The bed with a load detection function according to any one of Claims 1 to 3,
wherein the substrate constituting the load cell is incorporated into the leg section.

19. The bed with a load detection function according to any one of Claims 1 to 3,
wherein the leg section includes a caster mechanism, and the substrate constituting the load cell is incorporated into the caster mechanism.

20. A load detector for a bed that is attached to a bed body, including:
a bed surface-forming section that forms a bed surface;
a leg section that touches an installation surface on which the bed body is to be installed; and
a connecting and supporting section that connects the bed surface-forming section and the leg section together and transmits a load from the bed surface-forming section toward the leg section so that the bed surface-forming section is located above the installation surface, and
that thereby measures a change in a load applied to the bed body, and detects the state of a user on a bed surface of the bed body,
the load detector comprising:
a load cell having
a substrate that generates a strain according to the load from the bed surface-forming section side; and
a strain sensor that is attached to the substrate in order to detect the strain of the substrate,
wherein the substrate is configured so as to be attached to a portion that receives the load from the bed surface-forming section side and transmits the load to the installation surface side, in any place in a load transmission path that leads from the bed surface-forming section of the bed body via the connecting and supporting section to the leg section,
wherein the substrate has:
a load-receiving portion that receives the load from the bed surface-forming section;
an actuating portion that has the strain sensor attached thereto and is deflected by the load; and
a load-transmitting portion that transmits the load to the structural member of the bed body on the installation surface side, and
wherein a bearing portion, that is equipped with a supporting surface that touches a portion of an outer peripheral surface of a spindle that is provided in the load transmission path of the bed body and has a substantially horizontal axis, is formed in any one of the load-receiving portion and the load-transmitting portion of the substrate.

21. The load detector for a bed according to Claim 20,
wherein the bearing portion has a recessed portion opening to the spindle side so as to accommodate at least a portion of the outer peripheral surface of the spindle, and a portion of an inner surface of the recessed portion forms the supporting surface.

22. The load detector for a bed according to Claim 20,
wherein the supporting surface is a substantially horizontal plane.

23. The load detector for a bed according to Claim 20,
wherein a stopper member, which covers the spindle with a gap from the spindle and is not mechanically coupled with the substrate, is provided on the side of the spindle that faces the bearing portion.

24. The load detector for a bed according to Claim 23,
wherein the stopper member has wall surfaces that face the outer peripheral surface of the spindle at positions at both ends of the supporting surface.

25. The load detector for a bed that according to Claim 20,
wherein the substrate is configured so that at least the load-transmitting portion of the substrate is inserted into a tubular member fixed to the structural member on the installation surface side of the bed body, and the stopper member is configured so as to be supported by the tubular member.
